(19) Europäisches Patentamt
European Patent Office
Office européen des brevets

(11) EP 4 197 538 A1

(12) EUROPEAN PATENT APPLICATION
published in accordance with Art. 153(4) EPC

(43) Date of publication:
**21.06.2023 Bulletin 2023/25**

(21) Application number: **21842292.1**

(22) Date of filing: **14.07.2021**

(51) International Patent Classification (IPC):
***A61K 31/519*** *(2006.01)* ***A61P 35/00*** *(2006.01)*
***A61P 43/00*** *(2006.01)* ***C07D 487/04*** *(2006.01)*

(52) Cooperative Patent Classification (CPC):
**A61K 31/519; A61P 35/00; A61P 43/00; C07D 487/04;** Y02P 20/55

(86) International application number:
**PCT/JP2021/026461**

(87) International publication number:
**WO 2022/014639 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.07.2020 JP 2020121525**

(71) Applicant: **Taiho Pharmaceutical Co., Ltd.**
**Chiyoda-ku, Tokyo 101-8444 (JP)**

(72) Inventor: **OGUCHI Kei**
**Tsukuba-shi, Ibaraki 300-2611 (JP)**

(74) Representative: **Blodig, Wolfgang**
**Wächtershäuser & Hartz**
**Patentanwaltspartnerschaft mbB**
**Weinstrasse 8**
**80333 München (DE)**

Remarks:
The complete document including Reference Table(s) and the Sequence Listing(s) can be downloaded from the EPO website

# (54) EGFR INHIBITOR

(57) Provided is a therapeutic agent for a disease associated with EGFR, the agent comprising a compound that has EGFR inhibitory activity and has brain penetration properties as an active ingredient. The present invention provides a compound represented by the formula (I) wherein $R_1$, $R_2$, $R_3$, $R_4$ and $R_5$ are as defined in the present specification, or a salt thereof, and a therapeutic agent for a disease associated with EGFR, the agent comprising a compound represented by the following formula (I), or a salt thereof as an active ingredient.

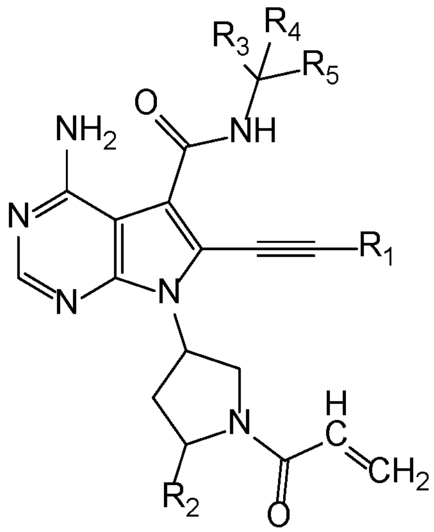

(I)



Processed by Luminess, 75001 PARIS (FR)

(Cont. next page)

[Figure 1]

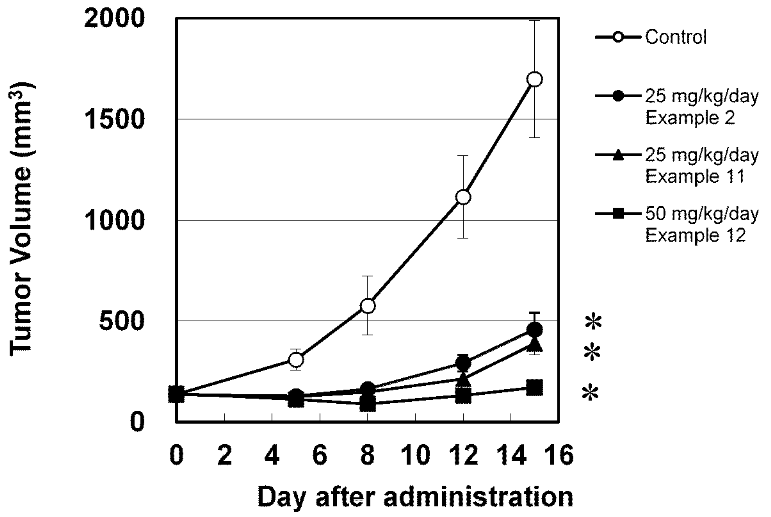


＊ : P<0.001

## Description

Technical Field

The present invention relates to an EGFR inhibitor containing a pyrimidine compound or a salt thereof.

Background Art

EGFR (which is also referred to as "ErbB 1 ") is receptor tyrosine kinase belonging to the ErbB family and has been reported to contribute to cell growth or apoptosis inhibition by binding to epidermal growth factor (which is also referred to as "EGF"), mainly in epithelial tissues, among normal tissues (Non Patent Literature 1).

EGFR is considered to be a proto-oncogene. It has been reported that EGFR gene amplification, overexpression, mutation and the like occur in various types of cancers. From non-clinical and clinical research data, it is considered that activation of EGFR and downstream signals play an important role in the survival and/or proliferation, etc. of cancer cells associated with the genetic abnormality, overexpression and the like of EGFR. For example, a mutation in exon 19 region of EGFR, which deletes amino acids at positions 746 to 750 (which is also referred to as "exon 19 deletion mutation"), and a mutation in exon 21 region, which substitutes an amino acid leucine at position 858 with arginine (which is also referred to as "L858R mutation") are considered to contribute to the survival and/or proliferation of cancer cells by autophosphorylating EGFR in an EGF-independent manner and thereby inducing kinase activity (Non Patent Literature 2). It has been reported that these mutations are present in approximately 30 to 50% of non-small cell lung cancer cases in East Asia and approximately 10% of non-small cell lung cancer cases in Europe and the United States (Non Patent Literature 3).

Accordingly, an inhibitor capable of regulating the kinase activity of EGFR is assumed to inhibit EGFR and downstream signals in cancer cells having EGFR gene amplification, overexpression and/or mutation, so as to exhibit antitumor effects on the cancer cells. Therefore, such an inhibitor is considered to be useful for the treatment, life-prolonging, or QOL improvement of cancer patients.

Hence, a plurality of EGFR inhibitors have heretofore been researched and developed as anticancer agents and are used in the treatment of EGFR mutation-positive tumor. For example, drugs such as afatinib, gefitinib, and erlotinib have been approved as therapeutic agents for EGFR mutation-positive lung cancer having exon 19 deletion mutation or L858R mutation. Also, osimertinib has been approved as a therapeutic agent for EGFR mutation-positive lung cancer having exon 19 deletion mutation or L858R mutation as well as a mutation in exon 20 region, which substitutes an amino acid threonine at position 790 with methionine (which is also referred to as "T790M mutation").

A mutation in exon 20 region, which insets one or more amino acids (which is also referred to as "exon 20 insertion mutation") is also considered as an activating mutation in lung cancer and the like (Non Patent Literature 4). It has been reported that cancer having such a mutation tends to be low sensitive to a plurality of existing EGFR inhibitors. For example, as for the clinical effect of afatinib on EGFR mutation-positive lung cancer, it has been reported that its effect on exon 20 insertion mutation tends to be much lower than that on exon 19 deletion mutation or L858R mutation (Non Patent Literature 5). Regarding lung cancer having exon 20 insertion mutation of EGFR, multiple clinical trials have been carried out. However, under the current circumstances, a therapeutic method therefor has not yet been established. Therefore, it has been desired to develop an EGFR inhibitor having inhibitory activity against exon 20 insertion mutation.

It has been reported that brain metastasis occurs in approximately 25% to 40% of lung cancer cases, in approximately 15% to 30% of breast cancer cases, and in certain percentages of other multiple cancer cases (Non Patent Literatures 6 and 7).

From the viewpoint of the control of pathological conditions including brain metastasis nidus, it has been desired to develop an EGFR inhibitor having inhibitory activity against EGFR mutations and also having brain penetration properties.

Citation List

Non Patent Literature

[Non Patent Literature 1] Nat. Rev. Cancer, vol. 6, pp. 803-812 (2006)
[Non Patent Literature 2] Nature Medicine, vol. 19, pp. 1389-1400 (2013)
[Non Patent Literature 3] Nat. Rev. Cancer, vol. 7, pp. 169-181 (2007)
[Non Patent Literature 4] Signal Transduct Target Ther. 4: 5, pp. 1-10 (2019)
[Non Patent Literature 5] Lancet Oncol. vol. 16, pp. 830-838 (2015)

[Non Patent Literature 6] Current Oncology, 25, p. S103-S114 (2018)
[Non Patent Literature 7] Breast Cancer Research, 18 (1), 8, p. 1-9 (2016)

Summary of Invention

Technical Problem

An object of the present invention is to provide a therapeutic agent for a disease associated with EGFR, comprising a compound that has EGFR inhibitory activity and has brain penetration properties as an active ingredient.

Solution to Problem

As a result of intensive studies, the present inventors have found that a compound represented by the following formula (I) having pyrimidine as a basic skeleton, or a salt thereof has EGFR inhibitory activity and brain penetration properties and is useful as a therapeutic agent for diseases associated with EGFR (particularly, malignant tumor) by inhibiting EGFR, reaching the completion of the present invention.
Specifically, one embodiment of the present invention includes the following:

[1] A therapeutic agent for a disease associated with EGFR, comprising a pyrimidine compound represented by the following formula (I), or a pharmaceutically acceptable salt thereof as an active ingredient:

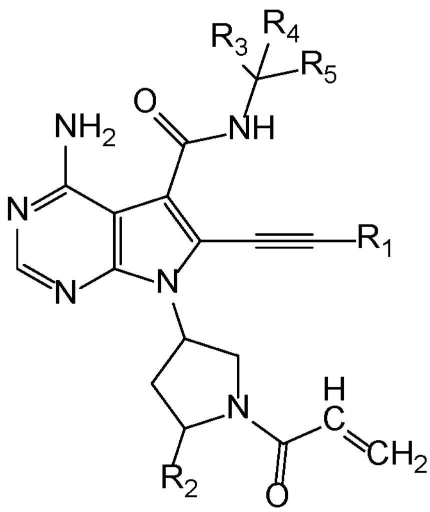

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[2] The therapeutic agent according to [1], wherein the pyrimidine compound is a compound represented by the following formula (II):

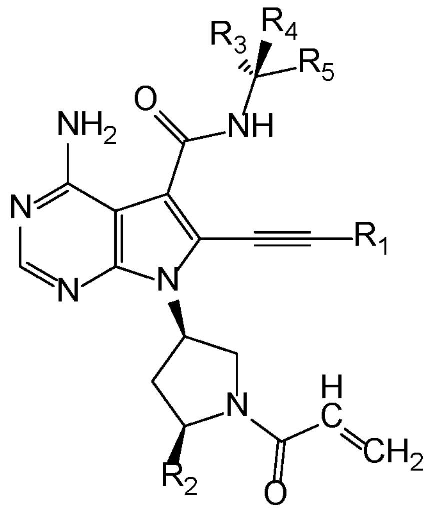

(II)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[3] The therapeutic agent according to [1] or [2], wherein the pyrimidine compound is a compound represented by the formula (I) or (II) wherein
$R_2$ is a C1-C6 alkyl group optionally having 1 to 5 C1-C4 alkoxy groups as a substituent(s).
[4] The therapeutic agent according to any of [1] to [3], wherein the pyrimidine compound is a compound represented by the formula (I) or (II) wherein
$R_3$ is a C1-C4 alkyl group optionally having 1 to 5 fluorine atoms as a substituent(s).
[5] The therapeutic agent according to any of [1] to [4], wherein the pyrimidine compound is a compound represented by the formula (I) or (II) wherein
$R_5$ is a phenyl group optionally having 1 or 2 substituents selected from the group consisting of fluorine atoms and chlorine atoms.
[6] The therapeutic agent according to any of [1] to [5], wherein the pyrimidine compound is a compound represented by the formula (I) or (II) wherein
$R_1$ is a methyl group, a tert-butyl group, or a cyclopropyl group.
[7] The therapeutic agent according to any of [1] to [6], wherein the pyrimidine compound is a compound represented by the formula (I) or (II) wherein
$R_2$ is a methyl group, an ethyl group, a methoxymethyl group, or an ethoxymethyl group.
[8] The therapeutic agent according to any of [1] to [7], wherein the pyrimidine compound is a compound represented by the formula (I) or (II) wherein
$R_3$ is a methyl group.
[9] The therapeutic agent according to any of [1] to [8], wherein the pyrimidine compound is a compound represented by the formula (I) or (II) wherein
$R_4$ is a hydrogen atom.
[10] The therapeutic agent according to any of [1] to [9], wherein the pyrimidine compound is a compound represented by the formula (I) or (II) wherein
$R_5$ is a phenyl group.
[11] The therapeutic agent according to any of [1] to [10], wherein the pyrimidine compound is a compound selected from the following (1) to (3):

(1) 7-((3R,5S)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide,

(2) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide, and
(3) 7-((3R,5S)-1-acryloyl-5-methylpyrrolidin-3-yl)-4-amino-6-(3,3-dimethylbut-1-yn-1-yl)-N-((R)-1-phenylethyl)-7H-pyrrolo[2,3-d]pyrimidine-5-carboxamide.

[12] The therapeutic agent according to any of [1] to [11], wherein the pyrimidine compound is 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide.
[13] The therapeutic agent according to any of [1] to [12], wherein the disease associated with EGFR is malignant tumor having EGFR overexpression, EGFR gene amplification, or an EGFR mutation.
[14] An EGFR inhibitor comprising a pyrimidine compound represented by the following formula (I), or a pharmaceutically acceptable salt thereof as an active ingredient:

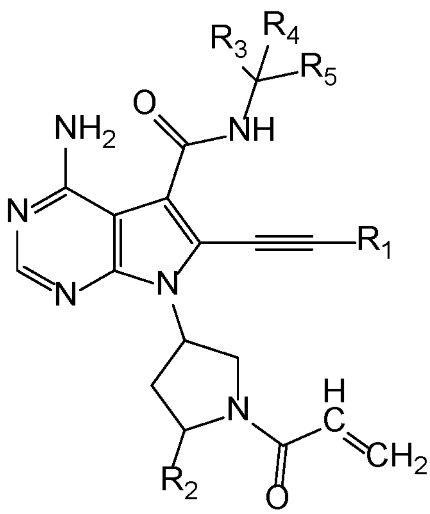

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[15] A therapeutic agent for EGFR-positive tumor, comprising a pyrimidine compound represented by the following formula (I), or a pharmaceutically acceptable salt thereof as an active ingredient:

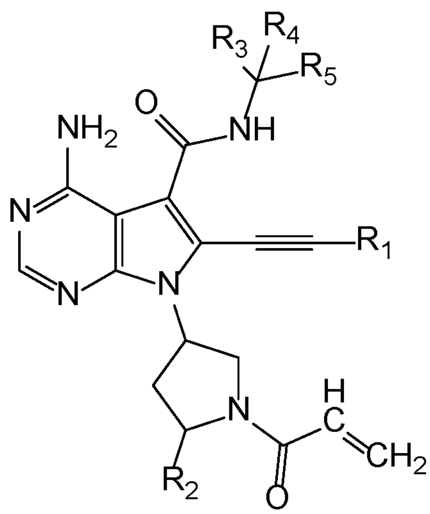

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[16] A pharmaceutical composition for use in the treatment of a disease associated with EGFR, the pharmaceutical composition comprising a pyrimidine compound represented by the following formula (I), or a pharmaceutically acceptable salt thereof and a pharmaceutically acceptable carrier:

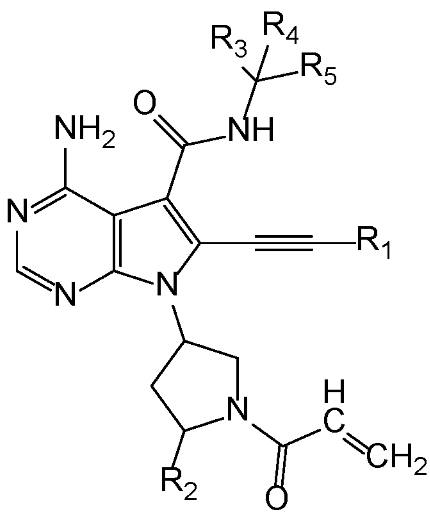

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine

atoms.

[17] The pharmaceutical composition according to [16], wherein the disease associated with EGFR is malignant tumor having EGFR overexpression, EGFR gene amplification, or an EGFR mutation.

The present invention also relates to the following aspects:
A compound represented by the formula (I) or a pharmaceutically acceptable salt thereof for treating a disease associated with EGFR.

Use of a compound represented by the formula (I) or a pharmaceutically acceptable salt thereof for treating a disease associated with EGFR.

Use of a compound represented by the formula (I) or a pharmaceutically acceptable salt thereof for producing a therapeutic agent for a disease associated with EGFR.

A compound represented by the formula (I) or a pharmaceutically acceptable salt thereof for treating EGFR-positive tumor.

Use of a compound represented by the formula (I) or a pharmaceutically acceptable salt thereof for treating EGFR-positive tumor.

Use of a compound represented by the formula (I) or a pharmaceutically acceptable salt thereof for producing a therapeutic agent for EGFR-positive tumor.

A method for treating a disease associated with EGFR, comprising administering an effective amount of a compound represented by the formula (I) or a pharmaceutically acceptable salt thereof to a subject in need thereof.

A method for treating EGFR-positive tumor, comprising administering an effective amount of a compound represented by the formula (I) or a pharmaceutically acceptable salt thereof to a subject in need thereof.

A compound represented by the formula (I) or a pharmaceutically acceptable salt thereof for treating malignant tumor having exon 18 mutant EGFR.

Use of a compound represented by the formula (I) or a pharmaceutically acceptable salt thereof for treating malignant tumor having exon 18 mutant EGFR.

Use of a compound represented by the formula (I) or a pharmaceutically acceptable salt thereof for producing a therapeutic agent for malignant tumor having exon 18 mutant EGFR.

A method for treating malignant tumor having exon 18 mutant EGFR, comprising administering an effective amount of a compound represented by the formula (I) or a pharmaceutically acceptable salt thereof to a subject in need thereof.

A compound represented by the formula (I) or a pharmaceutically acceptable salt thereof for treating malignant tumor having exon 19 mutant EGFR.

Use of a compound represented by the formula (I) or a pharmaceutically acceptable salt thereof for treating malignant tumor having exon 19 mutant EGFR.

Use of a compound represented by the formula (I) or a pharmaceutically acceptable salt thereof for producing a therapeutic agent for malignant tumor having exon 19 mutant EGFR.

A method for treating malignant tumor having exon 19 mutant EGFR, comprising administering an effective amount of a compound represented by the formula (I) or a pharmaceutically acceptable salt thereof to a subject in need thereof.

A compound represented by the formula (I) or a pharmaceutically acceptable salt thereof for treating malignant tumor having exon 20 mutant EGFR.

Use of a compound represented by the formula (I) or a pharmaceutically acceptable salt thereof for treating malignant tumor having exon 20 mutant EGFR.

Use of a compound represented by the formula (I) or a pharmaceutically acceptable salt thereof for producing a therapeutic agent for malignant tumor having exon 20 mutant EGFR.

A method for treating malignant tumor having exon 20 mutant EGFR, comprising administering an effective amount of a compound represented by the formula (I) or a pharmaceutically acceptable salt thereof to a subject in need thereof.

A compound represented by the formula (I) or a pharmaceutically acceptable salt thereof for treating malignant tumor having exon 21 mutant EGFR.

Use of a compound represented by the formula (I) or a pharmaceutically acceptable salt thereof for treating malignant tumor having exon 21 mutant EGFR.

Use of a compound represented by the formula (I) or a pharmaceutically acceptable salt thereof for producing a therapeutic agent for malignant tumor having exon 21 mutant EGFR.

A method for treating malignant tumor having exon 21 mutant EGFR, comprising administering an effective amount of a compound represented by the formula (I) or a pharmaceutically acceptable salt thereof to a subject in need thereof.

A compound represented by the formula (I) or a pharmaceutically acceptable salt thereof for treating malignant tumor resistant to an existing EGFR inhibitor.

Use of a compound represented by the formula (I) or a pharmaceutically acceptable salt thereof for treating malignant tumor resistant to an existing EGFR inhibitor.

Use of a compound represented by the formula (I) or a pharmaceutically acceptable salt thereof for producing a therapeutic agent for malignant tumor resistant to an existing EGFR inhibitor.

A method for treating malignant tumor resistant to an existing EGFR inhibitor, comprising administering an effective amount of a compound represented by the formula (I) or a pharmaceutically acceptable salt thereof to a subject in need thereof.

Advantageous Effects of Invention

The therapeutic agent for a disease associated with EGFR according to the present invention provides a novel therapeutic method for treating a disease associated with EGFR, or EGFR-positive tumor.

The compound of the present invention or a salt thereof has excellent inhibitory activity against wild-type EGFR and mutant EGFR and has excellent brain penetration properties, antitumor effect on malignant tumor, and effect of extending a survival period.

Brief Description of Drawings

[Figure 1] Figure 1 shows the antitumor effects of compounds of Example 2, 11 and 12 against models involving subcutaneous transplantation of the H1975-EGFRinsSVD cell line.

[Figure 2] Figure 2 shows the body weight change percentage of mice when the compounds of Examples 2, 11 and 12 were administered to the models involving subcutaneous transplantation of the H1975-EGFRinsSVD cell line.

[Figure 3] Figure 3 shows the antitumor effects of the compound of Example 11 against models involving direct brain transplantation of the Luciferase gene-introduced exon 20 insertion mutant EGFR expressing cell line (H1975-EGFRinsSVD-Luc).

[Figure 4] Figure 4 shows the survival rate of mice when the compound of Example 11 was administered to the models involving direct brain transplantation of the Luciferase gene-introduced exon 20 insertion mutant EGFR expressing cell line (H1975-EGFRinsSVD-Luc).

Description of Embodiments

One embodiment of the present invention relates to a compound represented by the following formula (I), or a salt thereof:

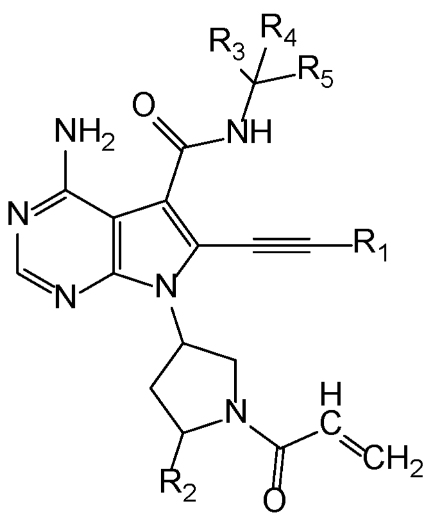

(I)

(wherein $R_1$ to $R_5$ represents as defined above.)

One preferred embodiment of the present invention is a pyrimidine compound represented by the following

formula (II), or a salt thereof

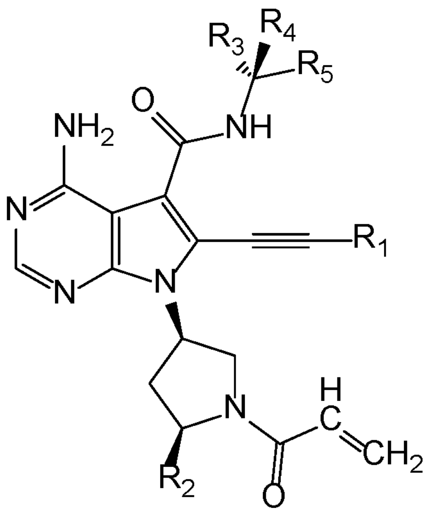

(II)

(wherein $R_1$ to $R_5$ represents as defined above.)

The compound represented by the above formula (I) or formula (II) of the present invention is a compound having pyrrolo[2,3-d]pyrimidine as a basic structure, and this is a novel compound described in none of the aforementioned prior art publications, etc.

In the present description, specific examples of the "halogen atom" may include a chlorine atom, a bromine atom, a fluorine atom, and an iodine atom. Among these, a chlorine atom and a fluorine atom are preferable, and a fluorine atom is more preferable.

In the present description, the "alkyl group" means a linear or branched saturated hydrocarbon group. Specific examples of the alkyl group may include a methyl group, an ethyl group, an n-propyl group, an isopropyl group, an n-butyl group, an isobutyl group, a sec-butyl group, a tert-butyl group, a pentyl group, and a hexyl group. Among these, a linear or branched alkyl group containing 1 to 4 carbon atoms is preferable, and a methyl group and a tert-butyl group are more preferable.

In the present description, the "haloalkyl group" means a linear or branched saturated hydrocarbon group, in which one to all hydrogen atoms are substituted with the above-described halogen atoms. Specific examples of the haloalkyl group may include a monofluoromethyl group, a difluoromethyl group, a trifluoromethyl group, a 1-fluoroethyl group, a 2-fluoroethyl group, a 1,1-difluoroethyl group, a 1,2-difluoroethyl group, a 2,2-difluoroethyl group, a 2,2,2-trifluoroethyl group, a monochloromethyl group, a dichloromethyl group, a trichloromethyl group, a 1-chloroethyl group, a 2-chloroethyl group, and a 1,1-dichloroethyl group. Among these, a linear or branched saturated hydrocarbon group containing 1 to 6 carbon atoms, in which 1 to 3 hydrogen atoms are substituted with the above-described halogen atoms, is preferable, and a monofluoromethyl group is more preferable.

In the present description, the "cycloalkyl group" means a monocyclic or polycyclic saturated hydrocarbon group containing 3 to 7 carbon atoms. Specific examples of the cycloalkyl group may include a cyclopropyl group, a cyclobutyl group, a cyclopentyl group, a cyclohexyl group, and a cycloheptyl group. Among these, a cyclopropyl group and a cyclobutyl group are preferable.

In the present description, the "aromatic hydrocarbon group" means a cyclic substituent consisting of carbon and hydrogen, having an unsaturated bond, in which 4e + 2 (wherein e represents an integer of 1 or greater) electrons are contained in the cyclic $\pi$ electron system.

In the present description, the "C6-C14 aromatic hydrocarbon group" means a monocyclic or polycyclic aromatic hydrocarbon group containing 6 to 14 carbon atoms. Specific examples of the C6-C14 aromatic hydrocarbon group may include a phenyl group, a naphthyl group, a tetrahydronaphthyl group, and an anthracenyl group. Among these, a phenyl group is preferable.

In the present description, the "aralkyl group" means the above-described alkyl group substituted with the above-described aromatic hydrocarbon group. Specific examples of the aralkyl group may include C7-C16 aralkyl groups such as a benzyl group, a phenylethyl group, a phenylpropyl group, a naphthylmethyl group, and a naphthylethyl group. Among these, a benzyl group is preferable.

In the present description, the "unsaturated hydrocarbon group" means a linear or branched hydrocarbon group containing 2 to 6 carbon atoms, which comprises at least one carbon-carbon double bond or triple bond. Specific examples of the unsaturated hydrocarbon group may include a vinyl group, an allyl group, a methylvinyl group, a propenyl group,

a butenyl group, a pentenyl group, a hexenyl group, an ethynyl group, and a 2-propynyl group. Among these, a vinyl group, an allyl group, and a 1-propenyl group are preferable.

In the present description, the "alkenyl group" means a linear or branched hydrocarbon group containing 2 to 6 carbon atoms, which comprises at least one carbon-carbon double bond. Specific examples of the alkenyl group may include C2-C6 alkenyl groups, such as a vinyl group, an allyl group, a 2-methyl-2-propenyl group, an isopropenyl group, a 1-, 2- or 3-butenyl group, a 2-, 3- or 4-pentenyl group, a 2-methyl-2-butenyl group, a 3-methyl-2-butenyl group, and a 5-hexenyl group. Among these, a vinyl group, an allyl group, a 1-propenyl group, and a 2-methyl-2-propenyl group are preferable.

In the present description, the "alkynyl group" means a linear or branched unsaturated hydrocarbon group having at least one triple bond (for example, 1 or 2, and preferably 1 triple bond). Specific examples of the alkynyl group may include C2-C6 alkynyl groups such as an ethynyl group, a 1- or 2-propynyl group, a 1-, 2- or 3-butynyl group, and a 1-methyl-2-propynyl group. Among these, an ethynyl group and a 2-propynyl group are preferable.

In the present description, the "C3-C10 cyclic unsaturated hydrocarbon group" means a monocyclic or polycyclic hydrocarbon group containing 3 to 10 carbon atoms, which comprises at least one carbon-carbon double bond. Specific examples of the C3-C10 cyclic unsaturated hydrocarbon group may include a cyclopropenyl group, a cyclobutenyl group, a cyclopentenyl group, a cyclohexenyl group, a cycloheptenyl group, a cyclooctenyl group, and a cyclononyl group. Among these, a monocyclic or polycyclic hydrocarbon group containing 3 to 7 carbon atoms, which comprises at least one carbon-carbon double bond, is preferable, and a cyclopropenyl group is more preferable.

In the present description, the "alkoxy group" means an oxy group having the above-described alkyl group. Specific examples of the alkoxy group may include C1-C6 alkoxy groups such as a methoxy group, an ethoxy group, an n-propoxy group, an isopropoxy group, an n-butoxy group, an isobutoxy group, a sec-butoxy group, a tert-butoxy group, a pentyloxy group, an isopentyloxy group, and a hexyloxy group. Among these, a methoxy group and an ethoxy group are preferable, and a methoxy group is more preferable.

In the present description, the "haloalkoxy group" may include the above-described alkoxy group having at least one halogen atom (preferably 1 to 13, and more preferably 1 to 3 halogen atoms). Specific examples of the haloalkoxy group may include C1-C6 haloalkoxy groups such as a fluoromethoxy group, a difluoromethoxy group, a trifluoromethoxy group, a trichloromethoxy group, a fluoroethoxy group, a 1,1,1-trifluoroethoxy group, a monofluoro-n-propoxy group, a perfluoro-n-propoxy group, and a perfluoro-isopropoxy group.

In the present description, the "cycloalkoxy group" means an oxy group having the above-described cycloalkyl group. Specific examples of the cycloalkoxy group may include C3-C7 cycloalkoxy groups such as a cyclopropoxy group, a cyclobutoxy group, a cyclopentyloxy group, a cyclohexyloxy group, and a cycloheptyloxy group. Among these, a cyclobutoxy group, a cyclopentyloxy group, and a cyclohexyloxy group are preferable.

In the present description, the "aralkyloxy group" means an oxy group having the above-described aralkyl group. Specific examples of the aralkyloxy group may include C7-C20 aralkyloxy groups such as a benzyloxy group, a phenethyloxy group, a naphthylmethyloxy group, and a fluorenylmethyloxy group. Among these, a benzyloxy group is preferable.

In the present description, the "alkylthio group" means a thioxy group having the above-described alkyl group. Specific examples of the alkylthio group may include C1-C6 alkylthio groups such as a methylthio group, an ethylthio group, an n-propylthio group, an isopropylthio group, an n-butylthio group, an isobutylthio group, a tert-butylthio group, an n-pentylthio group, an isopentylthio group, and a hexylthio group. Among these, a methylthio group, an ethylthio group, and an n-propylthio group are preferable.

In the present description, the "alkoxyalkyl group" means the above-described alkyl group having at least one of the above-described alkoxy groups. Specific examples of the alkoxyalkyl group may include C1-C6 alkoxy-C1-C6 alkyl groups such as a methoxymethyl group, an ethoxyethyl group, a methoxyethyl group, and a methoxypropyl group.

In the present description, the "alkylamino group" means an amino group in which 1 or 2 hydrogen atoms are substituted with a linear or branched hydrocarbon group(s) containing 1 to 6 carbon atoms. Specific examples of the alkylamino group may include a methylamino group, an ethylamino group, a dimethylamino group, a diethylamino group, and an ethylmethylamino group. Among these, preferable is an amino group in which 1 or 2 hydrogen atoms are substituted with a linear or branched hydrocarbon group containing 1 to 3 carbon atoms.

In the present description, the "monoalkylamino group" means an amino group in which one hydrogen atom is substituted with a linear or branched hydrocarbon group. Specific examples of the monoalkylamino group may include a methylamino group, an ethylamino group, an n-propylamino group, an isopropylamino group, an n-butylamino group, an isobutylamino group, a sec-butylamino group, a tert-butylamino group, a pentylamino group, and a hexylamino group. Among these, preferable is an amino group in which one hydrogen atom is substituted with a linear or branched hydrocarbon group containing 1 to 3 carbon atoms.

In the present description, the "dialkylamino group" means an amino group in which two hydrogen atoms are substituted with linear or branched hydrocarbon groups containing 1 to 6 carbon atoms. Specific examples of the dialkylamino group may include a dimethylamino group, a diethylamino group, and an ethylmethylamino group. Among

these, an amino group in which two hydrogen atoms are substituted with linear or branched hydrocarbon groups containing 1 to 3 carbon atoms is preferable, and a dimethylamino group is more preferable.

In the present description, the "acyl group" means a formyl group in which a hydrogen atom is substituted with a linear or branched hydrocarbon group. Specific examples of the acyl group may include an acetyl group, an n-propanoyl group, an isopropanoyl group, an n-butyloyl group, and a tert-butyloyl group. Among these, a formyl group in which a hydrogen atom is substituted with a linear or branched hydrocarbon group containing 1 to 3 carbon atoms is preferable, and an acetyl group is more preferable.

In the present description, the "acyloxy group" means an oxy group having the above-described acyl group. Specific examples of the acyloxy group may include an alkylcarbonyloxy group and an arylcarbonyloxy group. Among these, an oxy group in which a hydrogen atom of formyl group is substituted with a linear or branched hydrocarbon group containing 1 to 3 carbon atoms is preferable, and an alkylcarbonyloxy group is more preferable.

In the present description, the "alkoxycarbonyl group" means a carbonyl group having the above-described alkoxy group. Specific examples of the alkoxycarbonyl group may include (C1-C6alkoxy)carbonyl groups such as a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, a pentyloxycarbonyl group, an isopentyloxycarbonyl group, and a hexyloxycarbonyl group. Among these, a tert-butoxycarbonyl group is preferable.

In the present description, the "aralkyloxycarbonyl group" means a carbonyl group having the above-described aralkyloxy. Specific examples of the aralkyloxycarbonyl group may include (C6-C20 aralkyl)oxycarbonyl groups such as a benzyloxycarbonyl group, a phenethyloxycarbonyl group, a naphthylmethyloxycarbonyl group, and a fluorenylmethyloxycarbonyl group. Among these, a benzyloxycarbonyl group is preferable.

In the present description, the "saturated heterocyclic group" means a monocyclic or polycyclic saturated heterocyclic group having at least one heteroatom (preferably 1 to 5, and more preferably 1 to 3 heteroatoms) selected from nitrogen atoms, oxygen atoms, and sulfur atoms. Specific examples of the saturated heterocyclic group may include an aziridinyl group, an azetidinyl group, an imidazolidinyl group, a morpholino group, a pyrrolidinyl group, a piperidinyl group, a piperazinyl group, a tetrahydrofuranyl group, a tetrahydropyranyl group, a tetrahydrothiophenyl group, a thiazolidinyl group, and an oxazolidinyl group. Among these, an azetidinyl group, a pyrrolidinyl group, and a piperidinyl group are preferable, and an azetidinyl group and a pyrrolidinyl group are more preferable.

In the present description, the "unsaturated heterocyclic group" means a monocyclic or polycyclic completely unsaturated or partially unsaturated heterocyclic group having at least one heteroatom (preferably 1 to 5, and more preferably 1 to 3 heteroatoms) selected from nitrogen atoms, oxygen atoms, and sulfur atoms. Specific examples of the unsaturated heterocyclic group may include an imidazolyl group, a thienyl group, a pyrrolyl group, an oxazolyl group, an isoxazolyl group, a thiazolyl group, an isothiazolyl group, a thiadiazolyl group, an oxadiazolyl group, a pyrazolyl group, a triazolyl group, a tetrazolyl group, a pyridyl group, a pyrazyl group, a pyrimidinyl group, a pyridazinyl group, an indolyl group, an isoindolyl group, an indazolyl group, a triazolopyridyl group, a benzimidazolyl group, a benzoxazolyl group, a benzothiazolyl group, a benzothienyl group, a furanyl group, a benzofuranyl group, a purinyl group, a quinolyl group, an isoquinolyl group, a quinazolinyl group, a quinoxalyl group, a methylenedioxyphenyl group, an ethylenedioxyphenyl group, and a dihydrobenzofuranyl group. Among these, an imidazolyl group, a pyrazolyl group, a thiazolyl group, an isoxazolyl group, and a furanyl group are preferable; an imidazolyl group, a pyrazolyl group, and a thiazolyl group are more preferable; and an imidazolyl group is most preferable.

In the present description, the "saturated heterocyclic oxy group" means an oxy group having the above-described saturated heterocyclic group. Specific examples of the saturated heterocyclic oxy group may include a morpholinyloxy group, a 1-pyrrolidinyloxy group, a piperidinooxy group, a piperazinyloxy group, a 4-methyl-1-piperazinyloxy group, a tetrahydrofuranyloxy group, a tetrahydropyranyloxy group, a tetrahydrothiophenyloxy group, a thiazolidinyloxy group, and an oxazolidinyloxy group. Among these, a 1-pyrrolidinyloxy group, a piperidinooxy group, and a piperazinyloxy group are preferable.

In the compound represented by the formula (I) or the formula (II) of the present invention, $R_1$ is a C1-C4 alkyl group optionally having a C1-C4 alkoxy group as a substituent, or a C3-C4 cycloalkyl group.

The "C1-C4 alkoxy group" in the "C1-C4 alkyl group optionally having a C1-C4 alkoxy group as a substituent" represented by $R_1$ is preferably a methoxy group or an ethoxy group, and most preferably a methoxy group. Herein, the number of substituents is preferably 1 to 3, and most preferably 1. When the C1-C4 alkyl group has two or more substituents, the substituents may be identical to or different from each other.

The "C1-C4 alkyl group" in the "C1-C4 alkyl group optionally having a C1-C4 alkoxy group as a substituent" represented by $R_1$ is preferably a methyl group, an ethyl group, an n-propyl group, an isopropyl group, or a tert-butyl group, more preferably a methyl group, an ethyl group, an isopropyl group, or a tert-butyl group, and most preferably a methyl group or a tert-butyl group.

The "C1-C4 alkyl group optionally having a C1-C4 alkoxy group as a substituent" represented by $R_1$ is preferably a C1-C4 alkyl group having 1 to 3 methoxy groups as substituents, more preferably a methyl group, an ethyl group, an isopropyl group, a tert-butyl group, or a 1-methyl-1-methoxyethyl group, and most preferably a methyl group or a tert-butyl group.

The "C3-C4 cycloalkyl group" represented by $R_1$ is preferably a cyclopropyl group or a cyclobutyl group, and most preferably a cyclopropyl group.

$R_1$ is preferably a C1-C4 alkyl group optionally having 1 to 3 C1-C4 alkoxy groups as substituents, or a C3-C4 cycloalkyl group.

$R_1$ is more preferably a C1-C4 alkyl group optionally having 1 to 3 methoxy groups as substituents, or a C3-C4 cycloalkyl group.

$R_1$ is further preferably a methyl group, an ethyl group, an isopropyl group, a tert-butyl group, a 1-methyl-1-methoxyethyl group, or a cyclopropyl group.

$R_1$ is most preferably a methyl group, a tert-butyl group, or a cyclopropyl group.

In the compound represented by the formula (I) or the formula (II) of the present invention, $R_2$ is a hydrogen atom, a halogen atom, a C1-C6 alkyl group optionally having 1 to 5 C1-C4 alkoxy groups or fluorine atoms each as a substituent(s), or a C1-C6 alkoxy group.

The "halogen atom" represented by $R_2$ is preferably a fluorine atom or a chlorine atom.

The "C1-C4 alkoxy group" in the "C1-C6 alkyl group optionally having 1 to 5 C1-C4 alkoxy groups or fluorine atoms each as a substituent(s)" represented by $R_2$ is preferably a methoxy group or an ethoxy group, and most preferably a methoxy group.

The "C1-C6 alkyl group optionally having 1 to 5 C1-C4 alkoxy groups or fluorine atoms each as a substituent(s)" represented by $R_2$ is preferably a methyl group, an ethyl group, an n-propyl group, an isopropyl group, or a tert-butyl group, and most preferably a methyl group.

The "C1-C6 alkyl group" in the "C1-C6 alkyl group optionally having 1 to 5 C1-C4 alkoxy groups or fluorine atoms each as a substituent(s)" represented by $R_2$ is preferably a C1-C6 alkyl group optionally having 1 to 5 methoxy groups, ethoxy groups, or fluorine atoms as a substituent(s) (specifically, a methyl group, a methoxymethyl group, an ethoxymethyl group, a methoxyethyl group, an ethoxyethyl group, a fluoromethyl group, a difluoromethyl group, a trifluoromethyl group, etc.), more preferably a C1-C6 alkyl group, further preferably a methyl group, an ethyl group, an n-propyl group, an isopropyl group, or a tert-butyl group, and most preferably a methyl group.

The "C1-C6 alkoxy group" represented by $R_2$ is preferably a methoxy group or an ethoxy group, and most preferably a methoxy group.

$R_2$ is preferably a C1-C6 alkyl group optionally having 1 to 5 C1-C4 alkoxy groups or fluorine atoms each as a substituent(s). In one embodiment, $R_2$ is a C1-C6 alkyl group optionally having 1 to 5 methoxy groups, ethoxy groups, or fluorine atoms as a substituent(s). In another embodiment, $R_2$ is a methyl group, an ethyl group, an n-propyl group, an isopropyl group, or a tert-butyl group (preferably a methyl group or an ethyl group, and more preferably a methyl group), each optionally having 1 to 5 methoxy groups, ethoxy groups, or fluorine atoms as a substituent(s).

$R_2$ is more preferably a C1-C6 alkyl group optionally having 1 to 5 C1-C4 alkoxy groups as a substituent(s). In one embodiment, $R_2$ is a C1-C6 alkyl group optionally having 1 to 5 methoxy groups or ethoxy groups as a substituent(s). In another embodiment, $R_2$ is a methyl group, an ethyl group, an n-propyl group, an isopropyl group, or a tert-butyl group (preferably a methyl group or an ethyl group, and more preferably a methyl group) each optionally having 1 to 5 methoxy groups or ethoxy groups as a substituent(s). In a further embodiment, $R_2$ is a methyl group, an ethyl group, a methoxymethyl group, or an ethoxymethyl group.

$R_2$ is even more preferably a C1-C6 alkyl group.

$R_2$ is further preferably a methyl group, an ethyl group, an n-propyl group, an isopropyl group, or a tert-butyl group.

$R_2$ is particularly preferably a methyl group or an ethyl group.

$R_2$ is most preferably a methyl group.

In the compound represented by the formula (I) or the formula (II) of the present invention, $R_3$ is a hydrogen atom, or a C1-C4 alkyl group optionally having 1 to 5 fluorine atoms as a substituent(s).

The "C1-C4 alkyl group" in the "C1-C4 alkyl group optionally having 1 to 5 fluorine atoms as a substituent(s)" represented by $R_3$ is preferably a methyl group, an ethyl group, an n-propyl group, an isopropyl group, or a tert-butyl group, more preferably a methyl group or an ethyl group, and most preferably a methyl group.

The "C1-C4 alkyl group optionally having 1 to 5 fluorine atoms as a substituent(s)" represented by $R_3$ is preferably a methyl group, a fluoromethyl group, a difluoromethyl group, a trifluoromethyl group, or an ethyl group, more preferably a methyl group, a trifluoromethyl group, or an ethyl group, and most preferably a methyl group.

$R_3$ is preferably a C1-C4 alkyl group optionally having 1 to 5 fluorine atoms as a substituent(s).

$R_3$ is more preferably a methyl group, a fluoromethyl group, a difluoromethyl group, a trifluoromethyl group, an ethyl group, a fluoroethyl group, a difluoroethyl group, a trifluoroethyl group, an n-propyl group, an isopropyl group, or a tert-butyl group.

$R_3$ is even more preferably a methyl group, a fluoromethyl group, a difluoromethyl group, a trifluoromethyl group, or an ethyl group.

$R_3$ is further preferably a methyl group, a trifluoromethyl group, or an ethyl group.

$R_3$ is particularly preferably a methyl group or an ethyl group.

$R_3$ is most preferably a methyl group.

In the compound represented by the formula (I) or the formula (II) of the present invention, $R_4$ is a hydrogen atom or a C1-C4 alkyl group.

The "C1-C4 alkyl group" represented by $R_4$ is preferably a methyl group, an ethyl group, an n-propyl group, an isopropyl group, or a tert-butyl group, more preferably a methyl group or an ethyl group, and most preferably a methyl group.

$R_4$ is preferably a hydrogen atom, a methyl group, an ethyl group, an n-propyl group, an isopropyl group, or a tert-butyl group.

$R_4$ is more preferably a hydrogen atom, a methyl group, or an ethyl group.

$R_4$ is further preferably a hydrogen atom or a methyl group.

$R_4$ is most preferably a hydrogen atom.

In the compound represented by the formula (I) or the formula (II) of the present invention, $R_5$ is a phenyl group optionally having 1 to 3 substituents selected from the group consisting of fluorine atoms and chlorine atoms.

$R_5$ is preferably a phenyl group optionally having 1 or 2 substituents selected from the group consisting of fluorine atoms and chlorine atoms.

$R_5$ is more preferably a phenyl group, a 2-fluorophenyl group, a 3-chlorophenyl group, a 2,3-difluorophenyl group, a 2,4-difluorophenyl group, or a 3,5-difluorophenyl group.

$R_5$ is most preferably a phenyl group.

The compound of the present invention is preferably the compound represented by the formula (I) or the formula (II), or a salt thereof, wherein, in the formula (I) or the formula (II),

$R_1$ is a C1-C4 alkyl group optionally having a C1-C4 alkoxy group as a substituent, or a C3-C4 cycloalkyl group,
$R_2$ is a C1-C6 alkyl group optionally having 1 to 5 C1-C4 alkoxy groups as a substituent(s),
$R_3$ is a C1-C4 alkyl group optionally having 1 to 5 fluorine atoms as a substituent(s),
$R_4$ is a hydrogen atom or a C1-C4 alkyl group, and
$R_5$ is a phenyl group optionally having 1 or 2 substituents selected from the group consisting of fluorine atoms and chlorine atoms.

The compound of the present invention is more preferably the compound represented by the formula (I) or the formula (II), or a salt thereof, wherein, in the formula (I) or the formula (II),

$R_1$ is a methyl group, an ethyl group, an n-propyl group, an isopropyl group, a tert-butyl group, a 1-methyl-1-methoxyethyl group, a cyclopropyl group, or a cyclobutyl group,
$R_2$ is a methyl group, an ethyl group, an n-propyl group, a tert-butyl group, a methoxymethyl group, or an ethoxymethyl group,
$R_3$ is a methyl group, a fluoromethyl group, a difluoromethyl group, a trifluoromethyl group, an ethyl group, a fluoroethyl group, a difluoroethyl group, a trifluoroethyl group, an n-propyl group, an isopropyl group, or a tert-butyl group,
$R_4$ is a hydrogen atom, a methyl group, an ethyl group, an n-propyl group, an isopropyl group, or a tert-butyl group, and
$R_5$ is a phenyl group, a 2-fluorophenyl group, a 3-fluorophenyl group, a 2,4-difluorophenyl group, a 2,3-difluorophenyl group, a 3,5-difluorophenyl group, a 2-chlorophenyl group, a 3-chlorophenyl group, a 2,4-dichlorophenyl group, or a 3,5-dichlorophenyl group.

The compound of the present invention is even more preferably the compound represented by the formula (II), or a salt thereof, wherein, in the formula (II),

$R_1$ is a methyl group, an ethyl group, an isopropyl group, a tert-butyl group, a 1-methyl-1-methoxyethyl group, or a cyclopropyl group,
$R_2$ is a methyl group, an ethyl group, a methoxymethyl group, or an ethoxymethyl group,
$R_3$ is a methyl group, a fluoromethyl group, a difluoromethyl group, a trifluoromethyl group, or an ethyl group,
$R_4$ is a hydrogen atom, a methyl group, or an ethyl group, and
$R_5$ is a phenyl group, a 2-fluorophenyl group, a 3-chlorophenyl group, a 2,3-difluorophenyl group, a 2,4-difluorophenyl group, or a 3,5-difluorophenyl group.

The compound of the present invention is further preferably the compound represented by the formula (II), or a salt thereof, wherein, in the formula (II),

$R_1$ is a methyl group, a tert-butyl group, or a cyclopropyl group,

$R_2$ is a methyl group, an ethyl group, a methoxymethyl group, or an ethoxymethyl group,
$R_3$ is a methyl group, a trifluoromethyl group, or an ethyl group,
$R_4$ is a hydrogen atom or a methyl group, and
$R_5$ is a phenyl group.

The compound of the present invention is further preferably the compound represented by the formula (II), or a salt thereof, wherein, in the formula (II),

$R_1$ is a methyl group, a tert-butyl group, or a cyclopropyl group,
$R_2$ is a methyl group, an ethyl group, or a methoxymethyl group,
$R_3$ is a methyl group,
$R_4$ is a hydrogen atom, and
$R_5$ is a phenyl group.

The compound of the present invention is particularly preferably the compound represented by the formula (II), or a salt thereof, wherein, in the formula (II),

$R_1$ is a methyl group, a tert-butyl group, or a cyclopropyl group,
$R_2$ is a methyl group,
$R_3$ is a methyl group,
$R_4$ is a hydrogen atom, and
$R_5$ is a phenyl group.

Specific examples of the compound represented by the formula (I) or the formula (II) may include compounds produced in the following Examples, but are not limited thereto.
One embodiment of the present invention relates to a compound selected from the following (1) to (18), or a salt thereof. One embodiment of the present invention relates to a compound selected from the following (1) to (15), or a salt thereof.

(1) 7-((3R,5S)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide,
(2) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide,
(3) 7-((3R,SS)-1-acryloyl-5-methylpyrrolidin-3-yl)-4-amino-6-(3,3-dimethylbut-1-yn-1-yl)-N-((R)-1-phenylethyl)-7H-pyrrolo[2,3-d]pyrimidine-5-carboxamide,
(4) 7-(R)-((3R,SS)-1-acryloyl-5-methylpyrrolidin-3-yl)-4-amino-N-((R)-1-(3,5-difluorophenyl)ethyl)-6-(prop-1-yn-1-yl)-7H-pyrrolo[2,3-d]pyrimidine-5-carboxamide,
(5) 7-((3R,5S)-1-acryloyl-5-methylpyrrolidin-3-yl)-4-amino-N-(2-phenylpropan-2-yl)-6-(prop-1-yn-1-yl)-7H-pyrrolo[2,3-d]pyrimidine-5-carboxamide,
(6) 7-((3R,SS)-1-acryloyl-5-methylpyrrolidin-3-yl)-4-amino-N-((R)-1-phenylpropyl)-6-(prop-1-yn-1-yl)-7H-pyrrolo[2,3-d]pyrimidine-5-carboxamide,
(7) 7-((3R,SS)-1-acryloyl-5-methylpyrrolidin-3-yl)-4-amino-N-(2-(2-fluorophenyl)propan-2-yl)-6-(prop-1-yn-1-yl)-7H-pyrrolo[2,3-d]pyrimidine-5-carboxamide,
(8) 7-((3R,5S)-1-acryloyl-5-methylpyrrolidin-3-yl)-4-amino-N-((R)-1-(3-chlorophenyl)ethyl)-6-(prop-1-yn-1-yl)-7H-pyrrolo[2,3-d]pyrimidine-5-carboxamide,
(9) 7-((3R,5S)-1-acryloyl-5-methylpyrrolidin-3-yl)-4-amino-N-((R)-1-(2,4-difluorophenyl)ethyl)-6-(prop-1-yn-1-yl)-7H-pyrrolo[2,3-d]pyrimidine-5-carboxamide,
(10) 7-((3R,5S)-1-acryloyl-5-methylpyrrolidin-3-yl)-4-amino-6-(prop-1-yn-1-yl)-N-((S)-2,2,2-trifluoro-1-phenylethyl)-7H-pyrrolo[2,3-d]pyrimidine-5-carboxamide,
(11) 7-((3R,5S)-1-acryloyl-5-methylpyrrolidin-3-yl)-4-amino-6-(cyclopropylethynyl)-N-(2-phenylpropan-2-yl)-7H-pyrrolo[2,3-d]pyrimidine-5-carboxamide,
(12) 7-((3R,5S)-1-acryloyl-5-methylpyrrolidin-3-yl)-4-amino-6-(cyclopropylethynyl)-N-((R)-1-(2,3-difluorophenyl)ethyl)-7H-pyrrolo[2,3-d]pyrimidine-5-carboxamide,
(13) 7-((3R,5S)-1-acryloyl-5-methylpyrrolidin-3-yl)-4-amino-6-(3-methoxy-3-methylbut-1-yn-1-yl)-N-((R)-1-phenylethyl)-7H-pyrrolo[2,3-d]pyrimidine-5-carboxamide,
(14) 7-((3R,5S)-1-acryloyl-5-methylpyrrolidin-3-yl)-4-amino-6-(but-1-yn-1-yl)-N-((R)-1-phenylethyl)-7H-pyrrolo[2,3-d]pyrimidine-5-carboxamide,
(15) 7-((3R,SS)-1-acryloyl-5-methylpyrrolidin-3-yl)-4-amino-N-(2-(2-fluorophenyl)propan-2-yl)-6-(3-methylbut-1-yn-1-yl)-7H-pyrrolo[2,3-d]pyrimidine-5-carboxamide,

(16) 7-((3R,SS)-1-acryloyl-5-ethylpyrrolidin-3-yl)-4-amino-N-((R)-1-phenylethyl)-6-(prop-1-yn-1-yl)-7H-pyrrolo[2,3-d]pyrimidine-5-carboxamide,

(17) 7-((3R,5S)-1-acryloyl-5-ethylpyrrolidin-3-yl)-4-amino-6-(cyclopropylethynyl)-N-((R)-1-phenylethyl)-7H-pyrrolo[2,3-d]pyrimidine-5-carboxamide,

(18) 7-((3R,5R)-1-acryloyl-5-(methoxymethyl)pyrrolidin-3-yl)-4-amino-6-(cyclopropylethynyl)-N-((R)-1-phenylethyl)-7H-pyrrolo[2,3-d]pyrimidine-5-carboxamide, and

(19) 7-((3R,5R)-1-acryloyl-5-(ethoxymethyl)pyrrolidin-3-yl)-4-amino-6-(cyclopropylethynyl)-N-((R)-1-phenylethyl)-7H-pyrrolo[2,3-d]pyrimidine-5-carboxamide.

A preferred example of the compound represented by the formula (I) or the formula (II) may be a compound selected from the following (1) to (3), or a salt thereof.

(1) 7-((3R,5S)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide,

(2) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide, and

(3) 7-((3R,SS)-1-acryloyl-5-methylpyrrolidin-3-yl)-4-amino-6-(3,3-dimethylbut-1-yn-1-yl)-N-((R)-1-phenylethyl)-7H-pyrrolo[2,3-d]pyrimidine-5-carboxamide.

The most preferable pyrimidine compound of the present invention is 7-((3R,5 S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide.

< Method for producing compound represented by formula (I) >

The compound according to the present invention can be produced by, for example, the following production method or the methods described in the Examples. However, the method for producing the compound according to the present invention is not limited to these examples.

The compounds (I) and (II) of the present invention can be produced by applying, for example, the following production method.

< Production Method >

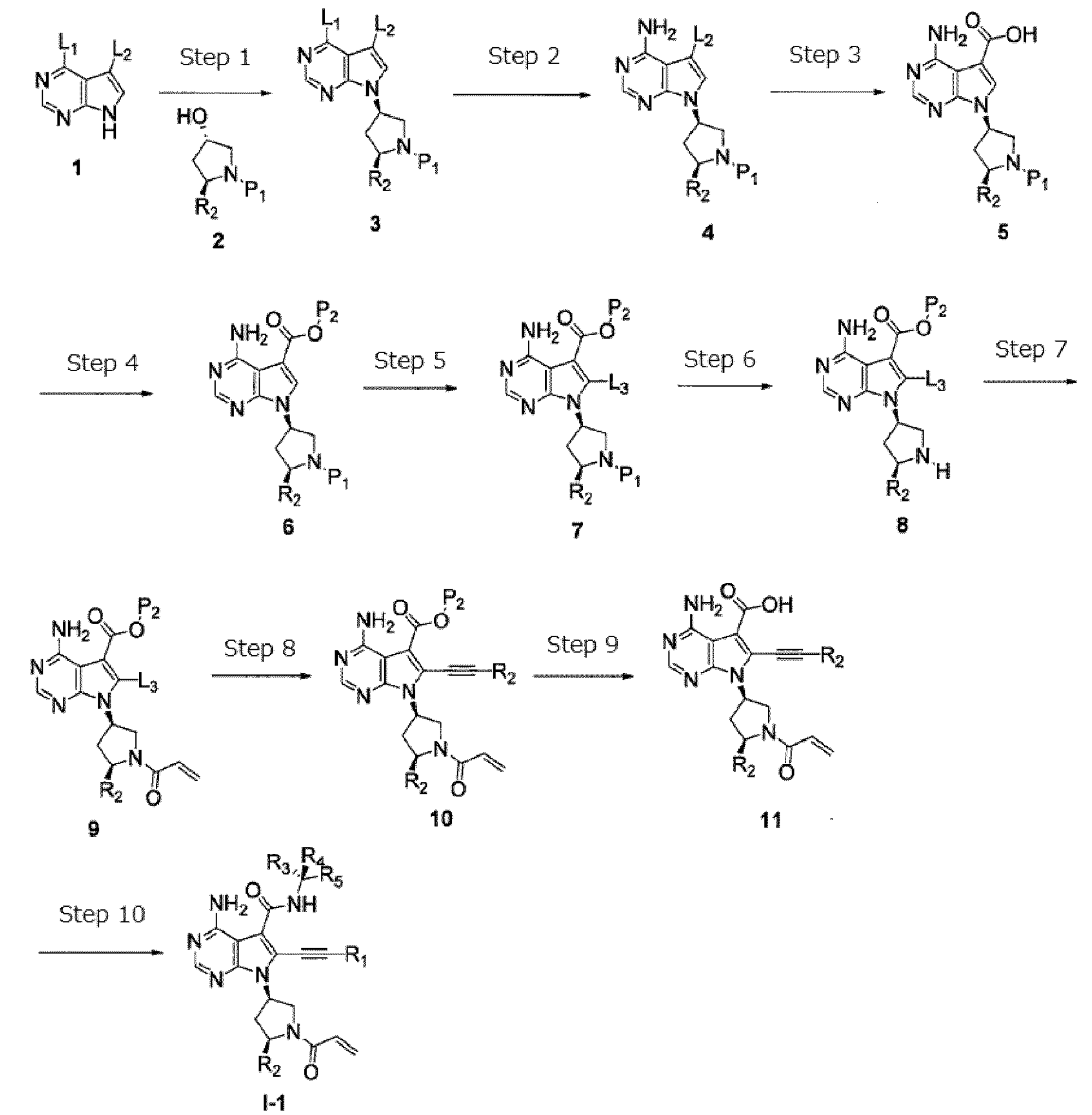


In the above process, $L_1$, $L_2$, and $L_3$, which are the same or different, each represent a leaving group; $P_1$ and $P_2$, which are the same or different, each represent a protective group; and other symbols are as defined above.

< Step 1 >

This step is a method of obtaining a compound represented by the formula 3 by performing a Mitsunobu reaction between a compound represented by the formula 1 and a compound represented by the formula 2 that is a commercially available compound or can be produced by a known method. The Mitsunobu reaction is generally carried out in the presence of a Mitsunobu reagent and a phosphine reagent.

The compound represented by the formula 2 (in the formula 2, $P_1$ represents a protective group for an amino group) can be used in an amount of 1 to 10 equivalents, and preferably 1 to 3 equivalents, based on the amount of the compound represented by the formula 1 (1 mole).

The "protective group for an amino group" is not particularly limited, as long as it has a protective function. Examples of the protective group for an amino group may include: aralkyl groups, such as a benzyl group, a p-methoxybenzyl group, a 3,4-dimethoxybenzyl group, an o-nitrobenzyl group, a p-nitrobenzyl group, a benzhydryl group, a trityl group, and a cumyl group; lower alkanoyl groups, such as, for example, a formyl group, an acetyl group, a propionyl group, a butyryl group, a pivaloyl group, a trifluoroacetyl group, and a trichloroacetyl group; for example, benzoyl groups; arylalkanoyl groups, such as, for example, a phenylacetyl group and a phenoxyacetyl group; lower alkoxycarbonyl groups, such as, for example, a methoxycarbonyl group, an ethoxycarbonyl group, a propyloxycarbonyl group, and a tert-butoxycarbonyl group; aralkyloxycarbonyl groups, such as, for example, a p-nitrobenzyloxycarbonyl group and a

phenethyloxycarbonyl group; lower alkylsilyl groups, such as, for example, a trimethylsilyl group and a tert-butyldimethylsilyl group; for example, tetrahydropyranyl groups; for example, trimethylsilylethoxymethyl groups; lower alkylsulfonyl groups, etc., such as, for example, a methylsulfonyl group, an ethylsulfonyl group, and a tert-butylsulfonyl group; lower alkylsulfinyl groups, etc., such as for example, a tert-butylsulfinyl group; arylsulfonyl groups, etc., such as, for example, a benzenesulfonyl group and a toluenesulfonyl group; and imide groups, such as, for example, a phthalimide group. Among these, a trifluoroacetyl group, an acetyl group, a tert-butoxycarbonyl group, a benzyloxycarbonyl group, a trimethylsilylethoxymethyl group, or a cumyl group is particularly preferable.

As a Mitsunobu reagent, diethyl azodicarboxylate, diisopropyl azodicarboxylate or the like is used. Such a Mitsunobu reagent is used in an amount of generally approximately 1 to 100 moles, and preferably approximately 1 to 10 moles, based on the compound represented by the formula 1 (1 mole).

As a phosphine reagent, triphenylphosphine, tributylphosphine, trifurylphosphine or the like is used. Such a phosphine reagent is used in an amount of generally approximately 1 to 100 moles, and preferably approximately 1 to 10 moles, based on the compound represented by the formula 1 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compound represented by the formula 3 can be isolated and purified by known separation and purification means, or can be subjected to the subsequent step without isolation and purification.

< Step 2 >

This step is a method of obtaining a compound represented by the formula 4 by allowing the compound represented by the formula 3 to react with ammonia or a salt thereof.

The ammonia or a salt thereof can be used in an amount of 1 to 1000 equivalents, and preferably 1 to 100 equivalents, based on the amount of the compound represented by the formula 3 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 150° C.

The thus obtained compound represented by the formula 4 can be isolated and purified by known separation and purification means, or can be subjected to the subsequent step without isolation and purification.

< Step 3 >

This step is a method of obtaining a compound represented by the formula 5 by reacting the compound represented by the formula 4 under a carbon monoxide atmosphere, for example, in the presence of a transition metal catalyst, a base and alcohol.

In this step, the pressure of the carbon monoxide is generally from 1 to 20 atmospheres, and preferably 1 to 10 atmospheres.

Examples of the alcohol may include methanol, ethanol, propanol, isopropanol, diethylaminoethanol, isobutanol, 4-(2-hydroxyethyl)morpholine, 3-morpholinopropanol, and diethylaminopropanol.

The alcohol is used in an amount of generally approximately 1 to 100 moles, and preferably approximately 1 to 50 moles, based on the amount of the compound represented by the formula 4 (1 mole).

Examples of the transition metal catalyst used herein may include palladium catalysts (e.g., palladium acetate, palladium chloride, tetrakistriphenylphosphine palladium, palladium carbon, etc.). A ligand (e.g., triphenylphosphine, tri-tert-butylphosphine, etc.) may be added, as necessary. The amount of the transition metal catalyst used is different depending on the type of the catalyst. The transition metal catalyst is used in an amount of generally approximately 0.0001 to 1 mole, and preferably approximately 0.01 to 0.5 moles, based on the amount of the compound 4 (1 mole). The ligand is used in an amount of generally approximately 0.0001 to 4 moles, and preferably approximately 0.01 to 2 moles, based on the amount of the compound represented by the formula 4 (1 mole).

Examples of the base may include organic amines (e.g., trimethylamine, triethylamine, diisopropylethylamine, N-methylmorpholine, 1,8-diazabicyclo[5,4,0]undec-7-ene, pyridine, N,N-dimethylaniline, etc.), alkaline metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (e.g., potassium hydride, sodium hydride, etc.), alkaline metal alkoxides (e.g., sodium methoxide, sodium ethoxide, sodium-tert-butoxide, potassium-tert-butoxide, etc.), and alkaline metal disilazides (e.g., lithium disilazide, sodium disilazide, potassium disilazide, etc.). Among others, alkaline metal salts such as potassium carbonate, cesium carbonate, sodium phosphate, and potassium phosphate, alkaline metal alkoxides such as sodium-tert-butoxide and potassium-tert-butoxide, organic amines such as triethylamine and diisopropylethylamine, and the like are preferable. The base is used in an amount of generally approximately 0.1 to 50 moles, and preferably approximately 1 to 20 moles, based on the amount of the compound represented by the formula 4 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, N-methylpyrrolidone, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 150° C.

After completion of this reaction, an ester form corresponding to the used alcohol, or a mixture of the ester form and the compound represented by the formula 5 is subjected to a hydrolysis reaction, so that it can be converted to the compound represented by the formula 5.

As such a base, sodium hydrogen carbonate, sodium carbonate, potassium carbonate, cesium carbonate, sodium hydroxide, potassium hydroxide, lithium hydroxide, or the like is preferably used. The base is used in an amount of generally approximately 0.5 to 100 moles, and preferably approximately 1 to 10 moles, based on the amount of the compound represented by the formula 4 (1 mole).

The solvent is not particularly limited, as long as it does not affect the reaction. For example, water, methanol, ethanol, isopropanol, tetrahydrofuran, 1,4-dioxane, N,N-dimethylformamide and the like can be used alone or in combination. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compound represented by the formula 5 can be isolated and purified by known separation and purification means, or can be subjected to the subsequent step without isolation and purification.

< Step 4 >

This step is a method of obtaining a compound represented by the formula 6 (wherein $P_2$ represents a protective group for a carboxyl group) by introducing a protective group into the compound represented by the formula 5. Protection can be carried out according to a generally known method, for example, the method described in Protective Groups in Organic Synthesis third edition, T. W. Greene and P. G. M. Wuts, John Wiley & Sons (1999), or a method equivalent thereto.

The "protective group for a carboxyl group" is not particularly limited, as long as it has a protective function. Examples of the protective group for a carboxyl group may include: lower alkyl groups, such as, for example, a methyl group, an ethyl group, a propyl group, an isopropyl group, and a tert-butyl group; halo lower alkyl groups, such as, for example, a 2,2,2-trichloroethyl group; lower alkenyl groups, such as, for example, an allyl group; for example, a trimethylsilylethoxymethyl group; and aralkyl groups, such as, for example, a benzyl group, a p-methoxybenzyl group, a p-nitrobenzyl group, a benzhydryl group, and a trityl group. In particular, a methyl group, an ethyl group, a tert-butyl group, an allyl group, a benzyl group, a p-methoxybenzyl group, or a trimethylsilylethoxymethyl group is preferable.

In the present reaction, a protective group such as, for example, a tert-butyl ester group, a methyl ester group, or an ethyl ester group, is preferably introduced.

The protective group agent used in the present reaction may be, for example, 2-tert-butyl-1,3-diisopropylisourea. Such a protective group agent is used in an amount of generally approximately 1 to 50 moles, and preferably approximately 1 to 10 moles, based on the amount of the compound represented by the formula 5 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, tert-butyl methyl ether, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compound represented by the formula 6 can be isolated and purified by known separation and purification means, or can be subjected to the subsequent step without isolation and purification.

< Step 5 >

This step is a method of obtaining a compound represented by the formula 7 (wherein $L_3$ represents a halogen atom) by halogenating the compound represented by the formula 6. Halogenation can be carried out by a method using fluorine, chlorine, bromine, iodine, etc., or by a method using N-chlorosuccinimide, N-bromosuccinimide, N-iodosuccinimide, etc. In the present reaction, the method using N-chlorosuccinimide, N-bromosuccinimide, N-iodosuccinimide, etc. is preferable.

N-chlorosuccinimide, N-bromosuccinimide, N-iodosuccinimide, etc. can be used in an amount of 1 to 10 equivalents, and preferably 1 to 3 equivalents, based on the amount of the compound represented by the formula 6 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 100°.

The thus obtained compound represented by the formula 7 can be isolated and purified by known separation and purification means, or can be subjected to the subsequent step without isolation and purification.

< Step 6 >

This step is a method of obtaining a compound represented by the formula 8 by removing the protective group for an amino group (Pi in the formula 7) from the compound represented by the formula 7 (deprotection). Such deprotection can be carried out according to a generally known method, for example, the method described in Protective Groups in Organic Synthesis third edition, T. W. Greene and P. G. M. Wuts, John Wiley & Sons (1999), or a method equivalent thereto.

The protective group may be, for example, tert-butyloxycarbonyl. When such a tert-butyloxycarbonyl group is used, for example, as a protective group, deprotection is preferably carried out under acidic conditions. Examples of the acid used herein may include hydrochloric acid, acetic acid, trifluoroacetic acid, sulfuric acid, and tosylic acid.

The acid is preferably used in an amount of approximately 1 to 100 equivalents based on the amount of the compound represented by the formula 7 (1 mole).

The solvent used in the reaction is not particularly limited, as long as it does not affect the reaction. Examples of the solvent used herein may include alcohols (e.g., methanol, etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile, etc.), ethers (e.g., dimethoxyethane, tetrahydrofuran, etc.), aprotic polar solvents (e.g., N,N-dimethylformamide, dimethyl sulfoxide, hexamethylphosphoramide, etc.), and mixtures thereof. The reaction time is 0.1 to 100 hours, and preferably 0.5 to 24 hours. Thereafter, the reaction temperature is 0°C to 100°C, and preferably 0°C to 50°.

The thus obtained compound represented by the formula 8 can be isolated and purified by known separation and purification means, or can be subjected to the subsequent step without isolation and purification.

< Step 7 >

This step is a method of obtaining a compound represented by the formula 9 by performing an amidation reaction between an amino group of the compound represented by the formula 8 and acrylic acid halide or acrylic acid anhydride.

In the case of using acrylic acid halide or acrylic acid anhydride, such acrylic acid halide or acrylic acid anhydride is used in an amount of generally approximately 0.5 to 10 moles, and preferably approximately 1 to 5 moles, based on the amount of the compound represented by the formula 8 (1 mole). It is to be noted that the present acrylic acid halide or acrylic acid anhydride can be obtained as a commercially available product or can be produced according to a known method.

In addition, a base can be added, as necessary. Examples of the base may include organic amines (e.g., trimethylamine, triethylamine, isopropylethylamine, diisopropylethylamine, N-methylmorpholine, 1,8-diazabicyclo[5,4,0]undec-7-ene, pyridine, N,N-dimethylaniline, etc.), alkaline metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (e.g., potassium hydride, sodium hydride, etc.), and alkaline metal alkoxides (e.g., sodium methoxide, sodium ethoxide, sodium-tert-butoxide, potassium-tert-butoxide, etc.). The base is used in an amount of generally approximately 1 to 100 moles, and preferably approximately 1 to 10 moles, based on the amount of the compound represented by the formula 8 (1 mole).

The solvent used in the reaction is not particularly limited, as long as it does not affect the reaction. Examples

of the solvent used herein may include alcohols (e.g., methanol, etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile, etc.), ethers (e.g., dimethoxyethane, tetrahydrofuran, etc.), aprotic polar solvents (e.g., N,N-dimethylformamide, dimethyl sulfoxide, hexamethylphosphoramide, , etc.), and mixtures thereof. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compound represented by the formula 9 can be isolated and purified by known separation and purification means, or can be subjected to the subsequent step without isolation and purification.

< Step 8 >

This step is a method of obtaining a compound represented by the formula 10 by performing a Sonogashira reaction between the compound represented by the formula 9 and an acetylene derivative that is a commercially available product or can be produced by a known method.

The acetylene derivative can be used in an amount of 1 to 50 equivalents, and preferably 1 to 10 equivalents, based on the amount of the compound represented by the formula 9 (1 mole).

Examples of the transition metal catalyst used herein may include palladium catalysts (e.g., palladium acetate, palladium chloride, tetrakistriphenylphosphinepalladium, dichlorobis(triphenylphosphine)palladium, dichlorobis(triphenylphosphine)dipalladium, etc.), and nickel catalysts (e.g., nickel chloride, etc.). As necessary, a ligand (e.g., triphenylphosphine, tri-tert-butylphosphine, etc.) may be added, and a copper catalyst (e.g., copper iodide, copper bromide, or copper chloride) or the like may be used as a co-catalyst. The amount of the transition metal catalyst used is different depending on the type of the catalyst. The transition metal catalyst is used in an amount of generally approximately 0.0001 to 1 mole, and preferably approximately 0.01 to 0.5 moles, based on the amount of the compound represented by the formula 9 (1 mole). The ligand is used in an amount of generally approximately 0.0001 to 4 moles, and preferably approximately 0.01 to 2 moles, based on the amount of the compound represented by the formula 9 (1 mole). The copper catalyst is used in an amount of generally approximately 0.0001 to 4 moles, and preferably approximately 0.010 to 2 moles, based on the amount of the compound represented by the formula 9 (1 mole).

Examples of the base may include organic amines (e.g., trimethylamine, triethylamine, diisopropylethylamine, N-methylmorpholine, 1,8-diazabicyclo[5,4,0]undec-7-ene, pyridine, N,N-dimethylaniline, etc.), alkaline metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (e.g., potassium hydride, sodium hydride, etc.), alkaline metal alkoxides (e.g., sodium methoxide, sodium ethoxide, sodium-tert-butoxide, potassium-tert-butoxide, etc.), and alkaline metal disilazide (e.g., lithium disilazide, sodium disilazide, potassium disilazide, etc.). Among these, preferred examples of the base may include: alkaline metal salts, such as potassium carbonate, cesium carbonate, sodium phosphate, and potassium phosphate; alkaline metal alkoxides, such as sodium-tert-butoxide and potassium-tert-butoxide; and organic amines, such as triethylamine and diisopropylethylamine. The base is used in an amount of generally approximately 0.1 to 10 moles, and preferably approximately 1 to 5 moles, based on the amount of the compound represented by the formula 9 (1 mole).

The solvent is not particularly limited, as long as it does not affect the reaction. Examples of the solvent may include hydrocarbons (e.g., benzene, toluene, xylene, etc.), halogenated hydrocarbons (e.g., chloroform, 1,2-dichloroethane, etc.), nitriles (e.g., acetonitrile, etc.), ethers (e.g., dimethoxyethane, tetrahydrofuran, etc.), alcohols (e.g., methanol, ethanol, etc.), aprotic polar solvents (e.g., N,N-dimethylformamide, dimethyl sulfoxide, hexamethylphosphoramide, etc.), water, and mixtures thereof. The reaction time is 0.1 to 100 hours, and preferably 0.5 to 24 hours. Thereafter, the reaction temperature is 0°C to the temperature at which the solvent is boiled, and preferably 0°C to 150° C.

The thus obtained compound represented by the formula 10 can be isolated and purified by known separation and purification means, or can be subjected to the subsequent step without isolation and purification.

< Step 9 >

This step is a method of obtaining a compound represented by the formula 11 by deprotecting the protective group for a carboxyl group ($P_2$ in the formula 10) of the compound represented by the formula 10. Deprotection can be carried out according to a generally known method, for example, the method described in Protective Groups in Organic Synthesis third edition, T. W. Greene and P. G. M. Wuts, John Wiley & Sons (1981), or a method equivalent thereto.

The protective group may be, for example, tert-butyl ester. When such a tert-butyl ester group is used as a protective group, for example, deprotection is preferably carried out under acidic conditions. Examples of the acid used herein may include hydrochloric acid, acetic acid, trifluoroacetic acid, sulfuric acid, and tosylic acid.

The acid is preferably used in an amount of approximately 1 to 100 equivalents based on the amount of the compound represented by the formula 10 (1 mole).

The solvent used in the reaction is not particularly limited, as long as it does not affect the reaction. Examples of the solvent used herein may include alcohols (e.g., methanol, etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile, etc.), ethers (e.g., dimethoxyethane, tetrahydrofuran, etc.), aprotic polar solvents (e.g., N,N-dimethylformamide, dimethyl sulfoxide, hexamethylphosphoramide, , etc.), and mixtures thereof. The reaction time is 0.1 to 100 hours, and preferably 0.5 to 24 hours. Thereafter, the reaction temperature is 0°C to 100°C, and preferably 0°C to 50°.

The thus obtained compound represented by the formula 11 can be isolated and purified by known separation and purification means, or can be subjected to the subsequent step without isolation and purification.

< Step 10 >

This step is a method of obtaining a compound represented by the formula (I) by performing an amidation reaction between a carboxyl group of the compound represented by the formula 11 and an amine that is a commercially available product or can be produced by a known method.

Amidation can be carried out according to a conventionally known method. Examples of the amidation method may include a method of performing the reaction in the presence of a condensing agent, and a method comprising activating a carboxylic acid portion according to a conventionally known method to obtain a reactive derivative, and then performing amidation between the derivative and an amine (for both methods, see "Peptide Gosei no Kiso to Jikken (Principle of Peptide Synthesis and Experiments)" (Nobuo IZUMIYA et al., Maruzen Co., Ltd., 1983)).

Examples of the condensing agent may include N,N'-dicyclohexylcarbodiimide (DCC), N,N'-diisopropylcarbodiimide (DIC), 1-ethyl-3-(3-dimethylaminopropyl)carbodiimide hydrochloride (WSC), diphenylphosphoryl azide (DPPA), benzotriazol-1-yl-oxytrisdimethylaminophosphonium hexafluorophosphate (BOP), benzotriazol-1-yl-oxytripyrrolidinophosphonium hexafluorophosphate (PyBOP), 7-azabenzotriazol-1-yloxytrispyrrolidinophosphonium phosphate (PyAOP), bromotrispyrrolidinophosphonium hexafluorophosphate (BroP), chlorotris(pyrrolidin-1-yl)phosphonium hexafluorophosphate (PyCroP), 3-(diethoxyphosphoryloxy)-1,2,3-benzotriazin-4(3H)-one (DEPBT), O-(azabenzotriazol-1-yl)-N,N,N',N'-tetramethyluronium hexafluorophosphate (HATU), and 4-(5,6-dimethoxy-1,3,5-triazin-2-yl)-4-methylmorpholine hydrochloride (DMTMM). Examples of the additive used at that time may include 1-hydroxybenzotriazole (HOBt), 1-hydroxy-7-azabenzotriazole (HOAt), and N-hydroxysuccinimide (HOSu). Such agents are used in an amount of generally approximately 1 to 100 moles, and preferably approximately 1 to 10 moles, based on the amount of the compound represented by the formula 11 (1 mole).

In addition, as necessary, a base can be added. Examples of such a base may include organic amines (e.g., trimethylamine, triethylamine, diisopropylethylamine, N-methylmorpholine, 1,8-diazabicyclo[5,4,0]undec-7-ene, pyridine, N,N-dimethylaniline, etc.), alkaline metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide, etc.), metal hydrides (e.g., potassium hydride, sodium hydride, etc.), and alkaline metal alkoxides (e.g., sodium methoxide, sodium ethoxide, sodium-tert-butoxide, potassium-tert-butoxide, etc.). The base is used in an amount of generally approximately 1 to 100 moles, and preferably approximately 1 to 10 moles, based on the amount of the compound represented by the formula 11 (1 mole).

The solvent used in the reaction is not particularly limited, as long as it does not affect the reaction. Examples of the solvent used herein may include alcohols (e.g., methanol, etc.), hydrocarbons (e.g., benzene, toluene, xylene, etc.), halogenated hydrocarbons (e.g., methylene chloride, chloroform, 1,2-dichloroethane, etc.), nitriles (e.g., acetonitrile, etc.), ethers (e.g., dimethoxyethane, tetrahydrofuran, etc.), aprotic polar solvents (e.g., N,N-dimethylformamide, dimethyl sulfoxide, hexamethylphosphoramide, , etc.), and mixtures thereof. The reaction time is 0.1 to 100 hours, and preferably 0.5 to 24 hours. Thereafter, the reaction temperature is 0°C to the temperature at which the solvent is boiled, and preferably 0°C to 100° C.

The thus obtained compounds (I) and (II) can be isolated and purified according to known separation and purification means, such as, for example, concentration , vacuum concentration, crystallization, solvent extraction, reprecipitation, or chromatography.

In the above-described production method, the steps ranging from the "introduction of a protective group into a carboxyl group of the compound represented by the formula 5" (Step 4) to the "amidation reaction between a carboxyl group of the compound represented by the formula 11 and an amine that is a commercially available product or can be produced by a known method" (Step 10) are successively carried out in this order. However, the order of performing these steps can be changed. Moreover, the "introduction of a protective group into a carboxyl group of the compound represented by the formula 5" (Step 4) and the "removal of the protective group for a carboxy group from the compound represented by the formula 10" (Step 9) can be omitted.

Specifically, individual steps are carried out in the order of the "amidation reaction between a carboxyl group of the compound represented by the formula 11 and an amine that is a commercially available product or can be produced by a known method" (Step 10), the "halogenation of the compound represented by the formula 6" (Step 5), the "removal

of the protective group for an amino group from the compound represented by the formula 7" (Step 6), the "amidation reaction between an amino group of the compound represented by the formula 8 and acrylic acid halide or acrylic acid anhydride" (Step 7), and the "Sonogashira reaction between the compound represented by the formula 9 and an acetylene derivative that is a commercially available product or can be produced by a known method, when L3 of the compound represented by the formula 9 has a leaving group such as halogen" (Step 8), so that the concerned compound can be induced to the compounds represented by the formulae (I) and (II). The conditions applied in individual steps are the same as those as described above.

When the compound of the present invention has an isomer, such as an optical isomer, a stereoisomer, a rotational isomer, or a tautomer, all of these isomers or mixtures thereof are included in the compound of the present invention, unless otherwise stated. For example, when the compound of the present invention has an optical isomer, both a racemate, and an optical isomer obtained as a result of racemic resolution are included in the compound of the present invention, unless otherwise stated.

The salt of the compound of the present invention means a pharmaceutically acceptable salt, and it may be, for example, a base-added salt or an acid-added salt.

The pyrimidine compound of the present invention or a salt thereof also includes a prodrug. The "prodrug" means a compound that is converted to the compound of the present invention or a salt thereof as a result of the reaction with an enzyme, stomach acid, etc. under physiological conditions in a living body; namely, a compound that enzymatically causes oxidation, reduction, hydrolysis, etc., so that it is converted to the compound of the present invention or a salt thereof, or a compound that causes hydrolysis, etc. with stomach acid or the like, so that it is converted to the compound of the present invention or a salt thereof. Otherwise, it may also be a compound that is converted to the compound of the present invention or a salt thereof under physiological conditions as described in "Iyakuhin no Kaihatsu (Development of Pharmaceutical Products)," Hirokawa Shoten, 1990, Vol. 7*,* Bunshi Sekkei (Molecular Designing), pp. 163 to 198.

The pyrimidine compound of the present invention or a salt thereof may be an amorphous material or a crystal. Although the crystal form thereof may be a single crystal or a polymorphic mixture, they are included in the compound of the present invention or a salt thereof. The crystal can be produced by crystallizing the compound of the present invention or a salt thereof, applying a known crystallization method. The compound of the present invention or a salt thereof may be either a solvate (e.g., a hydrate, etc.), or a non-solvate, and both of them are included in the compound of the present invention or a salt thereof. Compounds labeled with radioisotopes (e.g., $^{3}H$, $^{14}C$, $^{35}S$, $^{125}I$, etc.) and the like are also included in the compound of the present invention or a salt thereof.

The compound of the present invention or a salt thereof has excellent EGFR inhibitory activity. Accordingly, the compound of the present invention or a salt thereof is useful as an antitumor agent against malignant tumor having EGFR overexpression, EGFR gene amplification, or EGFR mutation, etc. In addition, since significant weight reduction was not found in mice, the present compound or a salt thereof is advantageous in that it has a few side effects.

In the present description, the term "EGFR" includes the EGFR of a human or a non-human mammal, and it is preferably human EGFR. The NCBI Gene ID for the human EGFR is 1956. Furthermore, the term "EGFR" includes isoforms.

Since the compound of the present invention or a salt thereof has excellent EGFR inhibitory activity, it is useful as a medicament for preventing or treating disease associated with EGFR.

The "disease associated with EGFR" means disease, in which a reduction in the incidence, or the remission, alleviation and/or complete recovery of the symptoms thereof is achieved by deleting, suppressing and/or inhibiting the function of EGFR. Examples of such disease may include malignant tumors, but are not limited thereto. Preferred examples of the disease may include malignant tumors having EGFR overexpression, EGFR gene amplification, or EGFR mutation.

One embodiment of the present invention provides a therapeutic agent for a disease associated with EGFR, comprising the compound of the present invention or a salt thereof. One embodiment of the present invention provides an EGFR inhibitor comprising the compound of the present invention or a salt thereof. One embodiment of the present invention provides a therapeutic agent for EGFR-positive tumor, comprising the compound of the present invention or a salt thereof. One embodiment of the present invention provides the compound of the present invention or a salt thereof for treating a disease associated with EGFR. One embodiment of the present invention provides use of the compound of the present invention or a salt thereof for treating a disease associated with EGFR. One embodiment of the present invention provides use of the compound of the present invention or a salt thereof for producing a therapeutic agent for a disease associated with EGFR. One embodiment of the present invention provides a method for treating a disease associated with EGFR, comprising administering an effective amount of the compound of the present invention or a salt thereof to a subject in need thereof. One embodiment of the present invention provides the compound of the present invention or a salt thereof for treating EGFR-positive tumor. One embodiment of the present invention provides use of the compound of the present invention or a salt thereof for treating EGFR-positive tumor. One embodiment of the present invention provides use of the compound of the present invention or a salt thereof for producing a therapeutic agent for EGFR-positive tumor. One embodiment of the present invention provides a method for treating EGFR-positive tumor,

comprising administering an effective amount of the compound of the present invention or a salt thereof to a subject in need thereof.

The compound according to one embodiment of the present invention or a salt thereof inhibits wild-type EGFR, and mutant EGFR having an insertion mutation, a point mutation, or a deletion mutation, etc. One embodiment of the present invention provides a compound having inhibitory activity against wild-type EGFR and mutant EGFR, or a salt thereof, or a medicament or a pharmaceutical composition comprising the same. One embodiment of the present invention provides an inhibitor for wild-type EGFR and mutant EGFR, comprising the compound of the present invention or a salt thereof. One embodiment of the present invention provides a method for inhibiting wild-type EGFR and mutant EGFR, comprising administering an effective amount of the compound of the present invention or a salt thereof to a subject in need thereof. One embodiment of the present invention provides use of the compound of the present invention or a salt thereof for producing an inhibitor for wild-type EGFR and mutant EGFR. One embodiment of the present invention provides the compound of the present invention or a salt thereof for use as an inhibitor for wild-type EGFR and mutant EGFR. One embodiment of the present invention provides use of the compound of the present invention or a salt thereof for inhibiting wild-type EGFR and mutant EGFR. Another embodiment of the present invention provides use of the compound of the present invention or a salt thereof for preventing or treating diseases associated with wild-type EGFR and mutant EGFR.

The human wild-type EGFR gene is shown in, for example, SEQ ID NO: 1. The human wild-type EGFR protein consists of the amino acid sequence set forth in, for example, SEQ ID NO: 2. The nucleotide sequence information of the human wild-type EGFR gene and the amino acid sequence information of the human wild-type EGFR protein can be obtained from, for example, NCBI Reference Sequence: NM_005228 and NCBI Reference Sequence: NP_005219, respectively.

In several embodiments, the pyrimidine compound of the present invention or a salt thereof exhibits inhibitory activity against mutant EGFR. In the present description, "mutant EGFR" is EGFR having one or more activating mutations or resistance acquiring mutations, such as insertion mutations point mutations, or deletion mutations, in exon 18 region, exon 19 region, exon 20 region, exon 21 region or the like of human wild-type EGFR.

In the present description, "exon 18" corresponds to a region from positions 688 to 728 in the amino acid sequence of the human wild-type EGFR protein (e.g., a protein consisting of the amino acid sequence set forth in SEQ ID NO: 2).

In the present invention, "exon 18 mutation" refers to a point mutation or a deletion mutation in exon 18 region resulting in an amino acid mutation in the human wild-type EGFR protein (e.g., a protein consisting of the amino acid sequence set forth in SEQ ID NO: 2). Examples of the point mutation in exon 18 include point mutation E709X or G719X in exon 18 region, which substitutes glutamic acid at position 709 or glycine at position 719 with any amino acid. Examples of E709X include point mutation E709K in exon 18 region, which substitutes glutamic acid at position 709 with lysine, and point mutation E709A in exon 18 region, which substitutes glutamic acid at position 709 with alanine. Examples of G719X include point mutation G719A in exon 18 region, which substitutes glycine at position 719 with alanine, point mutation G719S in exon 18 region, which substitutes glycine at position 719 with serine, and point mutation G719C in exon 18 region, which substitutes glycine at position 719 with cysteine. The deletion mutation in exon 18 region encompasses not only a mutation in exon 18 region, which deletes some amino acids, but also a mutation therein, which inserts any one or more amino acids in addition to the amino acid deletion. Examples of the deletion mutation in exon 18 include a mutation in exon 18 region, which deletes glutamic acid at position 709 and threonine at position 710 and then inserts aspartic acid (Del E709-T710insD).

In the present description, "exon 19" corresponds to a region from positions 729 to 761 in the amino acid sequence of the human wild-type EGFR protein (e.g., a protein consisting of the amino acid sequence set forth in SEQ ID NO: 2).

In the present description, "exon 19 mutation" refers to a mutation in exon 19 region, which deletes one or more amino acids in the human wild-type EGFR protein (e.g., a protein consisting of the amino acid sequence set forth in SEQ ID NO: 2). The deletion mutation in exon 19 region encompasses not only a mutation in exon 19 region, which deletes some amino acids, but also a mutation therein, which inserts any one or more amino acids in addition to the amino acid deletion. Examples of the exon 19 deletion mutation include a mutation in exon 19 region, which deletes 5 amino acids from glutamic acid at position 746 to alanine at position 750 (Del E746-A750 (or also referred to as d746-750)), a mutation in exon 19 region, which deletes 7 amino acids from leucine at position 747 to proline at position 753 and then inserts serine (Del L747-P753insS), a mutation in exon 19 region, which deletes 5 amino acids from leucine at position 747 to threonine at position 751 (Del L747-T751), and a mutation in exon 19 region, which deletes 4 amino acids from leucine at position 747 to alanine at position 750 and then inserts proline (Del L747-A750insP). In one preferred embodiment of the present invention, the exon 19 deletion mutation is a mutation in exon 19 region, which deletes 5 amino acids from glutamic acid at position 746 to alanine at position 750 (Del E746-A750).

In the present description, "exon 20" corresponds to a region from positions 762 to 823 in the amino acid sequence of the human wild-type EGFR protein (e.g., a protein consisting of the amino acid sequence set forth in SEQ

ID NO: 2).

In the present invention, "exon 20 mutation" refers to a point mutation, an insertion mutation, a deletion mutation, or the like in exon 20 region resulting in an amino acid mutation in the human wild-type EGFR protein (e.g., a protein consisting of the amino acid sequence set forth in SEQ ID NO: 2). Examples of the exon 20 mutation include A763insFQEA, A767insASV, S768dupSVD, V769insASV, D770insNPG, D770insSVD, and D773insNPH (Nature medicine,24,p638-646,2018). In one preferred embodiment of the present invention, the exon 20 mutation is one or more insertion mutations or point mutations selected from V769_D770insASV, D770_N771insNPG, D770_N771insSVD, H773_V774insNPH, and T790M.

In the present description, "exon 21" corresponds to a region from positions 824 to 875 in the amino acid sequence of the human wild-type EGFR protein (e.g., a protein consisting of the amino acid sequence set forth in SEQ ID NO: 2).

In the present invention, "exon 21 mutation" refers to a point mutation in exon 21 region resulting in an amino acid mutation in the human wild-type EGFR protein (e.g., a protein consisting of the amino acid sequence set forth in SEQ ID NO: 2). Examples of the point mutation in exon 21 include point mutations in exon 21 region, which substitute one amino acid and preferably include position mutation L858X or L861X in exon 21 region, which substitutes leucine at position 858 or leucine at position 861 with any amino acid. Examples of L858X include point mutation L858R in exon 21 region, which substitutes leucine at position 858 with arginine. Examples of L861X include point mutation L861Q in exon 21 region, which substitutes leucine at position 861 with glutamine. In one preferred embodiment of the present invention, the point mutation in exon 21 is L858R.

In several embodiments, with regard to a mutation in a certain EGFR isoform, even when position of the mutation is different from position of an amino acid shown in SEQ ID NO: 2 due to deletion or insertion of an amino acid(s), it is understood that the mutation is the same as the mutation at a position corresponding to position of the amino acid shown in SEQ ID NO: 2. Hence, for example, the threonine at position 790 in the EGFR shown in SEQ ID NO: 2 corresponds to threonine at position 745 in EGFR consisting of the amino acid sequence set forth in SEQ ID NO: 4. For example, the term "T790M" means that the threonine at position 790 in the EGFR shown in SEQ ID NO: 2 is mutated to methionine. Since such "T790M" is at a position corresponding to the amino acid at position 745 in EGFR consisting of the amino acid sequence set forth in SEQ ID NO: 4, "T745M" in the EGFR consisting of the amino acid sequence set forth in SEQ ID NO: 4 corresponds to "T790M" in the EGFR shown in SEQ ID NO: 2. For example, the threonine at position 790 in the EGFR shown in SEQ ID NO: 2 corresponds to threonine at position 523 in EGFR consisting of the amino acid sequence set forth in SEQ ID NO: 6. For example, the term "T790M" means that the threonine at position 790 in the EGFR shown in SEQ ID NO: 2 is mutated to methionine. Since such "T790M" is at a position corresponding to the amino acid at position 523 in EGFR consisting of the amino acid sequence set forth in SEQ ID NO: 6, "T523M" in the EGFR consisting of the amino acid sequence set forth in SEQ ID NO: 6 corresponds to "T790M" in the EGFR shown in SEQ ID NO: 2. Besides, the position of an amino acid in SEQ ID NO: 2 that corresponds to a certain amino acid in a certain EGFR isoform can be confirmed by Multiple Alignment of BLAST.

SEQ ID NOs: 1 to 6 are as described below.

EGFR variant 1

Nucleotide sequence (SEQ ID NO: 1)

ATGCGACCCTCCGGGACGG

CCGGGGCAGCGCTCCTGGCGCTGCTGGCTGCGCTCTGCCCGGCGAGTCGGGCTCTGGAGGAAAAGAAAGT

TTGCCAAGGCACGAGTAACAAGCTCACGCAGTTGGGCACTTTTGAAGATCATTTTCTCAGCCTCCAGAGG

ATGTTCAATAACTGTGAGGTGGTCCTTGGGAATTTGGAAATTACCTATGTGCAGAGGAATTATGATCTTT

CCTTCTTAAAGACCATCCAGGAGGTGGCTGGTTATGTCCTCATTGCCCTCAACACAGTGGAGCGAATTCC

TTTGGAAAACCTGCAGATCATCAGAGGAAATATGTACTACGAAAATTCCTATGCCTTAGCAGTCTTATCT

AACTATGATGCAAATAAAACCGGACTGAAGGAGCTGCCCATGAGAAATTTACAGGAAATCCTGCATGGCG

CCGTGCGGTTCAGCAACAACCCTGCCCTGTGCAACGTGGAGAGCATCCAGTGGCGGGACATAGTCAGCAG

TGACTTTCTCAGCAACATGTCGATGGACTTCCAGAACCACCTGGGCAGCTGCCAAAAGTGTGATCCAAGC
TGTCCCAATGGGAGCTGCTGGGGTGCAGGAGAGGAGAACTGCCAGAAACTGACCAAAATCATCTGTGCCC
AGCAGTGCTCCGGGCGCTGCCGTGGCAAGTCCCCCAGTGACTGCTGCCACAACCAGTGTGCTGCAGGCTG
CACAGGCCCCCGGGAGAGCGACTGCCTGGTCTGCCGCAAATTCCGAGACGAAGCCACGTGCAAGGACACC
TGCCCCCCACTCATGCTCTACAACCCCACCACGTACCAGATGGATGTGAACCCCGAGGGCAAATACAGCT
TTGGTGCCACCTGCGTGAAGAAGTGTCCCCGTAATTATGTGGTGACAGATCACGGCTCGTGCGTCCGAGC
CTGTGGGGCCGACAGCTATGAGATGGAGGAAGACGGCGTCCGCAAGTGTAAGAAGTGCGAAGGGCCTTGC
CGCAAAGTGTGTAACGGAATAGGTATTGGTGAATTTAAAGACTCACTCTCCATAAATGCTACGAATATTA
AACACTTCAAAAACTGCACCTCCATCAGTGGCGATCTCCACATCCTGCCGGTGGCATTTAGGGGTGACTC
CTTCACACATACTCCTCCTCTGGATCCACAGGAACTGGATATTCTGAAAACCGTAAAGGAAATCACAGGG
TTTTTGCTGATTCAGGCTTGGCCTGAAAACAGGACGGACCTCCATGCCTTTGAGAACCTAGAAATCATAC
GCGGCAGGACCAAGCAACATGGTCAGTTTTCTCTTGCAGTCGTCAGCCTGAACATAACATCCTTGGGATT
ACGCTCCCTCAAGGAGATAAGTGATGGAGATGTGATAATTTCAGGAAACAAAAATTTGTGCTATGCAAAT
ACAATAAACTGGAAAAAACTGTTTGGGACCTCCGGTCAGAAAACCAAAATTATAAGCAACAGAGGTGAAA
ACAGCTGCAAGGCCACAGGCCAGGTCTGCCATGCCTTGTGCTCCCCCGAGGGCTGCTGGGGCCCGGAGCC
CAGGGACTGCGTCTCTTGCCGGAATGTCAGCCGAGGCAGGGAATGCGTGGACAAGTGCAACCTTCTGGAG
GGTGAGCCAAGGGAGTTTGTGGAGAACTCTGAGTGCATACAGTGCCACCCAGAGTGCCTGCCTCAGGCCA
TGAACATCACCTGCACAGGACGGGGACCAGACAACTGTATCCAGTGTGCCCACTACATTGACGGCCCCCA
CTGCGTCAAGACCTGCCCGGCAGGAGTCATGGGAGAAAACAACACCCTGGTCTGGAAGTACGCAGACGCC
GGCCATGTGTGCCACCTGTGCCATCCAAACTGCACCTACGGATGCACTGGGCCAGGTCTTGAAGGCTGTC
CAACGAATGGGCCTAAGATCCCGTCCATCGCCACTGGGATGGTGGGGGCCCTCCTCTTGCTGCTGGTGGT
GGCCCTGGGGATCGGCCTCTTCATGCGAAGGCGCCACATCGTTCGGAAGCGCACGCTGCGGAGGCTGCTG
CAGGAGAGGGAGCTTGTGGAGCCTCTTACACCCAGTGGAGAAGCTCCCAACCAAGCTCTCTTGAGGATCT
TGAAGGAAACTGAATTCAAAAAGATCAAAGTGCTGGGCTCCGGTGCGTTCGGCACGGTGTATAAGGGACT
CTGGATCCCAGAAGGTGAGAAAGTTAAAATTCCCGTCGCTATCAAGGAATTAAGAGAAGCAACATCTCCG
AAAGCCAACAAGGAAATCCTCGATGAAGCCTACGTGATGGCCAGCGTGGACAACCCCCACGTGTGCCGCC
TGCTGGGCATCTGCCTCACCTCCACCGTGCAGCTCATCACGCAGCTCATGCCCTTCGGCTGCCTCCTGGA

CTATGTCCGGGAACACAAAGACAATATTGGCTCCCAGTACCTGCTCAACTGGTGTGTGCAGATCGCAAAG
GGCATGAACTACTTGGAGGACCGTCGCTTGGTGCACCGCGACCTGGCAGCCAGGAACGTACTGGTGAAAA
CACCGCAGCATGTCAAGATCACAGATTTTGGGCTGGCCAAACTGCTGGGTGCGGAAGAGAAAGAATACCA
TGCAGAAGGAGGCAAAGTGCCTATCAAGTGGATGGCATTGGAATCAATTTTACACAGAATCTATACCCAC
CAGAGTGATGTCTGGAGCTACGGGGTGACTGTTTGGGAGTTGATGACCTTTGGATCCAAGCCATATGACG
GAATCCCTGCCAGCGAGATCTCCTCCATCCTGGAGAAAGGAGAACGCCTCCCTCAGCCACCCATATGTAC
CATCGATGTCTACATGATCATGGTCAAGTGCTGGATGATAGACGCAGATAGTCGCCCAAAGTTCCGTGAG
TTGATCATCGAATTCTCCAAAATGGCCCGAGACCCCCAGCGCTACCTTGTCATTCAGGGGGATGAAAGAA
TGCATTTGCCAAGTCCTACAGACTCCAACTTCTACCGTGCCCTGATGGATGAAGAAGACATGGACGACGT
GGTGGATGCCGACGAGTACCTCATCCCACAGCAGGGCTTCTTCAGCAGCCCCTCCACGTCACGGACTCCC
CTCCTGAGCTCTCTGAGTGCAACCAGCAACAATTCCACCGTGGCTTGCATTGATAGAAATGGGCTGCAAA
GCTGTCCCATCAAGGAAGACAGCTTCTTGCAGCGATACAGCTCAGACCCCACAGGCGCCTTGACTGAGGA
CAGCATAGACGACACCTTCCTCCCAGTGCCTGAATACATAAACCAGTCCGTTCCCAAAAGGCCCGCTGGC
TCTGTGCAGAATCCTGTCTATCACAATCAGCCTCTGAACCCCGCGCCCAGCAGAGACCCACACTACCAGG
ACCCCCACAGCACTGCAGTGGGCAACCCCGAGTATCTCAACACTGTCCAGCCCACCTGTGTCAACAGCAC
ATTCGACAGCCCTGCCCACTGGGCCCAGAAAGGCAGCCACCAAATTAGCCTGGACAACCCTGACTACCAG
CAGGACTTCTTTCCCAAGGAAGCCAAGCCAAATGGCATCTTTAAGGGCTCCACAGCTGAAAATGCAGAAT
ACCTAAGGGTCGCGCCACAAAGCAGTGAATTTATTGGAGCATGA

Amino acid sequence (SEQ ID NO: 2)

MRPSGTAGAALLALLAALCPASRALEEKKVCQGTSNKLTQLGTFEDHFLSLQRMFNNCEVVLGNLEITYV
QRNYDLSFLKTIQEVAGYVLIALNTVERIPLENLQIIRGNMYYENSYALAVLSNYDANKTGLKELPMRNL
QEILHGAVRFSNNPALCNVESIQWRDIVSSDFLSNMSMDFQNHLGSCQKCDPSCPNGSCWGAGEENCQKL
TKIICAQQCSGRCRGKSPSDCCHNQCAAGCTGPRESDCLVCRKFRDEATCKDTCPPLMLYNPTTYQMDVN
PEGKYSFGATCVKKCPRNYVVTDHGSCVRACGADSYEMEEDGVRKCKKCEGPCRKVCNGIGIGEFKDSLS
INATNIKHFKNCTSISGDLHILPVAFRGDSFTHTPPLDPQELDILKTVKEITGFLLIQAWPENRTDLHAF
ENLEIIRGRTKQHGQFSLAVVSLNITSLGLRSLKEISDGDVIISGNKNLCYANTINWKKLFGTSGQKTKI

| |
|---|
| ISNRGENSCKATGQVCHALCSPEGCWGPEPRDCVSCRNVSRGRECVDKCNLLEGEPREFVENSECIQCHP<br>ECLPQAMNITCTGRGPDNCIQCAHYIDGPHCVKTCPAGVMGENNTLVWKYADAGHVCHLCHPNCTYGCTG<br>PGLEGCPTNGPKIPSIATGMVGALLLLLVVALGIGLFMRRRHIVRKRTLRRLLQERELVEPLTPSGEAPN<br>QALLRILKETEFKKIKVLGSGAFGTVYKGLWIPEGEKVKIPVAIKELREATSPKANKEILDEAYVMASVD<br>NPHVCRLLGICLTSTVQLITQLMPFGCLLDYVREHKDNIGSQYLLNWCVQIAKGMNYLEDRRLVHRDLAA<br>RNVLVKTPQHVKITDFGLAKLLGAEEKEYHAEGGKVPIKWMALESILHRIYTHQSDVWSYGVTVWELMTF<br>GSKPYDGIPASEISSILEKGERLPQPPICTIDVYMIMVKCWMIDADSRPKFRELIIEFSKMARDPQRYLV<br>IQGDERMHLPSPTDSNFYRALMDEEDMDDVVDADEYLIPQQGFFSSPSTSRTPLLSSLSATSNNSTVACI<br>DRNGLQSCPIKEDSFLQRYSSDPTGALTEDSIDDTFLPVPEYINQSVPKRPAGSVQNPVYHNQPLNPAPS<br>RDPHYQDPHSTAVGNPEYLNTVQPTCVNSTFDSPAHWAQKGSHQISLDNPDYQQDFFPKEAKPNGIFKGS<br>TAENAEYLRVAPQSSEFIGA |

EGFR variant 5

Nucleotide sequence (SEQ ID NO: 3)

ATGCGACCCTCCGGGACGG

CCGGGGCAGCGCTCCTGGCGCTGCTGGCTGCGCTCTGCCCGGCGAGTCGGGCTCTGGAGGAAAAGAAAGT

TTGCCAAGGCACGAGTAACAAGCTCACGCAGTTGGGCACTTTTGAAGATCATTTTCTCAGCCTCCAGAGG

ATGTTCAATAACTGTGAGGTGGTCCTTGGGAATTTGGAAATTACCTATGTGCAGAGGAATTATGATCTTT

CCTTCTTAAAGACCATCCAGGAGGTGGCTGGTTATGTCCTCATTGCCCTCAACACAGTGGAGCGAATTCC

TTTGGAAAACCTGCAGATCATCAGAGGAAATATGTACTACGAAAATTCCTATGCCTTAGCAGTCTTATCT

AACTATGATGCAAATAAAACCGGACTGAAGGAGCTGCCCATGAGAAATTTACAGGGCCAAAAGTGTGATC

CAAGCTGTCCCAATGGGAGCTGCTGGGGTGCAGGAGAGGAGAACTGCCAGAAACTGACCAAAATCATCTG

TGCCCAGCAGTGCTCCGGGCGCTGCCGTGGCAAGTCCCCCAGTGACTGCTGCCACAACCAGTGTGCTGCA

GGCTGCACAGGCCCCCGGGAGAGCGACTGCCTGGTCTGCCGCAAATTCCGAGACGAAGCCACGTGCAAGG

ACACCTGCCCCCCACTCATGCTCTACAACCCCACCACGTACCAGATGGATGTGAACCCCGAGGGCAAATA

CAGCTTTGGTGCCACCTGCGTGAAGAAGTGTCCCCGTAATTATGTGGTGACAGATCACGGCTCGTGCGTC

CGAGCCTGTGGGGCCGACAGCTATGAGATGGAGGAAGACGGCGTCCGCAAGTGTAAGAAGTGCGAAGGGC

CTTGCCGCAAAGTGTGTAACGGAATAGGTATTGGTGAATTTAAAGACTCACTCTCCATAAATGCTACGAA
TATTAAACACTTCAAAAACTGCACCTCCATCAGTGGCGATCTCCACATCCTGCCGGTGGCATTTAGGGGT
GACTCCTTCACACATACTCCTCCTCTGGATCCACAGGAACTGGATATTCTGAAAACCGTAAAGGAAATCA
CAGGGTTTTTGCTGATTCAGGCTTGGCCTGAAAACAGGACGGACCTCCATGCCTTTGAGAACCTAGAAAT
CATACGCGGCAGGACCAAGCAACATGGTCAGTTTTCTCTTGCAGTCGTCAGCCTGAACATAACATCCTTG
GGATTACGCTCCCTCAAGGAGATAAGTGATGGAGATGTGATAATTTCAGGAAACAAAAATTTGTGCTATG
CAAATACAATAAACTGGAAAAAACTGTTTGGGACCTCCGGTCAGAAAACCAAAATTATAAGCAACAGAGG
TGAAAACAGCTGCAAGGCCACAGGCCAGGTCTGCCATGCCTTGTGCTCCCCCGAGGGCTGCTGGGGCCCG
GAGCCCAGGGACTGCGTCTCTTGCCGGAATGTCAGCCGAGGCAGGGAATGCGTGGACAAGTGCAACCTTC
TGGAGGGTGAGCCAAGGGAGTTTGTGGAGAACTCTGAGTGCATACAGTGCCACCCAGAGTGCCTGCCTCA
GGCCATGAACATCACCTGCACAGGACGGGGACCAGACAACTGTATCCAGTGTGCCCACTACATTGACGGC
CCCCACTGCGTCAAGACCTGCCCGGCAGGAGTCATGGGAGAAAACAACACCCTGGTCTGGAAGTACGCAG
ACGCCGGCCATGTGTGCCACCTGTGCCATCCAAACTGCACCTACGGATGCACTGGGCCAGGTCTTGAAGG
CTGTCCAACGAATGGGCCTAAGATCCCGTCCATCGCCACTGGGATGGTGGGGGCCCTCCTCTTGCTGCTG
GTGGTGGCCCTGGGGATCGGCCTCTTCATGCGAAGGCGCCACATCGTTCGGAAGCGCACGCTGCGGAGGC
TGCTGCAGGAGAGGGAGCTTGTGGAGCCTCTTACACCCAGTGGAGAAGCTCCCAACCAAGCTCTCTTGAG
GATCTTGAAGGAAACTGAATTCAAAAAGATCAAAGTGCTGGGCTCCGGTGCGTTCGGCACGGTGTATAAG
GGACTCTGGATCCCAGAAGGTGAGAAAGTTAAAATTCCCGTCGCTATCAAGGAATTAAGAGAAGCAACAT
CTCCGAAAGCCAACAAGGAAATCCTCGATGAAGCCTACGTGATGGCCAGCGTGGACAACCCCCACGTGTG
CCGCCTGCTGGGCATCTGCCTCACCTCCACCGTGCAGCTCATCACGCAGCTCATGCCCTTCGGCTGCCTC
CTGGACTATGTCCGGGAACACAAAGACAATATTGGCTCCCAGTACCTGCTCAACTGGTGTGTGCAGATCG
CAAAGGGCATGAACTACTTGGAGGACCGTCGCTTGGTGCACCGCGACCTGGCAGCCAGGAACGTACTGGT
GAAAACACCGCAGCATGTCAAGATCACAGATTTTGGGCTGGCCAAACTGCTGGGTGCGGAAGAGAAAGAA
TACCATGCAGAAGGAGGCAAAGTGCCTATCAAGTGGATGGCATTGGAATCAATTTTACACAGAATCTATA
CCCACCAGAGTGATGTCTGGAGCTACGGGGTGACTGTTTGGGAGTTGATGACCTTTGGATCCAAGCCATA
TGACGGAATCCCTGCCAGCGAGATCTCCTCCATCCTGGAGAAAGGAGAACGCCTCCCTCAGCCACCCATA
TGTACCATCGATGTCTACATGATCATGGTCAAGTGCTGGATGATAGACGCAGATAGTCGCCCAAAGTTCC

GTGAGTTGATCATCGAATTCTCCAAAATGGCCCGAGACCCCCAGCGCTACCTTGTCATTCAGGGGGATGA
AAGAATGCATTTGCCAAGTCCTACAGACTCCAACTTCTACCGTGCCCTGATGGATGAAGAAGACATGGAC
GACGTGGTGGATGCCGACGAGTACCTCATCCCACAGCAGGGCTTCTTCAGCAGCCCCTCCACGTCACGGA
CTCCCCTCCTGAGCTCTCTGAGTGCAACCAGCAACAATTCCACCGTGGCTTGCATTGATAGAAATGGGCT
GCAAAGCTGTCCCATCAAGGAAGACAGCTTCTTGCAGCGATACAGCTCAGACCCCACAGGCGCCTTGACT
GAGGACAGCATAGACGACACCTTCCTCCCAGTGCCTGGTGAGTGGCTTGTCTGGAAACAGTCCTGCTCCT
CAACCTCCTCGACCCACTCAGCAGCAGCCAGTCTCCAGTGTCCAAGCCAGGTGCTCCCTCCAGCATCTCC
AGAGGGGGAAACAGTGGCAGATTTGCAGACACAGTGA

Amino acid sequence (SEQ ID NO: 4)

MRPSGTAGAALLALLAALCPASRALEEKKVCQGTSNKLTQLGTFEDHFLSLQRMFNNCEVVLGNLEITYV
QRNYDLSFLKTIQEVAGYVLIALNTVERIPLENLQIIRGNMYYENSYALAVLSNYDANKTGLKELPMRNL
QGQKCDPSCPNGSCWGAGEENCQKLTKIICAQQCSGRCRGKSPSDCCHNQCAAGCTGPRESDCLVCRKFR
DEATCKDTCPPLMLYNPTTYQMDVNPEGKYSFGATCVKKCPRNYVVTDHGSCVRACGADSYEMEEDGVRK
CKKCEGPCRKVCNGIGIGEFKDSLSINATNIKHFKNCTSISGDLHILPVAFRGDSFTHTPPLDPQELDIL
KTVKEITGFLLIQAWPENRTDLHAFENLEIIRGRTKQHGQFSLAVVSLNITSLGLRSLKEISDGDVIISG
NKNLCYANTINWKKLFGTSGQKTKIISNRGENSCKATGQVCHALCSPEGCWGPEPRDCVSCRNVSRGREC
VDKCNLLEGEPREFVENSECIQCHPECLPQAMNITCTGRGPDNCIQCAHYIDGPHCVKTCPAGVMGENNT
LVWKYADAGHVCHLCHPNCTYGCTGPGLEGCPTNGPKIPSIATGMVGALLLLLVVALGIGLFMRRRHIVR
KRTLRRLLQERELVEPLTPSGEAPNQALLRILKETEFKKIKVLGSGAFGTVYKGLWIPEGEKVKIPVAIK
ELREATSPKANKEILDEAYVMASVDNPHVCRLLGICLTSTVQLITQLMPFGCLLDYVREHKDNIGSQYLL
NWCVQIAKGMNYLEDRRLVHRDLAARNVLVKTPQHVKITDFGLAKLLGAEEKEYHAEGGKVPIKWMALES
ILHRIYTHQSDVWSYGVTVWELMTFGSKPYDGIPASEISSILEKGERLPQPPICTIDVYMIMVKCWMIDA
DSRPKFRELIIEFSKMARDPQRYLVIQGDERMHLPSPTDSNFYRALMDEEDMDDVVDADEYLIPQQGFFS
SPSTSRTPLLSSLSATSNNSTVACIDRNGLQSCPIKEDSFLQRYSSDPTGALTEDSIDDTFLPVPGEWLV
WKQSCSSTSSTHSAAASLQCPSQVLPPASPEGETVADLQTQ

EGFRvIII (del2-7 EGFR)

Nucleotide sequence (SEQ ID NO: 5)

ATGCGACCCTCCGGGACGG
CCGGGGCAGCGCTCCTGGCGCTGCTGGCTGCGCTCTGCCCGGCGAGTCGGGCTCTGGAGGAAAAGAAAGG
TAATTATGTGGTGACAGATCACGGCTCGTGCGTCCGAGCCTGTGGGGCCGACAGCTATGAGATGGAGGAA
GACGGCGTCCGCAAGTGTAAGAAGTGCGAAGGGCCTTGCCGCAAAGTGTGTAACGGAATAGGTATTGGTG
AATTTAAAGACTCACTCTCCATAAATGCTACGAATATTAAACACTTCAAAAACTGCACCTCCATCAGTGG
CGATCTCCACATCCTGCCGGTGGCATTTAGGGGTGACTCCTTCACACATACTCCTCCTCTGGATCCACAG
GAACTGGATATTCTGAAAACCGTAAAGGAAATCACAGGGTTTTTGCTGATTCAGGCTTGGCCTGAAAACA
GGACGGACCTCCATGCCTTTGAGAACCTAGAAATCATACGCGGCAGGACCAAGCAACATGGTCAGTTTTC
TCTTGCAGTCGTCAGCCTGAACATAACATCCTTGGGATTACGCTCCCTCAAGGAGATAAGTGATGGAGAT
GTGATAATTTCAGGAAACAAAAATTTGTGCTATGCAAATACAATAAACTGGAAAAAACTGTTTGGGACCT
CCGGTCAGAAAACCAAAATTATAAGCAACAGAGGTGAAAACAGCTGCAAGGCCACAGGCCAGGTCTGCCA
TGCCTTGTGCTCCCCCGAGGGCTGCTGGGGCCCGGAGCCCAGGGACTGCGTCTCTTGCCGGAATGTCAGC
CGAGGCAGGGAATGCGTGGACAAGTGCAACCTTCTGGAGGGTGAGCCAAGGGAGTTTGTGGAGAACTCTG
AGTGCATACAGTGCCACCCAGAGTGCCTGCCTCAGGCCATGAACATCACCTGCACAGGACGGGGACCAGA
CAACTGTATCCAGTGTGCCCACTACATTGACGGCCCCCACTGCGTCAAGACCTGCCCGGCAGGAGTCATG
GGAGAAAACAACACCCTGGTCTGGAAGTACGCAGACGCCGGCCATGTGTGCCACCTGTGCCATCCAAACT
GCACCTACGGATGCACTGGGCCAGGTCTTGAAGGCTGTCCAACGAATGGGCCTAAGATCCCGTCCATCGC
CACTGGGATGGTGGGGGCCCTCCTCTTGCTGCTGGTGGTGGCCCTGGGGATCGGCCTCTTCATGCGAAGG
CGCCACATCGTTCGGAAGCGCACGCTGCGGAGGCTGCTGCAGGAGAGGGAGCTTGTGGAGCCTCTTACAC
CCAGTGGAGAAGCTCCCAACCAAGCTCTCTTGAGGATCTTGAAGGAAACTGAATTCAAAAAGATCAAAGT
GCTGGGCTCCGGTGCGTTCGGCACGGTGTATAAGGGACTCTGGATCCCAGAAGGTGAGAAAGTTAAAATT
CCCGTCGCTATCAAGGAATTAAGAGAAGCAACATCTCCGAAAGCCAACAAGGAAATCCTCGATGAAGCCT
ACGTGATGGCCAGCGTGGACAACCCCCACGTGTGCCGCCTGCTGGGCATCTGCCTCACCTCCACCGTGCA
GCTCATCACGCAGCTCATGCCCTTCGGCTGCCTCCTGGACTATGTCCGGGAACACAAAGACAATATTGGC
TCCCAGTACCTGCTCAACTGGTGTGTGCAGATCGCAAAGGGCATGAACTACTTGGAGGACCGTCGCTTGG

TGCACCGCGACCTGGCAGCCAGGAACGTACTGGTGAAAACACCGCAGCATGTCAAGATCACAGATTTTGG
GCTGGCCAAACTGCTGGGTGCGGAAGAGAAAGAATACCATGCAGAAGGAGGCAAAGTGCCTATCAAGTGG
ATGGCATTGGAATCAATTTTACACAGAATCTATACCCACCAGAGTGATGTCTGGAGCTACGGGGTGACTG
TTTGGGAGTTGATGACCTTTGGATCCAAGCCATATGACGGAATCCCTGCCAGCGAGATCTCCTCCATCCT
GGAGAAAGGAGAACGCCTCCCTCAGCCACCCATATGTACCATCGATGTCTACATGATCATGGTCAAGTGC
TGGATGATAGACGCAGATAGTCGCCCAAAGTTCCGTGAGTTGATCATCGAATTCTCCAAAATGGCCCGAG
ACCCCCAGCGCTACCTTGTCATTCAGGGGGATGAAAGAATGCATTTGCCAAGTCCTACAGACTCCAACTT
CTACCGTGCCCTGATGGATGAAGAAGACATGGACGACGTGGTGGATGCCGACGAGTACCTCATCCCACAG
CAGGGCTTCTTCAGCAGCCCCTCCACGTCACGGACTCCCCTCCTGAGCTCTCTGAGTGCAACCAGCAACA
ATTCCACCGTGGCTTGCATTGATAGAAATGGGCTGCAAAGCTGTCCCATCAAGGAAGACAGCTTCTTGCA
GCGATACAGCTCAGACCCCACAGGCGCCTTGACTGAGGACAGCATAGACGACACCTTCCTCCCAGTGCCT
GAATACATAAACCAGTCCGTTCCCAAAAGGCCCGCTGGCTCTGTGCAGAATCCTGTCTATCACAATCAGC
CTCTGAACCCCGCGCCCAGCAGAGACCCACACTACCAGGACCCCCACAGCACTGCAGTGGGCAACCCCGA
GTATCTCAACACTGTCCAGCCCACCTGTGTCAACAGCACATTCGACAGCCCTGCCCACTGGGCCCAGAAA
GGCAGCCACCAAATTAGCCTGGACAACCCTGACTACCAGCAGGACTTCTTTCCCAAGGAAGCCAAGCCAA
ATGGCATCTTTAAGGGCTCCACAGCTGAAAATGCAGAATACCTAAGGGTCGCGCCACAAAGCAGTGAATT
TATTGGAGCATGA

Amino acid sequence (SEQ ID NO: 6)

MRPSGTAGAALLALLAALCPASRALEEKKGNYVVTDHGSCVRACGADSYEMEEDGVRKCKKCEGPCRKVC
NGIGIGEFKDSLSINATNIKHFKNCTSISGDLHILPVAFRGDSFTHTPPLDPQELDILKTVKEITGFLLI
QAWPENRTDLHAFENLEIIRGRTKQHGQFSLAVVSLNITSLGLRSLKEISDGDVIISGNKNLCYANTINW
KKLFGTSGQKTKIISNRGENSCKATGQVCHALCSPEGCWGPEPRDCVSCRNVSRGRECVDKCNLLEGEPR
EFVENSECIQCHPECLPQAMNITCTGRGPDNCIQCAHYIDGPHCVKTCPAGVMGENNTLVWKYADAGHVC
HLCHPNCTYGCTGPGLEGCPTNGPKIPSIATGMVGALLLLLVVALGIGLFMRRRHIVRKRTLRRLLQERE
LVEPLTPSGEAPNQALLRILKETEFKKIKVLGSGAFGTVYKGLWIPEGEKVKIPVAIKELREATSPKANK
EILDEAYVMASVDNPHVCRLLGICLTSTVQLITQLMPFGCLLDYVREHKDNIGSQYLLNWCVQIAKGMNY

| |
|---|
| LEDRRLVHRDLAARNVLVKTPQHVKITDFGLAKLLGAEEKEYHAEGGKVPIKWMALESILHRIYTHQSDV |
| WSYGVTVWELMTFGSKPYDGIPASEISSILEKGERLPQPPICTIDVYMIMVKCWMIDADSRPKFRELIIE |
| FSKMARDPQRYLVIQGDERMHLPSPTDSNFYRALMDEEDMDDVVDADEYLIPQQGFFSSPSTSRTPLLSS |
| LSATSNNSTVACIDRNGLQSCPIKEDSFLQRYSSDPTGALTEDSIDDTFLPVPEYINQSVPKRPAGSVQN |
| PVYHNQPLNPAPSRDPHYQDPHSTAVGNPEYLNTVQPTCVNSTFDSPAHWAQKGSHQISLDNPDYQQDFF |
| PKEAKPNGIFKGSTAENAEYLRVAPQSSEFIGA |

In the present invention, "EGFR-positive tumor" is tumor in which the EGFR protein or the EGFR gene is detected. The EGFR protein and the EGFR gene also include mutant EGFR protein and mutant EGFR gene having a point mutation, an insertion mutation, or a deletion mutation, etc.

Examples of the method for detecting the EGFR protein include usual detection methods commonly used, such as ELISA, Western blotting, and immunostaining using an antibody specifically binding to the EGFR protein. The antibody specifically binding to the EGFR protein may be a commercially available product or may be prepared by a usual method commonly used.

Examples of the method for detecting the EGFR gene include usual detection methods commonly used, such as Northern blotting, Southern blotting, RT-PCR, real-time PCR, digital PCR, DNA microarrays, *in situ* hybridization, and sequence analysis. Another example thereof includes a detection method using cobas EGFR mutation detection kit (Roche Diagnostics K.K.), which is a commercially available EGFR gene mutation detection kit.

In the present description, the term "effective amount" used regarding the pyrimidine compound of the present invention means the amount of the compound of the present invention that induces the biological or medical response of a subject, such as, for example, reduction or inhibition of enzyme or protein activity; or ameliorates symptoms, alleviates conditions, and retards or delays the progression of disease; or prevents disease; etc. (therapeutically effective amount).

In the present description, the term "subject" includes mammals and non-mammals. Examples of the mammal may include, but are not limited to, a human, a chimpanzee, an ape, a monkey, a bovine, a horse, sheep, a goat, a swine, a rabbit, a dog, a cat, a rat, a mouse, a Guinea pig, a hedgehog, a kangaroo, a mole, a wild pig, a bear, a tiger, and a lion. Examples of the non-mammal may include, but are not limited to, birds, fish, and reptiles. In one embodiment, the subject is a human, and may be a human who has been diagnosed to need the treatment for the symptoms, conditions or disease disclosed in the present description.

Upon the use of the compound of the present invention or a salt thereof as a medicament, a pharmaceutically acceptable carrier is mixed into it, as necessary, and various types of dosage forms can be adopted depending on the preventive or therapeutic purpose. Examples of the dosage form may include all of an oral agent, an injection, a suppository, an ointment, and a patch. Preferably, an oral agent is adopted. These dosage forms can be produced by commonly used production methods that are known to skilled persons.

One embodiment of the present invention provides an antitumor agent for oral administration, comprising the compound of the present invention or a salt thereof as an active ingredient. In addition, one embodiment of the present invention provides a method for preventing and/or treating tumor, comprising administering an effective amount of the compound of the present invention or a salt thereof to a subject in need thereof by oral administration. Moreover, one embodiment of the present invention provides use of the compound of the present invention or a salt thereof for the production of an antitumor agent for oral administration. Furthermore, one embodiment of the present invention provides the compound of the present invention or a salt thereof for use in the prevention and/or treatment of tumor by oral administration thereof.

One embodiment of the present invention provides a pharmaceutical composition comprising the compound of the present invention or a salt thereof. The pharmaceutical composition according to one embodiment of the present invention comprises the compound of the present invention or a salt thereof, and a pharmaceutically acceptable carrier. Further, one embodiment of the present invention provides use of the compound of the present invention or a salt thereof for the production of a pharmaceutical composition. Another embodiment of the present invention provides the compound of the present invention or a salt thereof for use as a medicament.

As pharmaceutically acceptable carriers, various types of organic or inorganic carrier substances, which are commonly used as preparation materials, are used. When the compound of the present invention is processed into a solid preparation, examples of the pharmaceutically acceptable carrier mixed into the compound of the present invention may include an excipient, a binder, a disintegrator, a lubricant, a coating agent, and a coloring agent. When the compound

of the present invention is processed into a liquid preparation, examples of the pharmaceutically acceptable carrier mixed into the compound of the present invention may include a solvent, a solubilizer, a suspending agent, a tonicity agent, a buffer, and a soothing agent. In addition, preparation additives such as an antiseptic, an antioxidant, a sweetener, and a stabilizer can also be used, as necessary.

In the case of preparing a solid preparation for oral administration, an excipient, and as necessary, a binder, a disintegrator, a lubricant, a coloring agent, a corrigent, etc. are added to the pyrimidine compound of the present invention, and thereafter, a tablet, a coated tablet, a granule, a powder agent, a capsule, etc. can be produced according to ordinary methods.

In the case of preparing an injection, a pH adjuster, a buffer, a stabilizer, a tonicity agent, a local anesthetic, etc. are added to the pyrimidine compound of the present invention, and thereafter, subcutaneous, intramuscular, and intravenous injections can be produced according to ordinary methods.

The amount of the compound of the present invention to be mixed into the above-described each dosage unit form depends on the symptoms of a subject to whom the present pyrimidine compound should be applied, the dosage form and the like, and thus, the amount of the compound of the present invention is not constant. In general, it is preferable that the applied dose is set to be 0.05 to 1000 mg per dosage unit form in the case of an oral agent, it is set to be 0.01 to 500 mg per dosage unit form in the case of an injection, and it is set to be 1 to 1000 mg per dosage unit form in the case of a suppository.

The daily dose of a drug having the above-described dosage form is different depending on the symptoms, body weight, age, sex and the like of a subject, and thus, it cannot be generally determined. However, the compound of the present invention may be administered to an adult (body weight: 50 kg) at a daily dose of generally 0.05 to 5000 mg, and preferably 0.1 to 1000 mg.

The malignant tumor that is the target of the present invention is not particularly limited. Examples of the tumor may include brain tumor, head and neck cancer, digestive cancer (esophageal cancer, stomach cancer, duodenal cancer, liver cancer, biliary tract cancer (gallbladder and/or bile duct cancer, etc.), pancreatic cancer, colorectal cancer (colon cancer, rectal cancer, etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary organ cancer (kidney cancer, bladder cancer, prostate cancer, testicular tumor, etc.), hematopoietic tumor (leukemia, malignant lymphoma, multiple myeloma, etc.), bone and/or soft tissue tumor, and skin cancer. Among these, preferable is lung cancer, breast cancer, stomach cancer, head and neck cancer, brain tumor, colorectal cancer, bladder cancer, biliary tract cancer or uterine cancer, and more preferable is lung cancer, breast cancer, colorectal cancer, or brain tumor.

In one embodiment, the tumor that is the target of the present invention is a malignant tumor having EGFR overexpression, EGFR gene amplification, or EGFR mutation. Specific examples of the tumor may include brain tumor, head and neck cancer, digestive cancer (esophageal cancer, stomach cancer, duodenal cancer, liver cancer, biliary tract cancer (gallbladder and/or bile duct cancer, etc.), pancreatic cancer, colorectal cancer (colon cancer, rectal cancer, etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary organ cancer (kidney cancer, bladder cancer, prostate cancer, testicular tumor, etc.), hematopoietic tumor (leukemia, malignant lymphoma, multiple myeloma, etc.), bone and/or soft tissue tumor, and skin cancer. Among these, preferable is lung cancer, breast cancer, stomach cancer, head and neck cancer, brain tumor, colorectal cancer, bladder cancer, biliary tract cancer or uterine cancer, and more preferable is lung cancer, breast cancer, colorectal cancer, or brain tumor.

In one embodiment, the tumor that is the target of the present invention is an EGFR positive tumor. Specific examples of the tumor may include brain tumor, head and neck cancer, digestive cancer (esophageal cancer, stomach cancer, duodenal cancer, liver cancer, biliary tract cancer (gallbladder and/or bile duct cancer, etc.), pancreatic cancer, colorectal cancer (colon cancer, rectal cancer, etc.), etc.), lung cancer (non-small cell lung cancer, small cell lung cancer, mesothelioma, etc.), breast cancer, genital cancer (ovarian cancer, uterine cancer (cervical cancer, endometrial cancer, etc.), etc.), urinary organ cancer (kidney cancer, bladder cancer, prostate cancer, testicular tumor, etc.), hematopoietic tumor (leukemia, malignant lymphoma, multiple myeloma, etc.), bone and/or soft tissue tumor, and skin cancer. Among these, preferable is lung cancer, breast cancer, stomach cancer, head and neck cancer, brain tumor, colorectal cancer, bladder cancer, biliary tract cancer or uterine cancer, and more preferable is lung cancer, breast cancer, colorectal cancer, or brain tumor.

In one embodiment, the tumor is a brain tumor. The pyrimidine compound of the present invention may be useful for the treatment of the symptoms of brain that is required to pass through the blood-brain barrier. The pyrimidine compound of one embodiment has favorable permeability through the blood-brain barrier for the delivery thereof into the brain, namely, excellent brain penetration properties. As an indicator of the penetration properties of the compound into the brain, the concentration of the compound in the brain or a Kp value (brain-to-plasma drug concentration ratio) is applied.

The brain tumor treated with the pyrimidine compound of the present invention includes metastatic brain tumor and primary brain tumor.

Examples of the brain tumor may include, but are not particularly limited to, metastatic brain tumor (e.g., brain metastasis of lung cancer, breast cancer, stomach cancer, colorectal cancer, bladder cancer, biliary tract cancer, uterine cancer, etc. (preferably, lung cancer, breast cancer, or stomach cancer)), piliocytic astrocytoma, diffuse astrocytoma, oligodendroma and/or oligodendroastrocytoma, anaplastic astrocytoma and/or anaplastic oligodendroglioma, anaplastic oligodendroastrocytoma, glioblastoma, ependymoma, anaplastic ependymoma, ganglioglioma, central neurocytoma, medulloblastoma, germinoma, central nervous system malignant lymphoma, meningioma, neurilemmoma, GH secreting pituitary adenoma, PRL-secreting pituitary adenoma, ACTH-secreting pituitary adenoma, nonfunctional pituitary adenoma, craniopharyngioma, chordoma, hemangioblastoma, and epidermoid tumor.

## EXAMPLES

Hereinafter, the present invention will be described in detail in the following examples. However, these examples are not intended to limit the scope of the present invention.

In the present description, "room temperature" generally means a temperature that is from approximately 10°C to approximately 35°C. In addition, in the following Examples regarding compounds, "%" indicates weight percent, unless otherwise specified.

Various types of reagents used in the Examples were commercially available products, unless otherwise specified. Silica gel chromatography was carried out using Biotage SNAP Cartridge Ultra, manufactured by Biotage Japan Ltd. Basic silica gel chromatography was carried out using Biotage SNAP Cartridge Isolute Flash-NH2, manufactured by Biotage Japan Ltd.

Preparative thin-layer chromatography was carried out using Kieselgel TM60F254, Art. 5744, manufactured by Merck, or NH2 Silica Gel 60F254 Plate-Wako, manufactured by FUJIFILM Wako Pure Chemical Industries, Ltd.

$^{1}$H-NMR was measured using tetramethylsilane as a reference material, and employing AL400 (400 MHz) manufactured by JEOL, Mercury (400 MHz) manufactured by Varian, or Inova (400 MHz) manufactured by Varian. Moreover, mass spectrum was measured using Micromass ZQ or SQD manufactured by Waters, according to electrospray ionization (ESI) or atmospheric pressure chemical ionization (APCI). Microwave reaction was carried out using Initiator manufactured by Biotage Japan Ltd.

Abbreviations have the following meanings.

s: Singlet
d: Doublet
t: Triplet
q: Quartet
dd: Double doublet
dt: Double triplet
td: Triple doublet
tt: Triple triplet
ddd: Double double doublet
ddt: Double double triplet
dtd: Double triple doublet
tdd: Triple double doublet
m: Multiplet
br: Broad
ATP: Adenosine triphosphate
DMSO-d6: Deuterated dimethyl sulfoxide
$CDCl_3$: Deuterated chloroform
EDTA: Ethylenediaminetetraacetic acid
THF: Tetrahydrofuran
DMF: N,N-dimethylformamide
DMSO: Dimethyl sulfoxide
NMP: N-methyl pyrrolidone
HATU: O-(7-azabenzotriazol-1-yl)-N,N,N',N'-tetramethyluronium hexafluorophosphate
HPMC: Hypromellose
$PdCl_2(PPh_3)_2$: Dichlorobis(triphenylphosphine)palladium(II)

< Reference Example 1 >

Reference Example 1(1)

tert-Butyl (2S,4R)-4-(4-amino-5-iodo-7H-pyrrolo[2,3-d1pyrimidin-7-yl)-2-methylpyrrolidine-1-carboxylate

tert-Butyl (2S,4S)-4-hydroxy-2-methylpyrrolidine-1-carboxylate (19.0 g) and 4-chloro-5-iodo-7H-pyrrolo[2,3-d]pyrimidine (13.1 g) were dissolved in THF (190 mL), and the obtained solution was then cooled to 0°C. Thereafter, triphenylphosphine (37.2 g) and diisopropyl azodicarboxylate (28.1 mL) were added to the reaction solution, and the temperature of the mixture was then increased to room temperature, followed by stirring for 1 hour. Thereafter, the reaction mixture was concentrated under reduced pressure, and the obtained residue was then purified by silica gel chromatography (hexane : ethyl acetate) to obtain the corresponding coupling body. The obtained compound was used in the subsequent reaction without being further purified.

The obtained coupling body, THF (114 mL) and ammonia water (114 mL) were added into a pressure resistant tube, and the obtained mixture was then stirred at 100°C for 14 hours. Thereafter, the reaction mixture was cooled to room temperature, and was then poured into water (285 mL). The thus obtained mixture was stirred at room temperature for 5 hours. Thereafter, the precipitated solid was collected by filtration, was then washed with water, and was then dried to obtain a product of interest (34.5 g).

$^1$HNMR ($CDCl_3$)δ: 8.27(s,1H) 7.15(s,1H) 5.55-5.73(m,2H) 5.12-5.25(m,1H) 3.86-4.18(m,2H) 3.43-3.57(m,1H) 2.59-2.69(m,1H) 1.92-2.03(m,1H) 1.48(s,9H) 1.30-1.40(m,3H)
ESI-MS m/z 444 (MH+)

Reference Example 1(2)

4-Amino-7-((3R,5S)-1-(tert-butoxycarbonyl)-5-methylpyrrolidin-3-yl)-7H-pyrrolo[2,3-d]pyrimidine-5-carboxylic acid

The compound of Reference Example 1(1) (28.0 g), 10% palladium carbon catalyst (720 mg), NMP (84 mL), methanol (26 mL), and triethylamine (17.6 mL) were added into a pressure resistant tube, followed by carbon monoxide substitution, and the obtained mixture was stirred at 100°C for 2 hours. Thereafter, the reaction mixture was cooled to room temperature, a 2 M sodium hydroxide aqueous solution (79 mL) was then added thereto, and the obtained mixture was then stirred at 80°C for 2 hours. Thereafter, the reaction mixture was cooled to room temperature, was then filtrated through Celite, and was then washed with methanol. Subsequently, methanol in the filtrate was concentrated under reduced pressure. Water was further added, and the water layer was then washed with tert-butyl methyl ether. A 1 M potassium hydrogen sulfate aqueous solution was added to the water layer to adjust the pH to approximately 3. The precipitated solid was collected by filtration, was then washed with water, and was then dried to obtain a product of interest (23.4 g).
$^1$HNMR (400MHz, DMSO-d6)δ: 8.14 (s, 1H) 8.08 (s, 1H) 5.16-4.93(m,1H) 4.07-3.79(m,2H) 3.61-3.45(m,1H) 2.53(m,1H) 2.33-2.02(m,1H) 1.42(s,9H) 1.29(d,J = 6.1Hz,3H) ESI-MS m/z 362 (MH+)

< Examples >

Example 1(1)

tert-Butyl-4-amino-6-bromo-7-((3R,5S)-1-(tert-butoxycarbonyl)-5-methylpyrrolidin-3-yl)-7H-pyrrolo[2,3-d]pyrimidine-5-carboxylate

Under a nitrogen atmosphere, the compound of Reference Example 1(2) (15.0 g) was dissolved in chloroform (150 mL), and 2-tert-butyl-1,3-diisopropylisourea (25 mL) was then added to the above obtained solution. The temperature of the obtained mixture was increased to 60°C, and the mixture was then stirred for 2 hours. Thereafter, 2-tert-butyl-1,3-diisopropylisourea (25 mL) was further added to the reaction mixture, and the thus obtained mixture was then stirred for 2 hours. Thereafter, the reaction mixture was cooled to room temperature, and was then concentrated under reduced pressure. To the obtained residue, tert-butyl methyl ether was added, and the precipitated solid was collected by filtration and was then washed with tert-butyl methyl ether. The filtrate was concentrated under reduced pressure, and tert-butyl methyl ether was then added to the obtained residue. The precipitated solid was collected by filtration, and was then washed with tert-butyl methyl ether. The obtained residue was purified by silica gel chromatography (hexane : ethyl acetate) to obtain a tert-butyl ester form. The obtained compound was used in the subsequent halogenation reaction without being further purified.

The obtained tert-butyl ester form was dissolved in chloroform (140 mL), and N-bromosuccinimide (11.8 g) was then added to the above obtained solution. The obtained mixture was stirred at room temperature for 24 hours. Thereafter, to the reaction mixture, chloroform and 10% sodium bisulfite aqueous solution were successively added, and the obtained mixture was then extracted with chloroform. The gathered organic layer was washed with saturated saline, was then dried over anhydrous sodium sulfate, and was then concentrated under reduced pressure. The obtained residue was purified by silica gel chromatography (hexane : ethyl acetate) to obtain a product of interest (13.8 g).

$^1$HNMR (CDCl3)$\delta$: 8.02 (s, 1H) 5.74-5.13(m,2H) 4.07-3.64(m,2H) 2.43-2.29(m,1H) 2.07-1.97(m,1H) 1.63(s,9H) 1.48(m,12H)
ESI-MS m/z 496,498 (MH+)

Example 1(2)

tert-Butyl-7-((3R,5S)-1-acryloyl-5-methylpyrrolidin-3-yl)-4-amino-6-bromo-7H-pyrrolo[2,3-d]pyrimidine-5-carboxylate

The compound of Example 1(1) (11.4 g) was dissolved in THF (57 mL), and the obtained solution was then cooled to 0°C. Thereafter, 4 M hydrogen chloride in 1,4-dioxane solution (114 mL) was added to the mixture, and the thus obtained mixture was then stirred at 0°C for 10 hours. Subsequently, to the reaction mixture, a 5 M sodium hydroxide aqueous solution (92 mL), acetonitrile (57 mL), diisopropylethylamine (20 mL), and acryloyl chloride (2.0 mL) were added, and the obtained mixture was then stirred for 30 minutes. Thereafter, the reaction mixture was extracted with ethyl acetate, and the gathered organic layer was washed with saturated saline, was then dried over anhydrous sodium sulfate, and was then concentrated under reduced pressure. The obtained residue was purified by silica gel chromatography (hexane : acetone) to obtain a product of interest (7.72 g).

$^1$HNMR ($CDCl_3$)$\delta$: 8.26-8.16(m,1H) 6.62-6.30(m,2H) 5.81-5.64(m,1H) 5.33-5.14(m,1H) 4.81-3.75(m,3H) 3.07-2.86(m,1H) 2.67-2.33(m,1H) 1.69-1.61(m,9H) 1.60-1.51(m,3H)
ESI-MS m/z 450,452 (MH+)

Example 1(3)

tert-Butyl-7-((3R,5S)-1-acryloyl-5-methylpyrrolidin-3-yl)-4-amino-6-(prop-1-yn-1-yl)-7H-pyrrolo[2,3-d]pyrimidine-5-carboxylate

1.0 M Propyne in DMF solution (85.7 mL) was added to the compound of Example 1(2) (7.72 g), acetonitrile (154 mL), triethylamine (7.2 mL), $PdCl_2(PPh_3)2$ (1.2 g), and copper(I) iodide (330 mg), followed by nitrogen substitution. Thereafter, the mixture was stirred at 70°C for 4 hours. Thereafter, the reaction mixture was cooled to room temperature, and ethyl acetate and a saturated sodium hydrogen carbonate aqueous solution were added to the mixture. Thereafter, the obtained mixture was extracted with ethyl acetate, and the gathered organic layer was washed with water, and then with saturated saline. The resultant was dried over anhydrous sodium sulfate, and was then concentrated under reduced pressure. The obtained residue was purified by silica gel chromatography (hexane : acetone) to obtain a product of interest (4.06 g).

$^1$HNMR ($CDCl_3$)$\delta$: 8.29-8.17(m,1H) 6.63-6.30(m,2H) 5.81-5.63(m,1H) 5.42-5.15(m,1H) 4.66-3.81(m,3H) 3.01-2.82(m,1H) 2.65-2.32(m,1H) 2.92-2.13(m,3H) 1.65-1.59(m,9H) 1.57-1.49(m,3H)
ESI-MS m/z 410 (MH+)

Example 1(4)

7-((3R,5S)-1-acryloyl-5-methylpyrrolidin-3-yl)-4-amino-6-(prop-1-yn-1-yl)-7H-pyrrolo[2,3-d]pyrimidine-carboxylic acid

The compound of Example 1(3) (1.52 g) was dissolved in chloroform (5 mL), and trifluoroacetic acid (5 mL) was then added to the above obtained solution. The mixture was stirred at room temperature for 2 hours, and the reaction mixture was then concentrated under reduced pressure. To the residue, chloroform was added, and the obtained mixture was concentrated under reduced pressure again. The residue was dried under reduced pressure to obtain a product of interest (1.25 g). ESI-MS m/z 354 (MH+)

Example 1(5)

7-(R)-((3R,5S)-1-acryloyl-5-methylpyrrolidin-3-yl)-4-amino-N-((R)-1-(3,5-difluorophenyl)ethyl)-6-(prop-1-yn-1-yl)-7H-pyrrolo[2,3-d]pyrimidine-5-carboxamide

To the compound of Example 1(4) (100 mg) in DMF (1.0 mL) solution, (R)-1-(3,5-difluorophenyl)ethan-1-amine (89.0 mg), diisopropylethylamine (0.25 mL), and HATU (215 mg) were added, and the obtained mixture was then stirred at room temperature for 2 hours. Thereafter, to the reaction mixture, a saturated sodium hydrogen carbonate aqueous solution was added, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain the title compound (60 mg).

$^1$HNMR (DMSO-d6)6: 8.51(d,*J* = 7.3Hz,1H) 8.16 (s, 1H) 7.25-7.07(m,3H) 6.74-6.47(m,1H) 6.25-6.08(m,1H) 5.78-5.58(m,1H) 5.41-5.21(m,1H) 5.21-5.06(m,1H) 4.45-4.29(m,1H) 4.24-3.91(m,2H) 2.78-2.58(m,1H) 2.52-2.41(m,1H) 2.23(s,3H) 1.48(d,J= 7.1Hz,3H) 1.39(d,J= 6.1Hz,3H)
ESI-MS m/z 493 (MH+)

Example 2

7-((3R,5S)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide

The title compound was obtained in the same manner as that of Example 1, with the exception that (R)-1-phenylethan-1-amine was used instead of (R)-1-(3,5-difluorophenyl)ethan-1-amine in Example 1(5).

$^1$HNMR (DMSO-d6)6: 8.35(d,J= 7.8Hz,1H) 8.17-8.13(m,1H) 7.48-7.23(m,5H) 6.76-6.46(m,1H) 6.28-6.06(m,1H) 5.81-5.58(m,1H) 5.43-5.02(m,2H) 4.42-4.28(m,1H) 4.21-3.96(m,2H) 2.74-2.59(m,1H) 2.54-2.41(m,1H) 2.17(s,3H) 1.50(d,J = 6.8Hz,3H) 1.42-1.33(m,3H)
ESI-MS m/z 457 (MH+)

Example 3

7-((3R,5S)-1-acryloyl-5-methylpyrrolidin-3-yl)-4-amino-N-(2-phenylpropan-2-yl)-6-(prop-1-yn-1-yl)-7H-pyrrolo[2,3-d]pyrimidine-5-carboxamide

The title compound was obtained in the same manner as that of Example 1, with the exception that 2-phenylpropan-2-amine was used instead of (R)-1-(3,5-difluorophenyl)ethan-1-amine in Example 1(5).

$^1$HNMR (DMSO-d6)6: 8.26(s,1H) 8.16-8.08(m,1H) 7.44(dd,J= 8.8,1.2Hz,2H) 7.38-7.28(m,2H) 7.21(tt,J= 7.3,1.27Hz,1H) 6.76-6.50(m,1H) 6.25-6.10(m,1H) 5.79-5.62(m,1H) 5.45-5.19(m,1H) 4.45-4.30(m,1H) 4.26-4.01(m,2H) 2.79-2.42(m,2H) 2.29-2.22(m,3H) 1.71(s,6H) 1.43-1.36(m,3H)
ESI-MS m/z 471 (MH+)

Example 4

7-((3R,5S)-1-acryloyl-5-methylpyrrolidin-3-yl)-4-amino-N-((R)-1-phenylpropyl)-6-(prop-1-yn-1-yl)-7H-pyrrolo[2,3-d]pyrimidine-5-carboxamide

The title compound was obtained in the same manner as that of Example 1, with the exception that (R)-1-phenylpropan-1-amine was used instead of (R)-1-(3,5-difluorophenyl)ethan-1-amine in Example 1(5).

$^1$HNMR (DMSO-d6)6: 8.35(brd,J= 8.0Hz,1H) 8.17-8.11(m,1H) 7.46-7.22(m,5H) 6.74-6.50(m,1H) 6.26-6.08(m,1H) 5.79-5.60(m,1H) 5.40-5.21(m,1H) 4.99-4.87(m,1H) 4.43-4.30(m,1H) 4.23-3.94(m,2H) 2.76-2.42(m,2H) 2.21(s,3H) 1.95-1.74(m,2H) 1.44-1.34(m,3H) 0.91(t,J= 7.3Hz,3H)
ESI-MS m/z 471 (MH+)

Example 5

7-((3R,5S)-1-acryloyl-5-methylpyrrolidin-3-yl)-4-amino-N-(2-(2-fluorophenyl)propan-2-yl)-6-(prop-1-yn-1-yl)-7H-pyrrolo[2,3-d]pyrimidine-5-carboxamide

The title compound was obtained in the same manner as that of Example 1, with the exception that 2-(2-fluorophenyl)propan-2-amine was used instead of (R)-1-(3,5-difluorophenyl)ethan-1-amine in Example 1(5).

$^1$HNMR ($CDCl_3$)δ: 8.28(s,1H) 8.11(d,J = 4.4Hz,1H) 8.02(s,1H) 7.47-7.42(m,1H) 7.29-7.23(m,1H) 7.15(t,J = 7.7Hz,1H) 7.02(ddd,J = 12.5,8.1,1.1Hz,1H) 6.58-6.35(m,2H) 5.79-5.70(m,1H) 5.30-5.19(m,1H) 4.53(t,J = 10.1Hz,0.7H) 4.38-4.25(m,1.6H) 3.92(t,J = 8.8Hz,0.7H) 2.91-2.78(m,1H) 2.70-2.60(m,0.3H) 2.54-2.43(m,0.7H) 2.28(d,J = 7.0Hz,3H) 1.88(dt,J = 10.0,5.0Hz,6H) 1.53(t,J = 6.2Hz,3H)
ESI-MS m/z 489 (MH+)

Example 6

7-((3R,5S)-1-acryloyl-5-methylpyrrolidin-3-yl)-4-amino-N-((R)-1-(3-chlorophenyl)ethyl)-6-(prop-1-yn-1-yl)-7H-pyrrolo[2,3-d]pyrimidine-5-carboxamide

The title compound was obtained in the same manner as that of Example 1, with the exception that (R)-(+)-1-(3-chlorophenyl)ethylamine hydrochloride was used instead of (R)-1-(3,5-difluorophenyl)ethan-1-amine in Example 1(5).

$^1$HNMR ($CDCl_3$)δ: 8.22(d,J = 5.9Hz,1H) 7.75(d,J = 7.0Hz,1H) 7.38(s,1H) 7.35-7.27(m,3H) 6.58-6.33(m,2H) 5.78-5.66(m,1H) 5.29-5.19(m,2H) 4.56(t,J = 10.3Hz,0.7H) 4.39-4.20(m,1.6H) 3.89(t,J = 8.8Hz,0.7H) 2.94-2.82(m,1H) 2.66-2.58(m,0.3H) 2.46(dt,J = 14.5,6.1Hz,0.7H) 2.18(d,J = 11.0Hz,3H) 1.60(d,J = 7.0Hz,3H) 1.55-1.51(m,3H)
ESI-MS m/z 491,493 (MH+)

Example 7

7-((3R,5S)-1-acryloyl-5-methylpyrrolidin-3-yl)-4-amino-N-((R)-1-(2,4-difluorophenyl)ethyl)-6-(prop-1-yn-1-yl)-7H-pyrrolo[2,3-d]pyrimidine-5-carboxamide

The title compound was obtained in the same manner as that of Example 1, with the exception that (R)-(+)-1-(2,4-difluorophenyl)ethylamine hydrochloride was used instead of (R)-1-(3,5-difluorophenyl)ethan-1-amine in Example 1(5).

$^1$HNMR ($CDCl_3$)δ: 8.20(d,*J* = 5.9Hz,1H) 7.98(d,*J* = 7.7Hz,1H) 7.37-7.31(m,1H) 6.90-6.81(m,2H) 6.58-6.35(m,2H) 5.78-5.65(m,1H) 5.44-5.37(m,1H) 5.30-5.19(m,1H) 4.56(t,J= 10.1Hz,0.7H) 4.38-4.23(m,1.6H) 3.88(t,J= 8.8Hz,0.7H) 2.94-2.83(m,1H) 2.66-2.57(m,0.3H) 2.51-2.42(m,0.7H) 2.27(d,J= 9.2Hz,3H) 1.61(d,J= 7.0Hz,3H) 1.56-1.51(m,3H)
ESI-MS m/z 493 (MH+)

Example 8

7-((3R,5S)-1-acryloyl-5-methylpyrrolidin-3-yl)-4-amino-6-(prop-1-yn-1-yl)-N-((S)-2,2,2-trifluoro-1-phenylethyl)-7H-pyrrolo[2,3-d]pyrimidine-5-carboxamide

The title compound was obtained in the same manner as that of Example 1, with the exception that (S)-2,2,2-trifluoro-1-phenylethan-1-amine was used instead of (R)-1-(3,5-difluorophenyl)ethan-1-amine in Example 1(5).

$^1$HNMR ($CDCl_3$)δ: 8.40(d,J= 8.8Hz,1H) 8.16(s,1H) 7.44(s,5H) 6.58-6.38(m,2H) 5.92-5.84(m,1H) 5.81-5.69(m,1H) 5.29-5.19(m,1H) 4.55(t,J= 10.3Hz,0.7H) 4.41-4.24(m,1.6H) 3.91(t,J= 8.6Hz,0.7H) 2.92-2.80(m,1H) 2.70-2.61(m,0.3H) 2.54-2.46(m,0.7H) 2.35(d,J= 8.4Hz,3H) 1.54(t,J= 7.3Hz,3H)
ESI-MS m/z 511 (MH+)

Example 9

7-((3R,5S)-1-acryloyl-5-methylpyrrolidin-3-yl)-4-amino-6-(cyclopropylethynyl)-N-(2-phenylpropan-2-yl)-7H-pyrrolo[2,3-d]pyrimidine-5-carboxamide

The title compound was obtained in the same manner as that of Example 1, with the exceptions that cyclopropylacetylene was used instead of 1.0 M propyne in DMF solution in Example 1(3), and that 2-phenylpropan-2-amine was used instead of (R)-1-(3,5-difluorophenyl)ethan-1-amine in Example 1(5).

$^1$HNMR ($CDCl_3$)δ: 8.15(s,1H) 8.00(s,1H) 7.44(d,J= 7.7Hz,2H) 7.37(t,*J* = 7.7Hz,2H) 7.32-7.27(m, 1H) 6.66-6.30(m,2H) 5.81-5.69(m,1H) 5.38-5.24(m,1H) 4.48(t,J= 9.9Hz,0.7H) 4.42-4.29(m,1.6H) 4.22(t,J= 10.4Hz,0.7H) 2.77-2.68(m,1H) 2.67-2.60(m,0.3H) 2.59-2.52(m,0.7H) 1.83(s,6H) 1.60-1.52(m,4H) 1.08-1.01(m,2H) 0.92-0.88(m,2H)
ESI-MS m/z 497 (MH+)

Example 10

7-((3R,5S)-1-acryloyl-5-methylpyrrolidin-3-yl)-4-amino-6-(cyclopropylethynyl)-N-((R)-1-(2,3-difluorophenyl)ethyl)-7H-pyrrolo[2,3-d]pyrimidine-5-carboxamide

The title compound was obtained in the same manner as that of Example 1, with the exceptions that cyclopropylacetylene was used instead of 1.0 M propyne in DMF solution in Example 1(3), and that (R)-(+)-1-(2,3-difluorophenyl)ethylamine was used instead of (R)-1-(3,5-difluorophenyl)ethan-1-amine in Example 1(5).

$^1$HNMR ($CDCl_3$)δ: 8.17(d,J= 4.0Hz,1H) 8.04(d,J= 8.1Hz,1H) 7.15-7.05(m,3H) 6.58-6.36(m,2H)5.80-5.68(m,1H) 5.49-5.42(m,1H) 5.34-5.24(m,1H) 4.52(t,*J* = 10.1Hz,0.7H) 4.37-4.23(m,1.6H) 3.92(t,J= 8.8Hz,0.7H) 2.86-2.76(m,1H) 2.69-2.63(m,0.3H) 2.52-2.46(m,0.7H) 1.73-1.63(m,4H) 1.55(t,J= 5.3Hz,3H) 1.14-1.07(m,2H) 1.01-0.92(m,2H)
ESI-MS m/z 519 (MH+)

Example 11

Example 11(1)

tert-Butyl(2S,4R)-4-(4-amino-6-bromo-5-(((R)-1-phenylethyl)carbamoyl)-7H-pyrrolo[2,3-d]pyrimidin-7-yl)-2-methylpyrrolidine-1-carboxylate

The compound of Reference Example 1(2) (1.00 g), (R)-(+)-1-phenylethylamine (0.503 g), diisopropylethylamine (1.79 g), and N,N-dimethylformamide (10 mL) were added, and subsequently, HATU (1.58 g) was added. The obtained mixture was stirred at room temperature overnight. Thereafter, to the reaction mixture, ethyl acetate and a saturated sodium hydrogen carbonate aqueous solution were added, and the obtained mixture was then extracted with ethyl acetate. The gathered organic layer was washed with water, and then with saturated saline. The resultant was dried over anhydrous sodium sulfate, and was then concentrated under reduced pressure. The obtained residue was purified by silica gel chromatography (hexane : acetone) to obtain an amide form (1.53 g). The obtained compound was used in the subsequent reaction without being further purified.

To the amide form (1.53 g), chloroform (15 mL) was added, and the obtained mixture was then cooled to 0°C. Thereafter, N-bromosuccinimide (0.88 g) was added to the reaction mixture, and the obtained mixture was then stirred at 0°C for 1 hour. Thereafter, the reaction mixture was concentrated under reduced pressure, and the obtained residue was purified by silica gel chromatography (hexane : ethyl acetate) to obtain a product of interest (1.39 g).

$^1$HNMR ($CDCl_3$)δ: 8.21 (s, 1H) 7.42-7.28(m,5H) 6.97(d,J= 7.3Hz,1H) 5.36-5.29(m,1H) 5.20-5.07(m,1H) 4.30(t,J= 10.3Hz,1H) 4.04-3.72(m,2H) 3.00-2.86(m,1H) 2.38(dt,J= 14.3,6.0Hz, 1H) 1.63(d,J= 7.0Hz,3H) 1.53-1.43(m,12H)
ESI-MS m/z 543,545 (MH+)

Example 11(2)

7-((3R,5S)-1-acryloyl-5-methylpyrrolidin-3-yl)-4-amino-6-bromo-N-((R)-1-phenylethyl)-7H-pyrrolo[2,3-d1pyrimidine-5-carboxamide

To the compound of Example 11(1) (600 mg), chloroform(3 mL) was added, and the obtained mixture was then cooled to 0°C. Thereafter, trifluoroacetic acid (4.44 g) was added to the reaction mixture, and the thus obtained mixture was then stirred at room temperature for 1 hour. Thereafter, the reaction mixture was concentrated under reduced pressure, and acetonitrile (5 mL) was then added to the residue. The obtained mixture was concentrated under reduced pressure again to obtain an amine form. The obtained compound was used in the subsequent reaction without being further purified.

To the obtained amine form, acetonitrile (3 mL) was added, and the obtained mixture was then cooled to 0°C. Thereafter, acryloyl chloride (99.9 mg) and diisopropylethylamine (713 mg) were added, and the obtained mixture was then stirred at 0°C for 1 hour. Thereafter, the reaction mixture was concentrated under reduced pressure, and the obtained residue was purified by silica gel chromatography (ethyl acetate : methanol) to obtain a product of interest (281 mg).

$^1$HNMR ($CDCl_3$)δ: 8.20(d,J= 7.3Hz,1H) 7.42-7.36(m,4H) 7.32-7.28(m,1H) 7.00-6.94(m,1H) 6.57-6.33(m,2H) 5.76-5.66(m,1H) 5.36-5.29(m, 1H) 5.14-5.08(m,1H) 4,71(t,J= 9.9Hz,0.7H) 4.42-4.23(m,1.6H) 3.83(t,J= 8.6Hz,0.7H) 3.03-2.92(m,1H) 2.60-2.57(m,0.3H) 2.44-2.40(m, 0.7H) 1.64(d,J= 6.6Hz,3H) 1.56(dd,J= 11.7,6.2Hz,3H)
ESI-MS m/z 497,499 (MH+)

Example 11(3)

7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide

The compound of Example 11(2) (65 mg), dichlorobis(triphenylphosphine)dipalladium (9.2 mg), copper(I) iodide (5.0 mg), cyclopropylacetylene (13.0 mg), triethylamine (39.7 mg), and N,N-dimethylformamide (1.3 mL) were added, and the inside of the reaction system was then substituted with nitrogen. After that, the mixture was stirred at 70°C for 2.5 hours. Thereafter, to the reaction mixture, ethyl acetate and a saturated ammonium chloride aqueous solution were added, and the obtained mixture was then extracted with ethyl acetate. The gathered organic layer was washed with water, and then with saturated saline. The resultant was dried over anhydrous sodium sulfate, and was then concentrated under reduced pressure. The obtained residue was purified by silica gel chromatography (chloroform : methanol) to obtain a product of interest (50 mg).

$^1$HNMR (CDCl3)δ: 8.22(d,*J* = 5.1Hz,1H) 7.82(d,J= 7.3Hz,1H) 7.43-7.35(m,4H) 7.30(t,J= 6.8Hz,1H) 6.58-6.34(m,2H) 5.77-5.66(m,1H) 5.35-5.20(m,2H) 4.54(t,J= 10.1Hz,0.7H) 4.35-4.25(m,1.6H) 3.88(t,J= 8.8Hz,0.7H) 2.90-2.78(m,1H) 2.65-2.56(m,0.3H) 2.49-2.40(m,0.7H) 1.63 (d,J = 7.0Hz,3H) 1.56-1.45(m,4H) 1.03-0.91(m,2H) 0.84-0.69(m,2H)
ESI-MS m/z 483 (MH+)

Example 12

7-((3R,5S)-1-acryloyl-5-methylpyrrolidin-3-yl)-4-amino-6-(3,3-dimethylbut-1-yn-1-yl)-N-((R)-1-phenylethyl)-7H-pyrrolo[2,3-d]pyrimidine-5-carboxamide

The title compound was obtained in the same manner as that of Example 11, with the exception that 3,3-dimethyl-1-butyne was used instead of cyclopropylacetylene in Example 11(3).

$^1$HNMR ($CDCl_3$)δ: 8.22(d,J= 5.9Hz,1H) 7.75(d,J= 7.7Hz,1H) 7.38(dt,*J* = 15.5,7.1Hz,4H) 7.31-7.25(m,1H) 6.57-6.34(m,2H) 5.77-5.65(m,1H) 5.44-5.35(m,1H) 5.33-5.15(m,1H) 4.63(t,J= 10.1Hz,0.7H) 4.40-4.20(m,1.6H) 3.89(t,J= 8.8Hz,0.7H) 2.90-2.76(m,1H) 2.65-2.55(m,0.3H) 2.49-2.40(m,0.7H) 1.85(s,1H) 1.64(d,J= 7.0Hz,3H) 1.55(d,J= 5.9Hz,3H) 1.26(s,9H)
ESI-MS m/z 499 (MH+)

Example 13

7-((3R,5S)-1-acryloyl-5-methylpyrrolidin-3-yl)-4-amino-6-(3-methoxy-3-methylbut-1-yn-1-yl)-N-((R)-1-phenylethyl)-7H-pyrrolo[2,2-d]pyrimidine-5-carboxamide

The title compound was obtained in the same manner as that of Example 11, with the exception that 3-methoxy-3-methyl-1-butyne was used instead of cyclopropylacetylene in Example 11(3).

$^1$HNMR ($CDCl_3$)δ: 8.17 (s, 1H) 7.61(d,J= 7.7Hz,1H) 7.43-7.35(m,4H) 7.30(d,*J* = 7.0Hz,1H) 6.57-6.33(m,2H) 5.81-5.68(m,1H) 5.43-5.33(m,1H) 5.29-5.12(m,1H) 4.59(t,J= 10.1Hz,0.7H) 4.38-4.22(m,1.6H) 3.92(t,J= 8.6Hz,0.7H) 3.30(s,3H) 2.86-2.72(m,1H) 2.70-2.60(m,1.3H) 2.52-2.44(m,0.7H) 1.64(d,J= 7.0Hz,3H) 1.55(t,J= 5.5Hz,3H) 1.46(d,J= 2.2Hz,6H)
ESI-MS m/z 515 (MH+)

Example 14

7-((3R,5S)-1-acryloyl-5-methylpyrrolidin-3-yl)-4-amino-6-(but-1-yn-1-yl)-N-((R)-1-phenylethyl)-7H-pyrrolo[2,3-d]pyrimidine-5-carboxamide

The title compound was obtained in the same manner as that of Example 11, with the exception that 1-trimethylsilyl-1-butyne and tetra-n-butylammonium fluoride were used instead of cyclopropylacetylene in Example 11(3).

$^1$HNMR ($CDCl_3$)δ: 8.26-8.25(m,1H) 7.79(d,J = 7.3Hz,1H) 7.42-7.36(m,4H) 7.32-7.30(m,1H) 6.57-6.37(m,2H) 5.76-5.66(m,1H) 5.33-5.20(m,2H) 4.57(t,*J* = 10.3Hz,0.7H) 4.36-4.22(m,1.6H) 3.88(t,J= 8.8Hz,0.7H) 2.92-2.81(m,1H) 2.65-2.57(m,0.3H) 2.48-2.38(m,2.7H) 1.63 (d,J = 7.0Hz,3H) 1.54-1.51(m,3H) 1.17-1.12(m,3H)
ESI-MS m/z 471 (MH+)

Example 15

7-((3R,5S)-1-acryloyl-5-methylpyrrolidin-3-yl)-4-amino-N-(2-(2-fluorophenyl)propan-2-yl)-6-(3-methylbut-1-yn-1-yl)-7H-pyrrolo[2,3-d]pyrimidine-5-carboxamide

The title compound was obtained in the same manner as that of Example 11, with the exceptions that 2-(2-fluorophenyl)propan-2-amine was used instead of (R)-(+)-1-phenylethylamine in Example 11(1), and that 3-methyl-1-butyne was used instead of cyclopropylacetylene in Example 11(3).

$^1$HNMR ($CDCl_3$)δ: 7.92 (s, 1H) 7.44(t,*J* = 7.9Hz,1H) 7.30-7.23(m,1H) 7.14(t,*J* = 7.5Hz,1H) 7.02(dd,J= 12.6,8.2Hz,1H) 6.58-6.35(m,2H) 5.80-5.69(m,1H) 5.33-5.16(m,1H) 4.58(t,J= 9.9Hz,0.7H) 4.38-4.23(m,1.6H) 3.91(t,J= 8.4Hz,0.7H) 3.03-2.93(m,1H) 2.89-2.75(m,1H) 2.69-2.60(m,0.3H) 2.53-2.43(m,0.7H) 1.88(s,6H) 1.55(d,J = 5.1Hz,3H) 1.36(d,J = 6.6Hz,6H)
ESI-MS m/z 517 (MH+)

Example 16

Example 16(1)

tert-Butyl (2R,4S)-4-(benzyloxy)-2-((tosyloxy)methyl)pyrrolidine-1-carboxylate

tert-Butyl (2R,4S)-4-(benzyloxy)-2-(hydroxymethyl)pyrrolidine-1-carboxylate (2.0 g) was dissolved in methylene chloride (20 mL), and the obtained solution was then cooled to 0°C. Thereafter, 1,4-diazabicyclo[2.2.2]octane (2.2 g) and tosylate chloride (1.9 g) were added to the reaction solution, and the temperature of the mixture was then increased to room temperature. The mixture was stirred for 4 hours. Thereafter, a saturated sodium hydrogen carbonate aqueous solution was added to the reaction mixture, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : ethyl acetate) to obtain a product of interest (4.32 g).

$^1$HNMR ($CDCl_3$)δ: 7.78(d,J = 8.1Hz,2H), 7.42-7.29(m,7H), 4.57-4.41(m,2H), 4.39-3.96(m,4H),

3.61-3.20(m,2H),2.46(s,3H), 2.27-2.02(m,2H), 1.48-1.31(m,9H)
ESI-MS m/z 462 ($MH^+$)

Example 16(2)

tert-Butyl (2S,4S)-4-(benzyloxy)-2-ethylpyrrolidine-1-carboxylate

Under a nitrogen atmosphere, copper iodide (2.04 g) was suspended in diethyl ether (12 mL), and the obtained suspension was then cooled to 0°C. Thereafter, 1.04 M methyl lithium in diethyl ether solution (0.36 mL) was added, and the obtained mixture was then stirred at 0°C for 30 minutes. Subsequently, the compound of Example 16(1) (1.98 g) in methylene chloride (4.0 mL) solution was added to the reaction mixture, and the temperature of the obtained mixture was then increased to room temperature. The mixture was stirred for 1 hour. Thereafter, the reaction mixture was cooled to 0°C, and a saturated ammonium chloride aqueous solution was then added to the reaction mixture. The thus obtained mixture was extracted with ethyl acetate. The gathered organic layer was washed with saturated saline, was then dried over anhydrous sodium sulfate, and was then concentrated under reduced pressure. The obtained residue was purified by silica gel chromatography (hexane : ethyl acetate) to obtain a product of interest (707 mg).

$^1$HNMR ($CDCl_3$)$\delta$ 7.42-7.25(m,5H), 4.66-4.40(m,2H), 4.17-4.03(m,1H), 4.00-3.26(m,3H), 2.24-2.09(m,1H), 1.96-1.71(m,2H), 1.48(s,9H),1.45- 1.31(m,1H), 0.86(t,J = 7.4Hz,3H)
ESI-MS m/z 306 ($MH^+$)

Example 16(3)

tert-Butyl (2S,4S)-2-ethyl-4-hydroxypyrrolidine-1-carboxylate

The compound of Example 16(2) (1.06 g) and a 10% palladium hydroxide carbon catalyst (160 mg) were suspended in ethanol (11 mL) and THF (11 mL), followed by hydrogen substitution, and the resultant was then stirred at room temperature for 20 hours. Thereafter, the reaction mixture was filtrated through Celite, and was then washed with ethanol, and the filtrate was then concentrated under reduced pressure. The obtained residue was purified by silica gel chromatography (hexane : ethyl acetate) to obtain a product of interest (709 mg). $^1$HNMR ($CDCl_3$)$\delta$ 4.46-4.36(m,1H), 4.02-3.81(m,1H), 3.71-3.35(m,2H), 2.15-1.99(m,1H), 1.95-1.72(m,2H), 1.49(s,9H), 1.46-1.35(m,1H), 0.86(t,J = 7.5Hz,3H) ESI-MS m/z 216 ($MH^+$)

Example 16(4)

tert-Butyl (2S,4R)-4-(4-chloro-5-iodo-7H-pyrrolo[2,3-d]pyrimidin-7-yl)-2-ethylpyrrolidine-1-carboxylate

The compound of Example 16(3) (709 mg) and 4-chloro-5-iodo-7H-pyrrolo[2,3-d]pyrimidine (1.11 g) were dissolved in THF (7.1 mL), and the obtained solution was then cooled to 0°C. Thereafter, triphenylphosphine (1.3 g) and diisopropyl azodicarboxylate (1.00 mL) were added, and the temperature of the obtained mixture was then increased to room temperature, followed by stirring the mixture for 1 hour. Thereafter, the reaction mixture was concentrated under reduced pressure, and the obtained residue was then purified by silica gel chromatography (hexane : ethyl acetate) to obtain the corresponding coupling body. The obtained compound was used in the subsequent reaction without being further purified. Into a pressure resistant tube, the obtained coupling body, THF (5.4 mL), and ammonia water (5.4 mL) were added, and the obtained mixture was then stirred at 100°C for 14 hours. Thereafter, the reaction mixture was cooled to room temperature, and was then poured into water (12.8 mL), and the mixed solution was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain a product of interest (797 mg).

$^1$HNMR ($CDCl_3$)$\delta$ 8.29(s,1H), 7.14(s,1H), 5.67(br s,2H), 5.32-5.09(m,1H), 4.24-4.08(m,1H), 3.95-3.79(m,1H), 3.46(dd,J = 9.3,11.0Hz,1H), 2.70-2.55(m,1H), 2.06-1.95(m,1H), 1.59-1.51(m,2H), 1.49(s,9H), 0.91(t,J = 7.5Hz,3H)
ESI-MS m/z 458 ($MH^+$)

Example 16(5)

tert-Butyl (2S,4R)-4-(4-amino-6-bromo-5-(((R)-1-phenylethyl)carbamoyl)-7H-pyrrolo [2,3-d]pyrimidin-7-yl)-2-ethylpyrrolidine-1-carboxylate

The compound of Example 16(4) (797 mg), dichlorobis(triphenylphosphine)dipalladium (25 mg), and (R)-(+)-1-phenylethylamine (0.55 mL) were suspended in DMF (8.0 mL), followed by carbon monoxide substitution, and the resultant was then stirred at 80°C for 2 hours. Thereafter, the reaction mixture was cooled to room temperature, water was then added thereto, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain the corresponding amide form. The obtained compound was used in the subsequent reaction without being further purified. The obtained amide form was dissolved in acetonitrile (8.2 mL), and the obtained solution was then cooled to - 10°C. Thereafter, N-bromosuccinimide (457 mg) in acetonitrile (8.2 mL) solution was slowly added dropwise to the solution, and the reaction mixture was then stirred for 30 minutes. Thereafter, to the reaction mixture, a sodium sulfite aqueous solution and a sodium hydrogen carbonate aqueous solution were added, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain a product of interest (650 mg).

> $^1$HNMR ($CDCl_3$)$\delta$ 8.23(s,1H), 7.49-7.29(m,5H), 6.98(d,J = 7.4Hz,1H), 5.41-5.28(m,1H), 5.24-5.04(m,1H), 4.38-4.22(m,1H), 4.07-3.68(m,1H), 3.19-2.83(m,1H), 2.43-2.29(m,1H), 2.25-1.67(m,3H), 1.66(d,J = 6.9Hz,3H), 1.51(s,9H), 0.98(t,J = 7.4Hz,3H)
> ESI-MS m/z 557,559 ($MH^+$)

Example 16(6)

7-((3R,5S)-1-acryloyl-5-ethylpyrrolidin-3-yl)-4-amino-6-bromo-N-((R)-1-phenylethyl)-7H-pyrrolo[2,3-d]pyrimidine-5-carboxamide

To the compound of Example 16(5) (650 mg), acetonitrile (9.7 mL) was added, and the obtained mixture was then cooled to 0°C. Thereafter, sodium iodide (1.05 g) and trimethylsilyl chloride (0.89 mL) were added, and the obtained mixture was then stirred at 0°C for 1 hour. Thereafter, to the reaction mixture, ethanol (9.7 mL), isopropylethylamine (2.0 mL), and acrylic acid anhydride (0.16 mL) were successively added, and the obtained mixture was then stirred at 0°C for 30 minutes. Thereafter, to the reaction mixture, ammonia water and water were added, and the obtained mixture was then extracted with ethyl acetate. The gathered organic layer was washed with saturated saline, was then dried over anhydrous sodium sulfate, and was then concentrated under reduced pressure. The obtained residue was purified by silica gel chromatography (hexane : acetone) to obtain a product of interest (256 mg).

> $^1$HNMR ($CDCl_3$)$\delta$ 8.27-8.16(m,1H), 7.47-7.29(m,5H), 6.98(d,J = 7.3Hz,1H), 6.61-6.29(m,2H), 5.84-5.63(m,1H), 5.43-5.26(m,1H), 5.22-5.01(m,1H), 4.80-3.82(m,3H), 3.23-2.92(m,1H), 2.58-2.30(m,1H), 2.22-1.79(m,2H),1.66(d,J = 7.0Hz,3H),1.07-0.96(m,3H)
> ESI-MS m/z 511,513 ($MH^+$)

Example 16(7)

7-((3R,5S)-1-acryloyl-5-ethylpyrrolidin-3-yl)-4-amino-N-((R)-1-phenylethyl)-6-(prop-1-yn-1-yl)-7H-pyrrolo[2,3-d]pyrimidine-5-carboxamide

1.0 M Propyne in DMF solution (0.70 mL) was added to the compound of Example 16(6) (120 mg), acetonitrile (1.2 mL), triethylamine (0.10 mL), $PdCl_2(PPh_3)_2$ (8.2 mg), and copper(I) iodide (0.4 mg), followed by nitrogen substitution, and the mixture was then stirred at 60°C for 2 hours. Thereafter, the reaction mixture was cooled to room temperature, and ethyl acetate and a saturated ammonium chloride aqueous solution were added to the mixture. The thus obtained mixture was extracted with ethyl acetate, and the gathered organic layer was washed with water, and then with saturated saline. The resultant was dried over anhydrous sodium sulfate, and was then concentrated under reduced pressure. The obtained residue was purified by silica gel chromatography (ethyl acetate : methanol) to obtain a product of interest (102 mg).

$^1$HNMR ($CDCl_3$)δ: 8.26(s,1H), 7.79(br d,J = 7.0Hz,1H), 7.46-7.30(m,5H), 6.58-6.31(m,2H), 5.80-5.65(m,1H), 5.33-5.15(m,2H), 4.59-3.85(m,3H), 3.03-2.33(m,2H), 2.25-1.70(m,5H), 1.65(d,J = 6.8Hz,6H), 1.09-0.91(m,3H)
ESI-MS m/z 471 ($MH^+$)

Example 17

7-((3R,5S)-1-acryloyl-5-ethylpyrrolidin-3-yl)-4-amino-6-(cyclopropylethynyl)-N-((R)-1-phenylethyl)-7H-pyrrolo[2,3-d]pyrimidine-5-carboxamide

The title compound was obtained in the same manner as that of Example 16, with the exception that cyclopropylacetylene was used instead of 1.0 M propyne in DMF solution in Example 16(7).

$^1$HNMR ($CDCl_3$)δ: 8.31-8.16(m,1H), 7.84(d,J = 7.4Hz,1H), 7.46-7.30(m,5H), 6.64-6.32(m,2H), 5.82-5.67(m,1H), 5.39-5.17(m,2H), 4.67-3.81(m,3H), 3.02-2.80(m,1H), 2.62-1.71(m,3H), 1.65(d,J = 6.9Hz,3H), 1.58-1.47(m,1H), 1.06-0.92(m,5H), 0.85-0.70(m,2H)
ESI-MS m/z 497 ($MH^+$)

Example 18

7-((3R,5R)-1-acryloyl-5-(methoxymethyl)pyrrolidin-3-yl)-4-amino-6-(cyclopropylethynyl)-N-((R)-1-phenylethyl)-7H-pyrrolo[2,3-d]pyrimidine-5-carboxamide

The title compound was obtained in the same manner as that of Example 16, with the exceptions that tert-butyl (2R,4S)-4-hydroxy-2-(methoxymethyl)pyrrolidine-1 -carboxylate was used instead of the compound of Example 16(3) in Example 16(4), and that cyclopropylacetylene was used instead of 1.0 M propyne in DMF solution in Example 16(7).

$^1$HNMR ($CDCl_3$)δ: 8.29-8.22(m,1H), 7.86-7.80(m,1H), 7.36-7.44(m,4H), 7.34-7.28(m,1H), 6.48-6.37(m,2H), 5.78-5.69(m,1H), 5.29-5.15(m,2H), 4.55-4.30(m,2H), 3.96-3.65(m,3H), 3.42(s,3H), 3.18-3.06(m,0.3H), 2.90-2.80(m,0.3H), 2.64-2.58(m,0.3H), 2.47-2.35(m,0.7H), 1.64(d,3H,J = 6.9Hz), 1.58-1.47(m,1H), 1.04-0.94(m,2H), 0.87-0.69(m,2H)
ESI-MS m/z 513 ($MH^+$)

Example 19

7-((3R,5R)-1-acryloyl-5-(ethoxymethyl)pyrrolidin-3-yl)-4-amino-6-(cyclopropylethynyl)-N-((R)-1-phenylethyl)-7H-pyrrolo[2,3-d]pyrimidine-5-carboxamide

The title compound was obtained in the same manner as that of Example 16, with the exceptions that tert-butyl (2R,4S)-2-(ethoxymethyl)-4-hydroxypyrrolidine-1 -carboxamide was used instead of the compound of Example 16(3) in Example 16(4), and that cyclopropylacetylene was used instead of 1.0 M propyne in DMF solution in Example 16(7).

$^1$HNMR ($CDCl_3$)δ: 8.28-8.18(m,1H),7.84(br d,J = 7.0Hz,1H), 7.47-7.29(m,5H), 6.82-6.35(m,2H), 5.79-5.68(m,1H), 5.40-5.14(m,2H), 4.63-3.53(m,7H), 3.20-2.79(m,1H), 2.69-2.40(m,1H), 1.67-1.63(m,3H), 1.59-1.47(m,1H),1.22(t,J = 7.0Hz,3H), 1.05-0.92(m,2H), 0.87-0.72(m,2H)
ESI-MS m/z 527 ($MH^+$)

Comparative Example 1

4-Amino-N-(4-(methoxymethyl)phenyl)-7-(1-methylcyclopropyl)-6-(prop-1-yn-1-yl)-7H-pyrrolo[2,3-d]pyrimidine-5-carboxamide

The title compound was obtained by the method described in Example 95 of International Publication No. WO 2017/146116.
ESI-MS m/z 390 (MH+)

Comparative Example 2

1-((3R,5S)-1-acryloyl-5-methylpyrrolidin-3-yl)-4-amino-N-(4-(2-(dimethylamino)-2-oxoethyl)-2,3-dimethylphenyl)-1H-pyrazolo[3,4-d]pyrimidine-3-carboxamide

The title compound was obtained by the method described in Example 79 of International Publication No. WO 2017/038838.
ESI-MS m/z 505 (MH+)

Comparative Example 3

7-((3R,5S)-1-acryloyl-5-methylpyrrolidin-3-yl)-4-amino-N-(cyclohexylmethyl)-6-(prop-1-yn-1-yl)-7H-pyrrolo[2,3-d]pyrimidine-5-carboxamide

The title compound was obtained in the same manner as that of Example 1, with the exception that cyclohexylmethanamine was used instead of (R)-1-(3,5-difluorophenyl)ethan-1-amine in Example 1(5).

$^1$HNMR (DMSO-d6)δ:8.68-8.31(m,1H) 8.20-8.10(m,1H) 8.09-7.97(m,1H) 7.59-7.20(m,1H) 6.74-6.49(m,1H) 6.25-6.09(m,1H) 5.78-5.60(m,1H) 5.40-5.20(m,1H) 4.44-4.29(m,1H) 4.23-3.92(m,2H) 3.25-3.12(m,2H) 2.76-2.40(m,2H) 2.25(s,3H) 1.81-1.45(m,5H) 1.43-1.34(m,3H) 1.30-0.90(m,6H)
ESI-MS m/z 449 (MH+)

Comparative Example 4

7-((3R,5S)-1-acryloyl-5-methylpyrrolidin-3-yl)-4-amino-N-(2-methylbenzyl)-6-(prop-1-yn-1-yl)-7H-pyrrolo[2,3-d]pyrimidine-5-carboxamide

The title compound was obtained in the same manner as that of Example 1, with the exception that o-tolylmethanamine was used instead of (R)-1-(3,5-difluorophenyl)ethan-1-amine in Example 1(5).

$^1$HNMR (DMSO-d6)δ:8.37-8.27(m,1H) 8.19-8.09(m,1H) 7.39-7.30(m,1H) 7.26-7.11(m,4H) 6.68-6.48(m,1H) 6.24-6.07(m,1H) 5.80-5.60(m,1H) 5.36-5.17(m,1H) 4.52(d,J= 5.7Hz,2H) 4.42-4.28(m,1H) 4.22-3.92(m,2H) 2.73-2.42(m,2H) 2.33(s,3H) 2.02(s,3H) 1.43-1.32(m,3H)
ESI-MS m/z 457 (MH+)

Comparative Example 5

7-((3R,5S)-1-acryloyl-5-methylpyrrolidin-3-yl)-4-amino-N-methyl-N-((R)-1-phenylethyl)-6-(prop-1-yn-1-yl)-7H-pyrrolo[2,3-d]pyrimidine-5-carboxamide

The title compound was obtained in the same manner as that of Example 1, with the exception that (R)-N-methyl-1-phenylethan-1-amine was used instead of (R)-1-(3,5-difluorophenyl)ethan-1-amine in Example 1(5).

$^1$HNMR ($CDCl_3$)δ: 8.23(d,*J* = 5.9Hz,1H) 7.50-7.28(m,4H) 7.09-6.88(m,1H) 6.57-6.34(m,2H) 5.79-5.64(m,1H) 5.22(t,J= 9.3Hz,1H) 4.48(t,J= 9.7Hz,0.6H) 4.39-4.20(m,1.9H) 3.90(t,J= 8.6Hz,0.5H) 2.85(s,4H) 2.66-2.63(m,0.4H) 2.51-2.44(m,0.6H) 2.07(s,2H) 1.66(d,J= 4.8Hz,3H) 1.52(d,J= 5.9Hz,3H)
ESI-MS m/z 471 (MH+)

Comparative Example 6

Comparative Example 6(1)

tert-Butyl (2S,4R)-4-(4-amino-5-(((R)-1-phenylethyl)carbamoyl)-6-(prop-1-yn-1-yl)-7H-pyrrolo[2,3-d]pyrimidin-7-yl)-2-methylpyrrolidine-1-carboxylate

1.0 M Propyne in DMF solution(2.1 mL) was added to the compound of Example 11(1) (230 mg), acetonitrile (4.6 mL), triethylamine (0.29 mL), $PdCl_2(PPh_3)_2$ (5.9 mg), and copper(I) iodide (1.6 mg), followed by nitrogen substitution, and the obtained mixture was then stirred at 70°C for 1 hour. Thereafter, the reaction mixture was cooled to room

temperature, and ethyl acetate and a saturated sodium hydrogen carbonate aqueous solution were then added to the mixture. The thus obtained mixture was extracted with ethyl acetate, and the gathered organic layer was washed with water and then with saturated saline. The resultant was dried over anhydrous sodium sulfate, and was then concentrated under reduced pressure. The obtained residue was purified by silica gel chromatography (hexane : ethyl acetate) to obtain a product of interest (193 mg).

$^1$HNMR ($CDCl_3$)δ: 8.23 (s, 1H) 7.79(d,*J* = 6.8Hz,1H)7.46-7.27(m,5H) 5.40-5.17(m,2H) 4.28-3.64(m,3H) 2.85-2.68(m,1H) 2.46-2.36(m,1H) 2.15-1.97(m,3H) 1.62(d,J= 6.8Hz,3H) 1.56-1.32(m,12H)
ESI-MS m/z 503 (MH+)

Comparative Example 6(2)

4-Amino-7-((3R,5S)-5-methylpyrrolidin-3-yl)-N-((R)-1-phenylethyl)-6-(prop-1-yn-1-yl)-7H-pyrrolo[2,3-d]pyrimidine-5-carboxamide hydrochloride

To the compound of Comparative Example 6(1) (530 mg), 4 M hydrochloric acid in 1,4-dioxane solution (5 mL) was added, and the obtained mixture was then stirred at room temperature for 2 hours. Thereafter, the reaction mixture was concentrated under reduced pressure to obtain a product of interest (420 mg).
ESI-MS m/z 403 (MH+)

Comparative Example 6(3)

4-Amino-7-((3R,5S)-1-((E)-but-2-enoyl)-5-methylpyrrolidin-3-yl)-N-((R)-1-phenylethyl)-6-(prop-1-yn-1-yl)-7H-pyrrolo[2,3-d]pyrimidine-5-carboxamide

To the compound of Comparative Example 6(2) (18 mg), acetonitrile (0.5 mL) was added, and the obtained mixture was then cooled to 0°C. Thereafter, acryloyl chloride (0.004 mL) and diisopropylethylamine (0.036 mL) were added to the reaction mixture, and the thus obtained mixture was then stirred at 0°C for 1 hour. Thereafter, the reaction mixture was concentrated under reduced pressure, and was then subjected to reverse phase preparative HPLC (water : acetonitrile (0.1% formic acid)) to obtain a product of interest (8.7 mg).

$^1$HNMR ($CDCl_3$)δ: 8.31 (s, 1H) 8.14(d,J= 6.2Hz,1H)7.77(d,*J* = 7.0Hz, 1H) 7.39-7.36(m,4H) 7.33-7.31(m,1H) 7.03-6.90(m,1H) 6.06(dd,*J* = 14.3Hz,1H) 5.28-5.17(m,2H) 4.48(t,*J* = 10.1Hz,1H) 4.35-4.20(m,2H) 3.88(t,8.8Hz,1H) 2.84-2.76(m,1H) 2.64-2.40(m,1H) 2.05(d,J= 10.6Hz,3H) 1.92(d,J= 6.6Hz,1H) 1.85(d,J = 7.0Hz,2H) 1.62(d,J= 7.0Hz,3H) 1.55(dd,J= 9.0,5.7Hz,3H)
ESI-MS m/z 471 (MH+)

The compounds synthesized in the above-described Examples and Comparative Examples are shown below.

[Table 1]

| Example No. | Structural formula | Example No. | Structural formula |
|---|---|---|---|
| 1 | 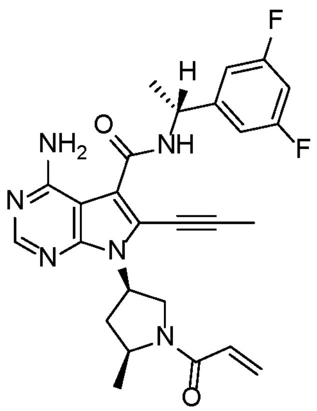 | 2 | 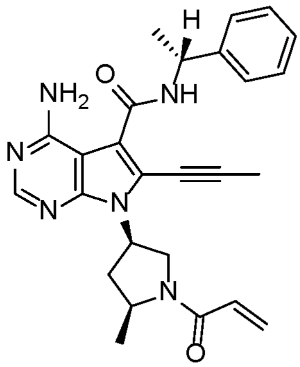 |

(continued)

| Example No. | Structural formula | Example No. | Structural formula |
|---|---|---|---|
| 3 | 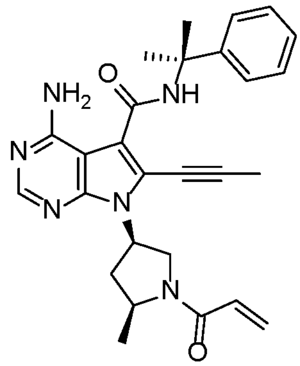 | 4 | 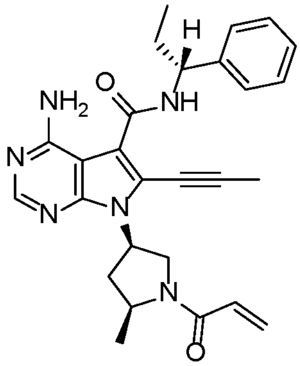 |
| 5 | 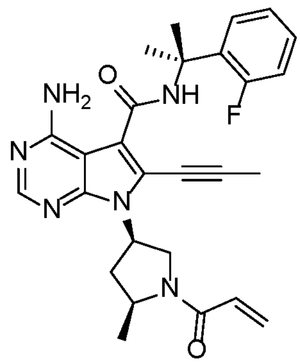 | 6 | 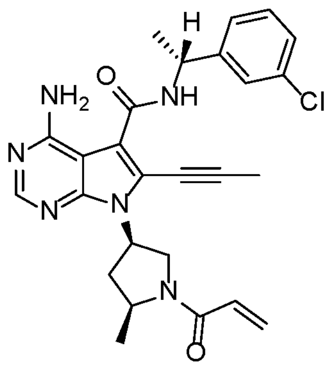 |
| 7 | 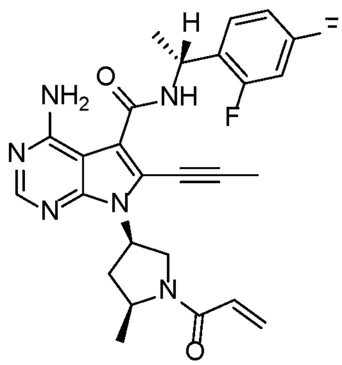 | 8 | 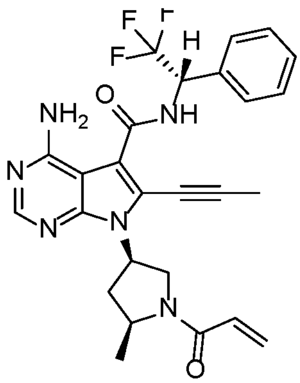 |
| 9 | 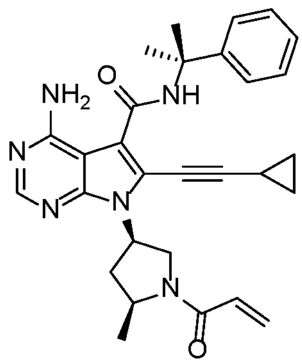 | 10 | 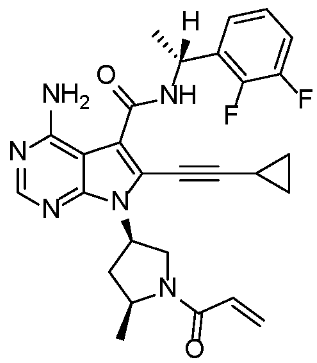 |

(continued)

| Example No. | Structural formula | Example No. | Structural formula |
|---|---|---|---|
| 11 | 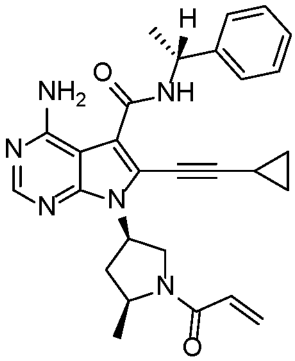 | 12 | 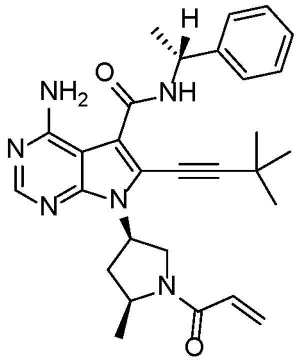 |
| 13 | 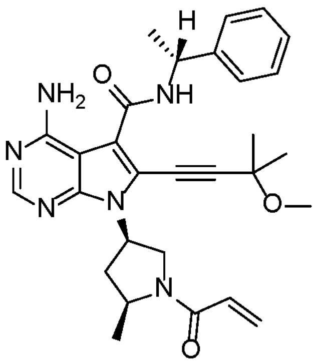 | 14 | 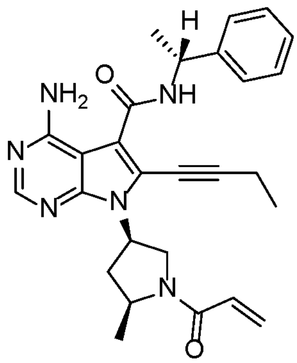 |
| 15 | 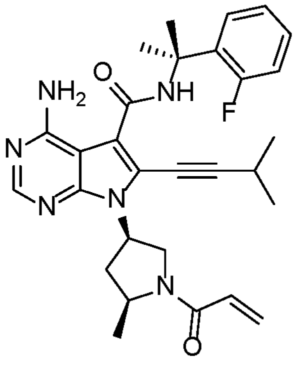 | 16 | 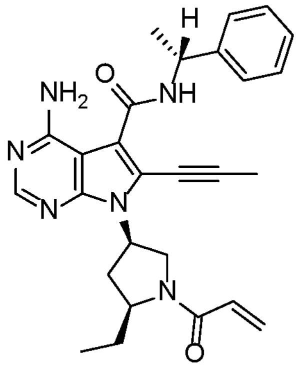 |
| 17 | 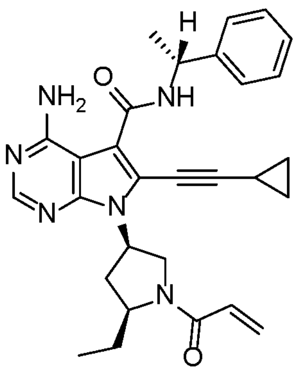 | 18 | 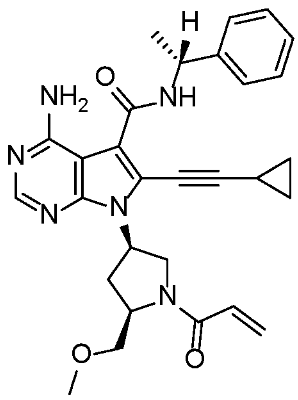 |

(continued)

| Example No. | Structural formula | Example No. | Structural formula |
|---|---|---|---|
| 19 | 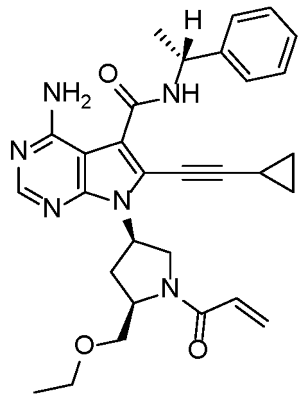 | | |

[Table 2]

| Comparative Example No. | Structural formula | Comparative Example No. | Structural formula |
|---|---|---|---|
| 1 | 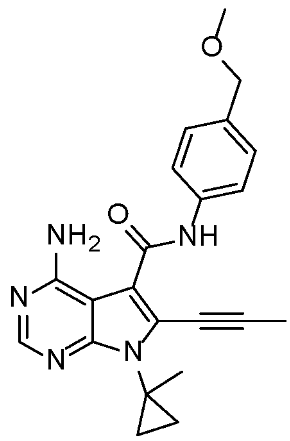 | 2 | 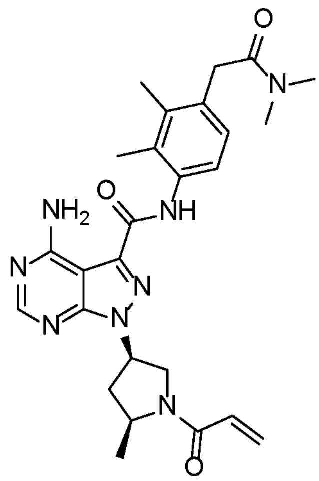 |
| 3 | 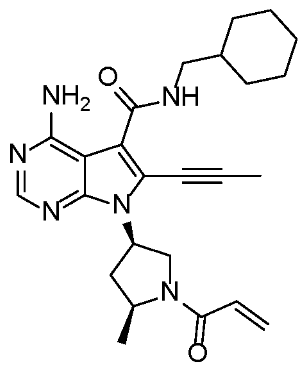 | 4 | 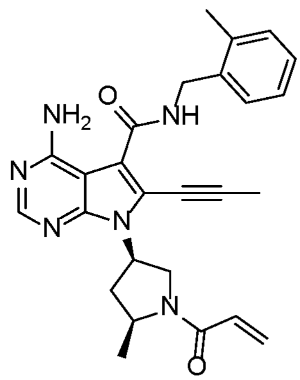 |

(continued)

| Comparative Example No. | Structural formula | Comparative Example No. | Structural formula |
|---|---|---|---|
| 5 | 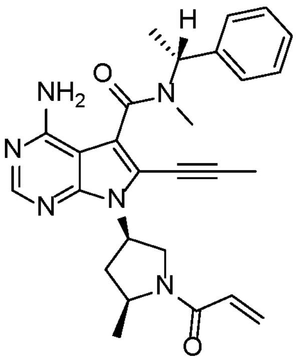 | 6 | 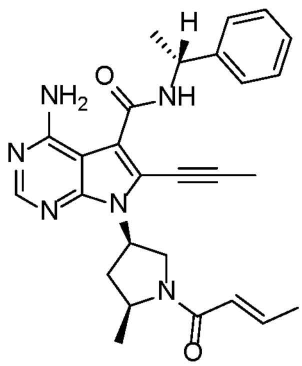 |

Test Example 1 Measurement of phosphorylation activity inhibitory effect *(in vitro)* on wild-type and mutant EGFR

The measurement of the *in vitro* inhibitory activity of a compound against wild-type and mutant EGFR was outsourced to Carna Biosciences, Inc. (Kinase Profiling Book, https://www.carnabio.com/japanese/product/search.cgi?mode=profiling).

Specifically, first, the compound of the present invention was diluted stepwise with dimethyl sulfoxide (DMSO). Subsequently, the EGFR protein, the substrate peptide (Srctide, final concentration: 1 μM), magnesium chloride (final concentration: 5 mM), manganese chloride (final concentration: 1 mM), ATP (final concentration: near Km of each EGFR), and the compound of the present invention in DMSO solution (final concentration of DMSO: 1%) were added to a buffer for the kinase reaction (20 mM HEPES (pH 7.5), 1 mM dithiothreitol, and 0.01% Triton X-100), and the obtained mixture was then incubated at room temperature for 1 hour, so that the kinase reaction was carried out. To the reaction solution, Termination Buffer was added, so as to terminate the kinase reaction. Finally, using LabChip(TM) EZ Reader II (PerkinElmer), an unphosphorylated substrate peptide (S) and a phosphorylated peptide (P) were separated and detected according to microchannel capillary electrophoresis. From the peak heights of S and P, the amount of the phosphorylation reaction was obtained, and the concentration of the compound capable of inhibiting the phosphorylation reaction by 50% was defined as an IC50 value (nM). The results are shown in the following table.

[Table 3]

| | IC50 (nmol/L) |
|---|---|
| kinases | Example compound 11 |
| EGFR | 0.47 |
| EGFR [d746-750] | 0.25 |
| EGFR [d746-750/T790M] | 6.8 |
| EGFR [L858R] | 0.36 |
| EGFR [T790M/L858R] | 6.6 |
| EGFR [D770_N771insNPG] | 0.55 |

From the above results, it was found that the compound of the present invention has excellent inhibitory activity against wild-type and mutant EGFR.

Test Example 2 Measurement of growth inhibitory activity against EGFR overexpressing cell line and exon 20 insertion mutant EGFR expressing cell line

Growth inhibitory activity against an EGFR overexpressing cell line and an exon 20 insertion mutant EGFR expressing cell line was evaluated using MCF10A_EGFR cells, MCF10A_EGFR/V769_D770insASV cells, MCF10A_EGFR/D770_N771insSVD cells, and MCF10A_EGFR/H773_V774insNPH cells (Fukushima Medical University) which were cells obtained by introducing the EGFR gene (WT, D769_N770insASV mutant, D770_N771insSVD mutant, H773_V774insNPH mutant) into MDA-MB-468 cells (ATCC) as an EGFR overexpressing human breast cancer cell line, NCI-H1975 cells (ATCC) as L858R and T790M mutant EGFR-positive human lung cancer cells, and MCF10A cells as human normal mammary gland cells.

The MDA-MB-468 cells were suspended in Leibovitz's L-15 medium containing 10% inactivated fetal bovine serum. The NCI-H1975 cells were suspended in RPMI-1640 medium containing 10% inactivated fetal bovine serum. The MCF10A_EGFR cells, the MCF10A_EGFR/V769_D770insASV cells, the MCF10A_EGFR/D770_N771insSVD cells, or the MCF10A_EGFR/H773_V774insNPH cells were suspended in DMEM/Ham's F-12 medium (containing L-glutamine, phenol red, HEPES, and sodium pyruvate) containing 10 μg/mL insulin, 500 ng/mL hydrocortisone, 5 μmol/L forskolin, and 5% inactivated horse serum in terms of their final concentrations. Each cell suspension was seeded in each well of a 96-well flat-bottom plate such that the number of cells per well was 500, and was then cultured in a carbon dioxide gas-free culture vessel for the MDA-MB-468 cells and in a 5% carbon dioxide gas-containing culture vessel for the other cells at 37°C for 1 day. The compound of the present invention was prepared at 1 mM in DMSO and then diluted 1/200 with a medium to prepare a 5 μM solution. Thereafter, the compound of the present invention in the DMSO solution was diluted with the medium used in the suspension of the cells, and the obtained solution was then added to each well so that the final concentration of the highest concentration of the test compound was 1000 nM. The obtained mixture was further cultured in a carbon dioxide gas-free culture vessel for the MDA-MB-468 cells and in the 5% carbon dioxide gas-containing culture vessel for the other cells at 37°C for 3 days.

The cells at the start of the culture (day 0) and after the culture (day 3) were counted using CellTiter-Glo(R) 2.0 Reagent (Promega Corp.) according to the protocol recommended by the manufacturer. The growth inhibition percentage was calculated according to the equation given below to determine the 50% inhibition concentration (GI50 (nM)) of the test compound. The results are shown in Table 4.

1) In the case of $T_{day3} \geq C_{day0}$

$$\text{Growth percentage (\%)} = (T_{day3} - C_{day0}) / (C_{day3} - C_{day0}) \times 100$$

T: Emission intensity from the well to which the test compound was added
C: Emission intensity from the well to which the test compound was not added
day0: Day on which the test compound was added
day3: Evaluation day

2) In the case of $T_{day3} < C_{day0}$

$$\text{Growth percentage (\%)} = (T_{day3} - C_{day0}) / (C_{day0}) \times 100$$

T: Emission intensity from the well to which the test compound was added

C: Emission intensity from the well to which the test compound was not added

day0: Day on which the test compound was added

day3: Evaluation day

[Table 4]

| | Cell growth inhibitory activity GI 50 value (nM) | | | | | |
|---|---|---|---|---|---|---|
| | MDA-MB-468 | NCI-H1975 | MCF10A EGFR cell (EGFR-WT) | MCF10A EGFR/ V769_D770insASV cell | MCF10A EGFR/ D770_N771insSVD cell | MCF10A_EGFR/ H773_V774insNPH cell |
| Example compound 2 | 81 | 77 | 55 | 6.7 | 8.2 | 16 |

(continued)

| | Cell growth inhibitory activity GI 50 value (nM) | | | | | |
|---|---|---|---|---|---|---|
| | MDA-MB-468 | NCI-H1975 | MCF10A EGFR cell (EGFR-WT) | MCF10A EGFR/ V769_D770insASV cell | MCF10A EGFR/ D770_N771insSVD cell | MCF10A_EGFR/ H773_V774insNPH cell |
| Example compound 11 | 99 | 87 | 81 | 7.3 | 10 | 18 |
| Example compound 12 | 221 | 153 | 206 | 24 | 27 | 50 |

From the above results, it was found that the compound group of the present invention also has excellent cell growth inhibitory activity against the wild-type EGFR overexpressing line MDA-MB-468 cells, the MCF10A_EGFR cells expressing the introduced wild-type EGFR gene, the L858R and T790M mutant EGFR-positive cells NCI-H1975 cells, and the exon 20 insertion mutant EGFR expressing cell lines (MCF10A_EGFR/V769_D770insASV cells, MCF10A_EGFR/D770_N771insSVD cells, and MCF10A_EGFR/H773_V774insNPH cells).

Test Example 3 Measurement of growth inhibitory activity against exon 20 insertion mutant EGFR expressing cell line

Growth inhibitory activity against exon 20 insertion mutant EGFR was evaluated using H1975-EGFRinsSVD cells obtained by genetically modifying NCI-H1975 cells so as to express D770_N771insSVD mutant EGFR and to knock out endogenous EGFR (T790M/L858R), and LXF 2478 cells (Charles River Laboratories, Inc.) of V769_D770insASV mutant EGFR-positive human lung cancer patient-derived tumor.

The H1975-EGFRinsSVD cells were prepared by introducing PB-CMV-MCS-EF1-RFP+Puro vector encoding D770_N771insSVD (insSVD), together with Super PiggyBacTransposase expression vector, to NCI-H1975 cells by electroporation with Amaxa(R) Cell Line Nucleofector(R) Kit R, then selecting cells using puromycin (Sigma-Aldrich Co. LLC.), then introducing thereto XTN(R) TALENs Site-Specific Nucleases (Transposagen Bio) by electroporation with Amaxa(R) Cell Line Nucleofector(R) Kit R, and selecting, by sequencing, cells in which endogenous EGFR (T790M/L858R) was knocked out.

Upon evaluation of cell growth inhibitory effect, the cells of each line were suspended in RPMI-1640 medium. The cell suspension was seeded in each well of a 96-well flat-bottom plate such that the number of cells per well was 3,000, and was then cultured in a 5% carbon dioxide gas-containing culture vessel at 37°C for 1 day. The compound of the present invention or a Comparative Example compound was dissolved at 1 mM in DMSO and then added to each well using Tecan D300e digital dispenser (Tecan Trading AG) such that the final concentration of the highest concentration of the test compound was 1000 nM and the common ratio was 3. The cells were cultured in a 5% carbon dioxide gas-containing culture vessel at 37°C for 3 days. The cells at the start of the culture (day 0) and after the culture (day 3) were counted using CellTiter-Glo(R) 2.0 Reagent (Promega Corp.) according to the protocol recommended by the manufacturer. The growth inhibition percentage was calculated according to the equation given below to determine the 50% inhibition concentration (GI50 (nM)) of the test compound. The results are shown in Table 5.

1) In the case of $T_{day3} \geq C_{day0}$

$$\text{Growth percentage (\%)} = (T_{day3} - C_{day0}) / (C_{day3} - C_{day0}) \times 100$$

T: Emission intensity from the well to which the test compound was added
C: Emission intensity from the well to which the test compound was not added
day0: Day on which the test compound was added
day3: Evaluation day

2) In the case of $T_{day3} < C_{day0}$

$$\text{Growth percentage (\%)} = (T_{day3} - C_{day0}) / (C_{day0}) \times 100$$

T: Emission intensity from the well to which the test compound was added
C: Emission intensity from the well to which the test compound was not added
day0: Day on which the test compound was added
day3: Evaluation day

[Table 5]

| | Cell growth inhibitory activity GI 50 value (nM) | |
|---|---|---|
| | H1975-EGFRinsSVD cell | LXF 2478 cell |
| Example compound 1 | 44 | 29 |
| Example compound 2 | 9.5 | 11 |
| Example compound 3 | 77 | 24 |
| Example compound 4 | 13 | 15 |
| Example compound 5 | 33 | 24 |
| Example compound 6 | 29 | 29 |
| Example compound 7 | 33 | 33 |
| Example compound 8 | 25 | 26 |
| Example compound 9 | 65 | 34 |
| Example compound 10 | 39 | 40 |
| Example compound 11 | 17 | 19 |
| Example compound 12 | 52 | 48 |
| Example compound 13 | 48 | 46 |
| Example compound 14 | 30 | 27 |
| Example compound 15 | 101 | 58 |
| Comparative Example 1 | > 1000 | > 1000 |
| Comparative Example 2 | > 1000 | > 1000 |

From the above results, it was found that the compound group of the present invention also has excellent cell growth inhibitory activity against the exon 20 insertion mutant EGFR expressing cell lines (H1975-EGFRinsSVD and LXF 2478).

Test Example 4 Evaluation of oral absorbability

The compound of the present invention was suspended or dissolved in 0.5% HPMC aqueous solution and 0.1 N hydrochloric acid, and the obtained suspension or solution was orally administered to BALB/cA mice (CLEA Japan, Inc.) at a dose of 50 mg/kg/day. At 0.5, 1, 2, 4 and 6 hours after completion of the oral administration, blood was collected from the facial vein over time, so as to obtain plasma. The concentration of the compound in the obtained plasma was measured by LC-MS/MS, and the oral absorbability of the present compound was evaluated.
The results are shown in the following Table 6.

[Table 6]

| Example No. | AUC 0 - 6 hr (μM·hr) | Example No. | AUC 0 - 6 hr (μM·hr) |
|---|---|---|---|
| 1 | 50 | 2 | 15 |
| 3 | 24 | 4 | 12 |

(continued)

| Example No. | AUC 0 - 6 hr (μM·hr) | Example No. | AUC 0 - 6 hr (μM·hr) |
|---|---|---|---|
| 5 | 20 | 6 | 17 |
| 7 | 15 | 8 | 15 |
| 9 | 51 | 10 | 50 |
| 11 | 31 | 12 | 36 |
| 13 | 18 | 14 | 27 |
| 15 | 34 | 16 | 15 |
| 17 | 21 | 18 | 15 |
| 19 | 6.1 | Comp. Ex. 2 | 1.5 |

From the above results, it was found that the compound of the present invention was contained in a sufficient concentration in the plasma, so that the present compound exhibited favorable oral absorbability. In contrast, the compound of Comparative Example 2 had oral absorbability that was more than 4 times more attenuated than the compound of the present invention.

Test Example 5 Evaluation of brain penetration properties

The compound of the present invention was suspended or dissolved in 0.5% HPMC aqueous solution and 0.1 N hydrochloric acid, and the obtained suspension or solution was orally administered to BALB/cA mice (CLEA Japan, Inc.) at a dose of 50 mg/kg/day. At 0.5 hours after completion of the oral administration, blood was collected from the facial vein, and whole brain was then excised, so as to obtain plasma and brain samples. Water was added to the obtained brain sample in 3 times the volume of the brain sample, and the resultant was then homogenized using an ultrasonic homogenizer, so as to obtain a brain homogenate. The concentration of the compound in the obtained plasma and brain homogenate was measured by LC-MS/MS, and the brain penetration properties of the present compound were evaluated from the brain/plasma concentration of the compound.

The results are shown in the following Table 7.

[Table 7]

| Example No. | Compound concentration in plasma (μM) | Compound concentration in brain (μM) | Kp value (Compound concentration in brain/plasma) |
|---|---|---|---|
| 1 | 9.1 | 1.4 | 0.15 |
| 2 | 6.6 | 1.8 | 0.27 |
| 3 | 11 | 1.4 | 0.13 |
| 4 | 8.3 | 2.8 | 0.34 |
| 5 | 15 | 2.2 | 0.15 |
| 6 | 7.5 | 1.3 | 0.17 |
| 7 | 7.9 | 1.1 | 0.14 |
| 8 | 9.9 | 3.3 | 0.33 |
| 9 | 13 | 2.4 | 0.18 |
| 10 | 13 | 2.4 | 0.18 |
| 11 | 12 | 2.7 | 0.23 |
| 12 | 11 | 3.2 | 0.29 |
| 13 | 13 | 2.8 | 0.22 |

(continued)

| Example No. | Compound concentration in plasma ($\mu$M) | Compound concentration in brain ($\mu$M) | Kp value (Compound concentration in brain/plasma) |
|---|---|---|---|
| 14 | 9.9 | 2.1 | 0.21 |
| 15 | 8.1 | 1.2 | 0.15 |
| 16 | 12 | 4.4 | 0.35 |
| 17 | 17 | 6.5 | 0.39 |
| 18 | 7.7 | 1.6 | 0.22 |
| 19 | 4.9 | 0.7 | 0.14 |
| Comp. Ex. 2 | 1.6 | 0.008 | 0.005 |

From the above results, it was found that the compound of the present invention had a high brain/plasma compound concentration (Kp value) and thus, exhibited favorable brain penetration properties. On the other hand, the brain concentration of the compound of Comparative Example 2 was more than 80 times more attenuated than that of the compound of the present invention.

Test Example 6 Antitumor effect confirmation test (*in vivo*) on subcutaneous transplantation models, into which H1975-EGFRinsSVD cell line is transplanted

H1975-EGFRinsSVD cell line was cultured in RPMI-1640 (containing 4.5 g/L glucose, 10 mM HEPES and 1 mM sodium pyruvate) (FUJIFILM Wako Pure Chemical Corporation) medium containing 10% inactivated fetal bovine serum (FBS) in a 5% $CO_2$ incubator at 37°C.

The H1975-EGFRinsSVD cells were resuspended at a concentration of $8 \times 10^7$ cells/mL in PBS. The cell suspension was subcutaneously transplanted at $8 \times 10^6$ cells/0.1 mL to the right chest of each 6 week old nude mouse (BALB/cAJcl-nu/nu, CLEA Japan, Inc.) using a 1 mL syringe for tuberculin and a 25 G injection needle.

When the tumor volumes of nude mice having tumor engraftment became on the order of 100 to 200 $mm^3$, the mice were assigned to groups each involving 6 animals by random stratification such that the average tumor volume was equal among the groups.

The test compound used was the compounds of Examples 2, 11, and 12, and a 0.5% HPMC aqueous solution was used as a control. The compounds of Examples 2, 11 and 12 were orally administered at doses of 25 mg/kg/day, 25 mg/kg/day and 50 mg/kg/day, respectively.

Each test compound or the control was orally administered every day for 14 days (Days 1 - 14) from the day following the grouping.

To compare the transition of tumor growth over time by the administration of each test compound, a tumor volume (which is also referred to as "TV" below) was measured at a frequency of twice a week over time. For the measurement of the body weight, an animal electronic balance was used. A body weight change percentage is also referred to as "BWC" below. A body weight change percentage on the $n^{th}$ day (BWCn) from the body weight on the $n^{th}$ day (BWn) was calculated according to the equation given below. The transition of average TV and BWC values of the individuals is shown in Figures 1 and 2.

$$\text{BWCn (\%)} = ((\text{body weight on } n^{th} \text{ day}) - (\text{body weight on grouping day})] / [(\text{body weight on grouping day}) \times 100$$

When the average TV value of the compound administration group on the final evaluation day (Day 15) was smaller than that of the control group and exhibited statistically significant difference (Dunnett type multiple comparison test), this compound was judged as being effective (P < 0.001) and is indicated by the mark * in the drawing. The results are shown in Figure 1.

As a result of conducting analysis by the Dunnett type multiple comparison test, it was shown that the tumor volume was statistically significantly low (P < 0.001) for all the invention compounds as compared with the control group.

From the results of this test, it was found that the compounds of Examples 2, 11, and 12 have excellent antitumor effects against the exon 20 insertion mutant EGFR expressing cell line (H1975-EGFRinsSVD) transplanted into the nude mouse chest. Moreover, a body weight reduction of 20% or more was not observed in all of the mice to which each compound had been administered.

Test Example 7 Antitumor effect confirmation test (*in vivo*) on direct brain transplantation models, into which luciferase gene-introduced exon 20 insertion mutant EGFR expressing cell line (H1975-EGFRinsSVD-Luc) is directly transplanted

The antitumor effects and life-extending effects of the invention compound on direct brain transplantation models were evaluated using H1975-EGFRinsSVD-Luc line obtained by introducing luciferase into the human mutant EGFR-introduced cell line H1975-EGFRinsSVD.

The H1975-EGFRinsSVD-Luc cells used were prepared by introducing pJTI(R) FAST DEST vector encoding luciferase, together with pJTI(R) PhiC31 Integrase expression vector, to NCI-H1975-EGFRinsSVD cells by electroporation with Amaxa(R) Cell Line Nucleofector(R) Kit R, followed by selection using hygromycin B (Nacalai Tesque, Inc.).

The H1975-EGFRinsSVD-Luc cell line was cultured in RPMI-1640 (containing 4.5 g/L glucose, 10 mM HEPES and 1 mM sodium pyruvate) (FUJIFILM Wako Pure Chemical Corporation) medium containing 10% inactivated fetal bovine serum (FBS) in a 5% $CO_2$ incubator at 37°C.

The H1975-EGFRinsSVD-Luc cells were resuspended at a concentration of $12.5 \times 10^7$ cells/mL in PBS.

Using a mouse ear bar, a nude mouse with 6 to 7 weeks old (BALB/cAJcl-nu/nu, CLEA Japan, Inc.) was fixed in a brain stereotaxic apparatus, and the skin on the top of the head was disinfected by the application of an Isodine-containing antiseptic solution using a sterile cotton swab and was then excised with a surgical knife.

A microdrill was used to drill a hole in the skull, and then, using a needle, a manipulator, and a syringe pump, 2 μL of the cell suspension was transplanted into the brain at a rate of 0.8 μL/min.

As a reference of the amount of brain tumor, 26 days after the transplantation, Total Flux (Photon/sec) was measured in all of the survival cases, using IVIS (PerkinElmer, Inc., model: Lumina II). Based on the obtained results, 10 animals were assigned to each group by random stratification such that the average total flux was equal among the groups.

The test compound used was the compound of Example 11, and a 0.5% HPMC aqueous solution was used as a control. The compound of Example 11 was administered at a dose of 12.5 mg/kg/day or 25 mg/kg/day.

The compound of the present invention or the control was orally administered once a day, every day, for 38 days (Days 27 - 64) from the following day of the grouping day.

For judgment of the presence or absence of antitumor effects, the value (Log10) obtained by logarithmic transformation of the total flux on the antitumor effect judgment day (Day 47) after 3-week drug administration from the following day (Day 27) of the grouping day was used.

A graph was prepared with the value obtained by the average total flux of each group as a vertical axis, and with the number of days (Day) after the transplantation as a horizontal axis. The transition of the total flux over time in the drug administration period was observed.

For judgment of the presence or absence of life-extending effects, the number of survival days on the final life-extending effect evaluation day from after cell transplantation (Days 0 - 65) in the test compound group compared with the control group was analyzed by the Log-Rank test.

The results are shown in Figures 3 and 4 given below. The value obtained by logarithmic transformation (Log10) of the total flux on Day 47 in each group was analyzed by the Dunnett type multiple comparison test. As a result, it was demonstrated that the aforementioned value of the test compound group was statistically significantly lower than the value of the control group (significance level (both sides): 5%) ($P < 0.001$). From the results of this test, it was found that the compound of the present invention has antitumor effects against the exon 20 insertion mutant EGFR expressing cell line (H1975-EGFRinsSVD-Luc) transplanted into the nude mouse brain.

In addition, the number of survival days on the final life-extending effect evaluation day from after cell transplantation (Days 0 - 65) in the test compound group compared with the control group was analyzed by the Log-Rank test. As a result, statistically significant life-extending effects were observed, as compared with the control group ($P < 0.05$).

As described above, the compound of the present invention or a salt thereof has EGFR inhibitory activity and has brain penetration properties and as such, is useful as an EGFR inhibitor or a therapeutic agent for EGFR-positive tumor.

## Claims

1. A therapeutic agent for a disease associated with EGFR, comprising a pyrimidine compound represented by the following formula (I), or a pharmaceutically acceptable salt thereof as an active ingredient:

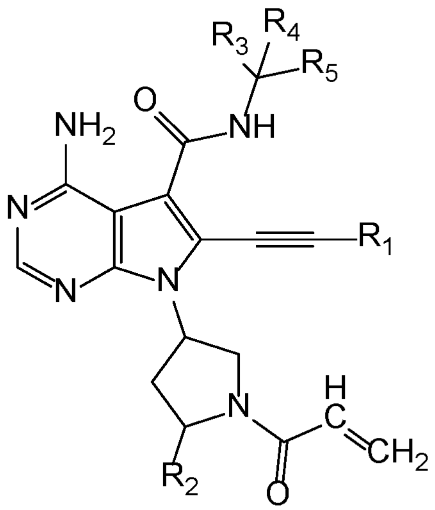

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**2.** The therapeutic agent according to claim 1, wherein the pyrimidine compound is a compound represented by the following formula (II):

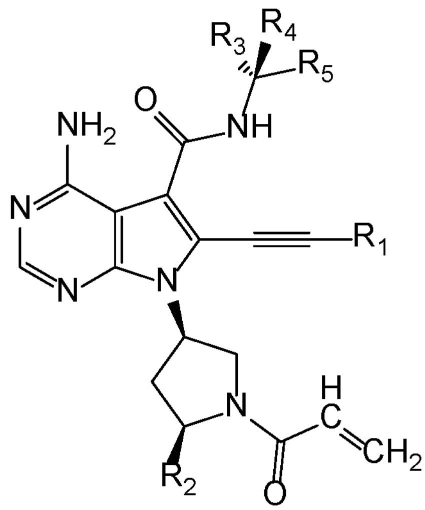

(II)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

3. The therapeutic agent according to claim 1 or 2, wherein the pyrimidine compound is a compound represented by the formula (I) or (II) wherein
$R_2$ is a C1-C6 alkyl group optionally having 1 to 5 C1-C4 alkoxy groups as a substituent(s).

4. The therapeutic agent according to any one of claims 1 to 3, wherein the pyrimidine compound is a compound represented by the formula (I) or (II) wherein
$R_3$ is a C1-C4 alkyl group optionally having 1 to 5 fluorine atoms as a substituent(s).

5. The therapeutic agent according to any one of claims 1 to 4, wherein the pyrimidine compound is a compound represented by the formula (I) or (II) wherein
$R_5$ is a phenyl group optionally having 1 or 2 substituents selected from the group consisting of fluorine atoms and chlorine atoms.

6. The therapeutic agent according to any one of claims 1 to 5, wherein the pyrimidine compound is a compound represented by the formula (I) or (II) wherein
$R_1$ is a methyl group, a tert-butyl group, or a cyclopropyl group.

7. The therapeutic agent according to any one of claims 1 to 6, wherein the pyrimidine compound is a compound represented by the formula (I) or (II) wherein
$R_2$ is a methyl group, an ethyl group, a methoxymethyl group, or an ethoxymethyl group.

8. The therapeutic agent according to any one of claims 1 to 7, wherein the pyrimidine compound is a compound represented by the formula (I) or (II) wherein
$R_3$ is a methyl group.

9. The therapeutic agent according to any one of claims 1 to 8, wherein the pyrimidine compound is a compound represented by the formula (I) or (II) wherein
$R_4$ is a hydrogen atom.

10. The therapeutic agent according to any one of claims 1 to 9, wherein the pyrimidine compound is a compound represented by the formula (I) or (II) wherein
$R_5$ is a phenyl group.

11. The therapeutic agent according to any one of claims 1 to 10, wherein the pyrimidine compound is a compound selected from the following (1) to (3):

(1) 7-((3R,SS)-1-acryloyl-5-methylpyrrolidin-3-yl)-4-amino-N-((R)-1-phenylethyl)-6-(prop-1-yn-1-yl)-7H-pyrrolo[2,3-d]pyrimidine-5-carboxamide,
(2) 7-((3R,5S)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide, and
(3) 7-((3R,SS)-1-acryloyl-5-methylpyrrolidin-3-yl)-4-amino-6-(3,3-dimethylbut-1-yn-1-yl)-N-((R)-1-phenylethyl)-7H-pyrrolo[2,3-d]pyrimidine-5-carboxamide.

12. The therapeutic agent according to any one of claims 1 to 11, wherein the pyrimidine compound is 7-((3R,SS)-1-acryloyl-5-methylpyrrolidin-3-yl)-4-amino-6-(cyclopropylethynyl)-N-((R)-1-phenylethyl)-7H-pyrrolo[2,3-d]pyrimidine-5-carboxamide.

13. The therapeutic agent according to any one of claims 1 to 12, wherein the disease associated with EGFR is malignant tumor having EGFR overexpression, EGFR gene amplification, or an EGFR mutation.

14. An EGFR inhibitor comprising a pyrimidine compound represented by the following formula (I), or a pharmaceutically acceptable salt thereof as an active ingredient:

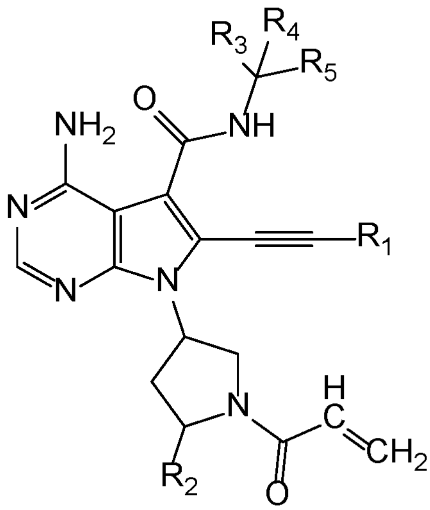

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**15.** A therapeutic agent for EGFR-positive tumor, comprising a pyrimidine compound represented by the following formula (I), or a pharmaceutically acceptable salt thereof as an active ingredient:

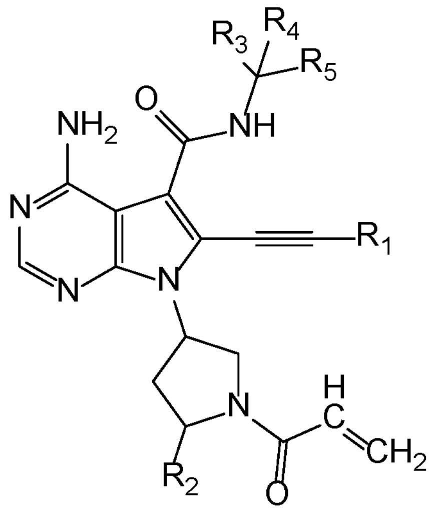

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**16.** A pharmaceutical composition for use in the treatment of a disease associated with EGFR, the pharmaceutical composition comprising a pyrimidine compound represented by the following formula (I), or a pharmaceutically acceptable salt thereof and a pharmaceutically acceptable carrier:

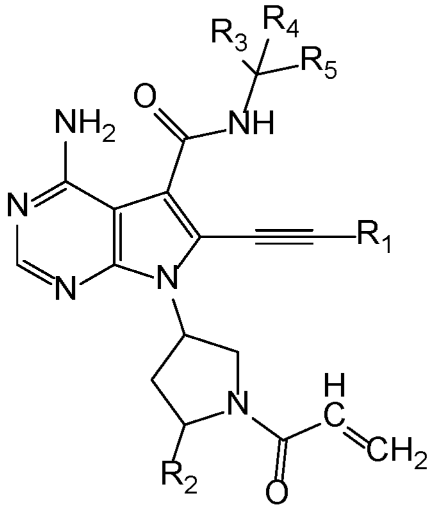

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**17.** The pharmaceutical composition according to claim 16, wherein the disease associated with EGFR is malignant tumor having EGFR overexpression, EGFR gene amplification, or an EGFR mutation.

**18.** A compound represented by the following formula (I) or a pharmaceutically acceptable salt thereof for use in the treatment of a disease associated with EGFR:

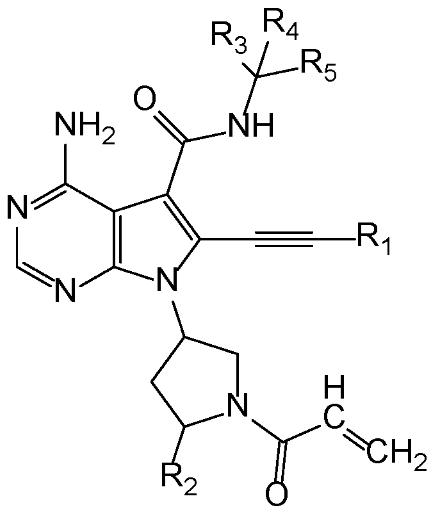

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**19.** Use of a compound represented by the following formula (I) or a pharmaceutically acceptable salt thereof for use in the treatment of a disease associated with EGFR:

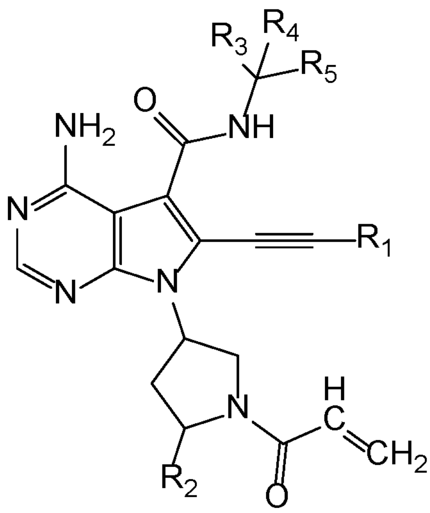

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**20.** Use of a compound represented by the following formula (I) or a pharmaceutically acceptable salt thereof for the manufacture of a therapeutic agent for use in the treatment of a disease associated with EGFR:

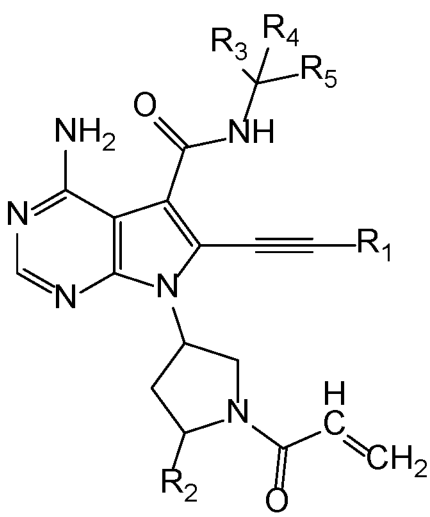

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**21.** A compound represented by the following formula (I) or a pharmaceutically acceptable salt thereof for use in the treatment of EGFR-positive tumor:

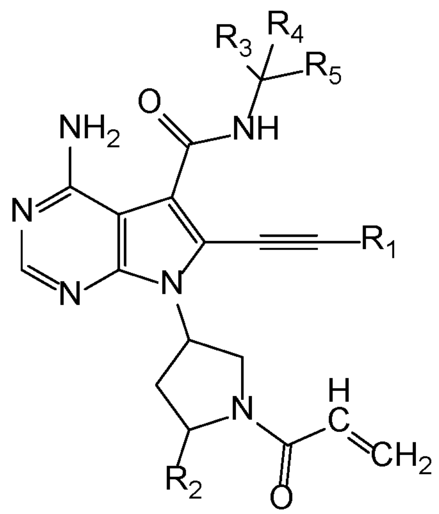

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**22.** Use of a compound represented by the following formula (I) or a pharmaceutically acceptable salt thereof for use in the treatment of EGFR-positive tumor:

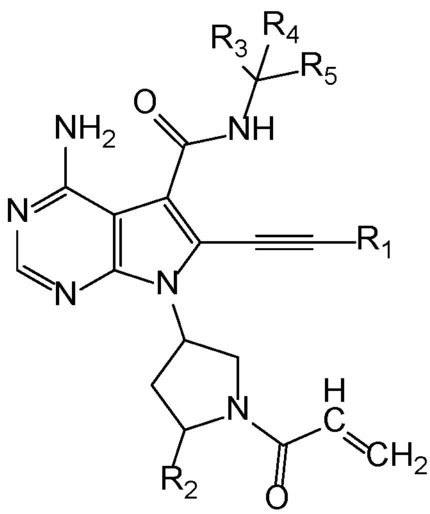

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**23.** Use of a compound represented by the following formula (I) or a pharmaceutically acceptable salt thereof for the manufacture of a therapeutic agent for use in the treatment of EGFR-positive tumor:

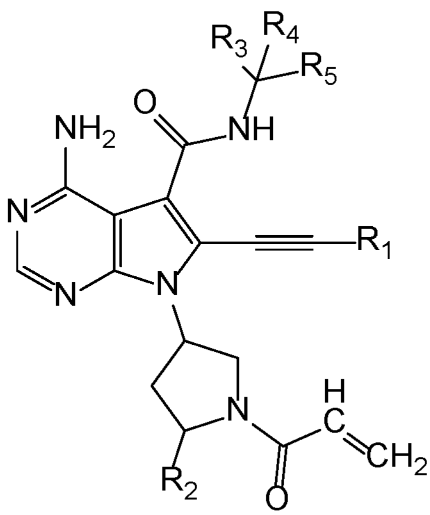

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**24.** A method for treatment of a disease associated with EGFR, the method comprising administering an effective amount of a compound represented by the following formula (I) or a pharmaceutically acceptable salt thereof to a subject in need thereof :

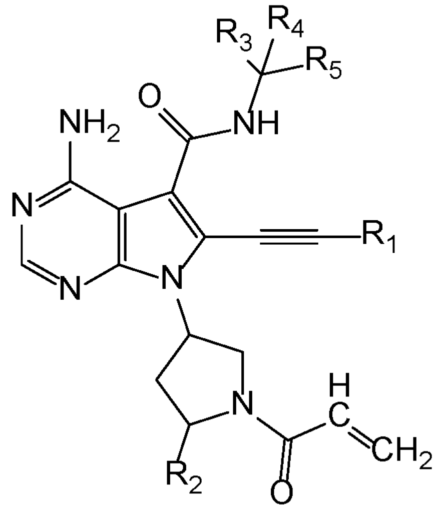

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

**25.** A method for treatment of EGFR-positive tumor, the method comprising administering an effective amount of a compound represented by the following formula (I) or a pharmaceutically acceptable salt thereof to a subject in need thereof :

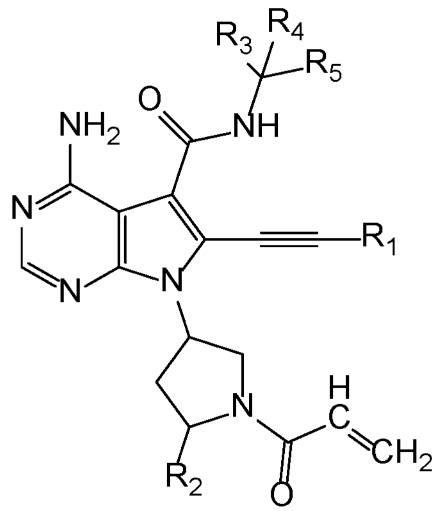

(I)

wherein $R_1$ represents a C1-C4 alkyl group optionally having a C1-C4 alkoxy group as a substituent, or a C3-C4 cycloalkyl group;

$R_2$ represents a hydrogen atom, a halogen atom, a C1-C6 alkyl group optionally having 1 to 5 C1-C4 alkoxy

groups or fluorine atoms each as a substituent(s), or a C1-C6 alkoxy group;
$R_3$ represents a hydrogen atom, or a C1-C4 alkyl group optionally having 1 to 5 fluorine atoms as a substituent(s);
$R_4$ represents a hydrogen atom or a C1-C4 alkyl group; and
$R_5$ represents a phenyl group optionally having 1 to 3 substituents selected from fluorine atoms and chlorine atoms.

[Figure 1]

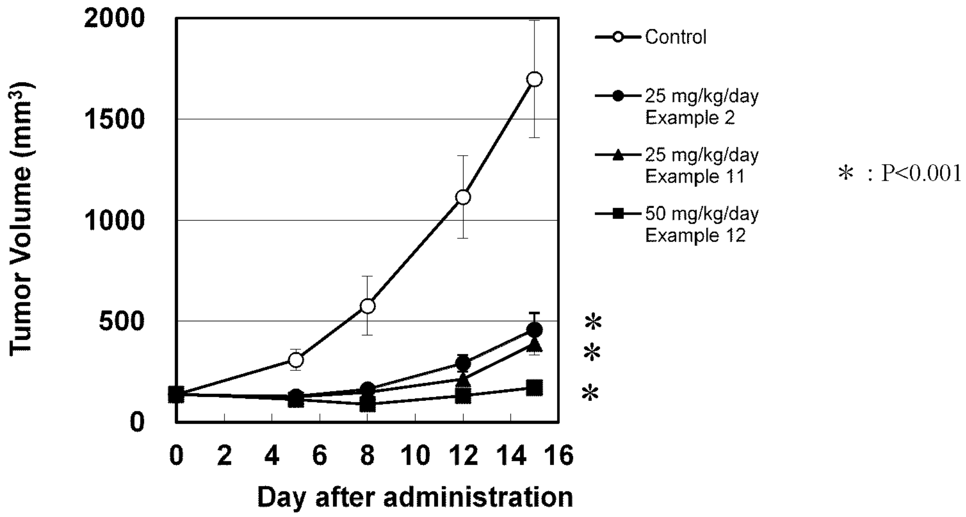


[Figure 2]

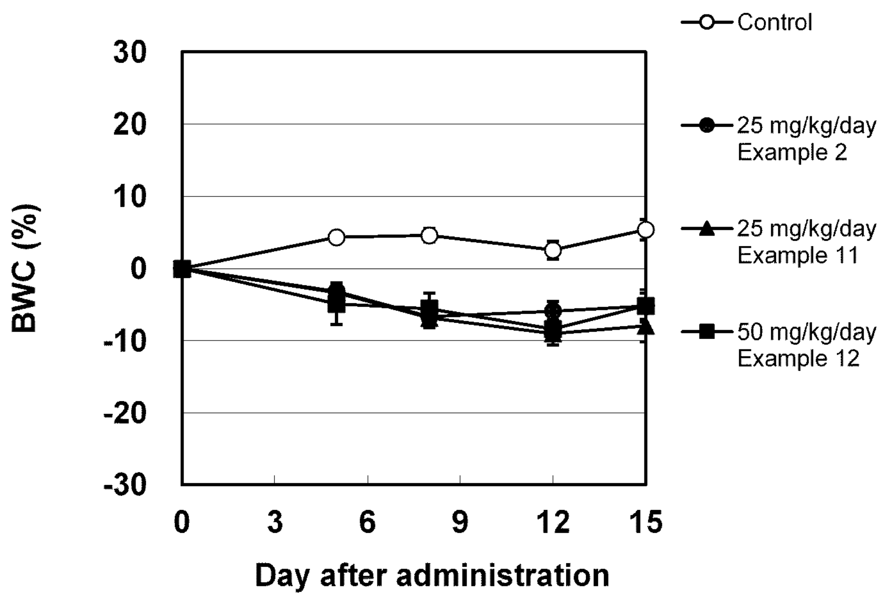

[Figure 3]

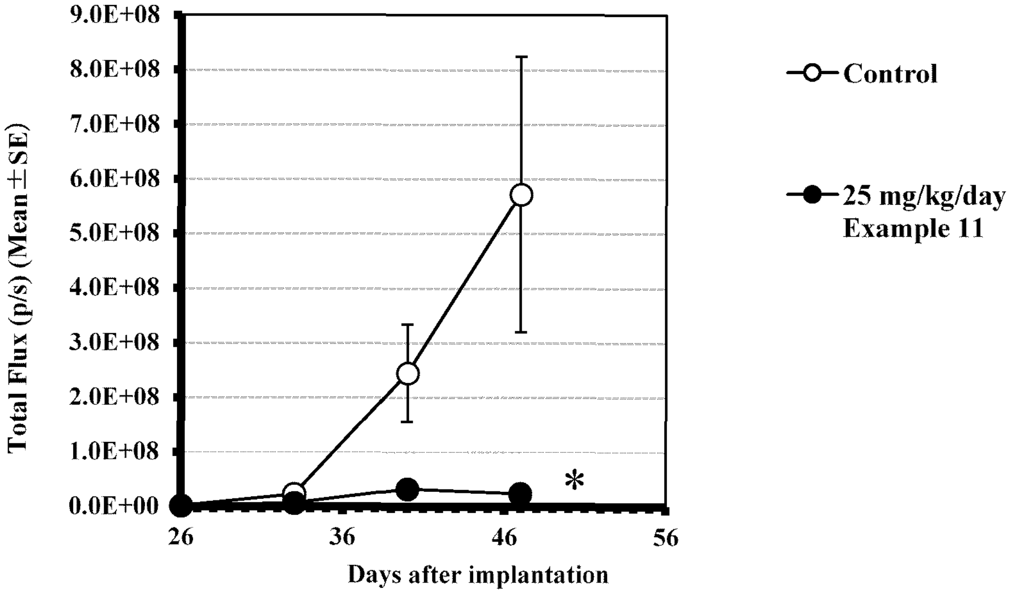


[Figure 4]

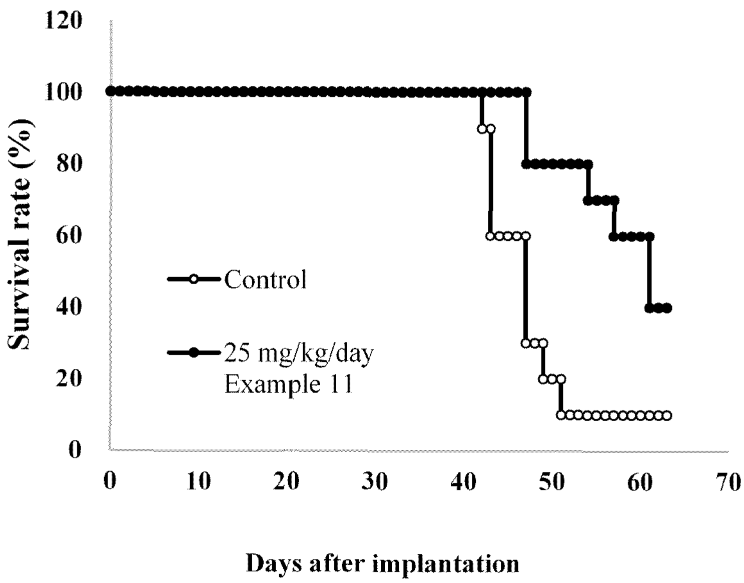

## INTERNATIONAL SEARCH REPORT

International application No.
PCT/JP2021/026461

A. CLASSIFICATION OF SUBJECT MATTER
A61K 31/519(2006.01)i; A61P 35/00(2006.01)i; A61P 43/00(2006.01)i; C07D 487/04(2006.01)i
FI: A61K31/519 ZNA; A61P35/00; A61P43/00 111; C07D487/04 CSP; C07D487/04 140
According to International Patent Classification (IPC) or to both national classification and IPC

B. FIELDS SEARCHED
Minimum documentation searched (classification system followed by classification symbols)
A61K31/519; A61P35/00; A61P43/00; C07D487/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| A | WO 2017/038838 A1 (TAIHO PHARMACEUTICAL CO., LTD.) 09 March 2017 (2017-03-09) claims, examples | 1-25 |
| A | WO 2013/118817 A1 (TAIHO PHARMACEUTICAL CO., LTD.) 15 August 2013 (2013-08-15) claims, examples | 1-25 |
| A | WO 2010/065898 A2 (PRINCIPIA BIOPHARMA INC.) 10 June 2010 (2010-06-10) claims, examples | 1-25 |
| P, X | WO 2020/145374 A1 (TAIHO PHARMACEUTICAL CO., LTD.) 16 July 2020 (2020-07-16) claims, examples | 1-25 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 August 2021 (30.08.2021) | 21 September 2021 (21.09.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/026461

| Patent Documents referred in the Report | Publication Date | Patent Family | Publication Date |
|---|---|---|---|
| WO 2017/038838 A1 | 09 Mar. 2017 | US 2017/0217970 A1<br>claims, examples | |
| WO 2013/118817 A1 | 15 Aug. 2013 | (Family: none) | |
| WO 2010/065898 A2 | 10 Jun. 2010 | US 2010/0144705 A1<br>claims, examples | |
| WO 2020/145374 A1 | 16 Jul. 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017146116 A
- WO 2017038838 A


### Non-patent literature cited in the description


- *Nat. Rev. Cancer,* 2006, vol. 6, 803-812
- *Nature Medicine,* 2013, vol. 19, 1389-1400
- *Nat. Rev. Cancer,* 2007, vol. 7, 169-181
- *Signal Transduct Target Ther.,* 2019, vol. 4 (5), 1-10
- *Lancet Oncol.,* 2015, vol. 16, 830-838
- *Current Oncology,* 2018, vol. 25, S103-S114
- *Breast Cancer Research,* 2016, vol. 18 (1), 1-9
- **T. W. GREENE ; P. G. M. WUTS.** Protective Groups in Organic Synthesis. John Wiley & Sons, 1999
- **T. W. GREENE ; P. G. M. WUTS.** Protective Groups in Organic Synthesis. John Wiley & Sons, 1981
- Peptide Gosei no Kiso to Jikken. **NOBUO IZUMIYA et al.** Principle of Peptide Synthesis and Experiments. Maruzen Co., Ltd, 1983
- **HIROKAWA SHOTEN.** Iyakuhin no Kaihatsu. *Development of Pharmaceutical Products,* 1990, vol. 7
- **BUNSHI SEKKEI.** *Molecular Designing,* 163-198